# EUROPÄISCHE PATENTSCHRIFT

(11) **EP 2 737 554 B1**
(45) Veröffentlichungstag und Bekanntmachung des Hinweises auf die Patenterteilung: **11.01.2017**
(21) Anmeldenummer: 12732983.7
(22) Anmeldetag: 03.07.2012
(51) Int. Cl.: H01L 51/54

(54) **VERBINDUNGEN FÜR ELEKTRONISCHE VORRICHTUNGEN**
COMPOUNDS FOR ELECTRONIC DEVICES
COMPOSÉS POUR DISPOSITIFS ÉLECTRONIQUES

(30) Priorität: 29.07.2011 EP 11006267
(43) Veröffentlichungstag der Anmeldung: 04.06.2014
(73) Patentinhaber: Merck Patent GmbH, 64293 Darmstadt (DE)
(72) Erfinder: PARHAM, Amir, Hossain, 65929 Frankfurt am Main (DE); PFLUMM, Christof, 64291 Darmstadt (DE); JATSCH, Anja, 60486 Frankfurt am Main (DE)
(86) Internationale Anmeldenummer: PCT/EP2012/002797
(87) Internationale Veröffentlichungsnummer: WO 2013/017189

(56) Entgegenhaltungen:
- WO-A1-2010/136109
- KR-A- 20110 002 156

## Beschreibung

Die vorliegende Erfindung betrifft Verbindungen zur Verwendung in elektronischen Vorrichtungen, bevorzugt organischen Elektrolumineszenzvorrichtungen. Weiterhin betrifft die Erfindung Herstellungsverfahren für die genannten Verbindungen sowie elektronische Vorrichtungen enthaltend die genannten Verbindungen, bevorzugt in einer Funktion als Matrixmaterialien und/oder als Elektronentransportmaterialien.

Die Entwicklung von neuartigen funktionellen Verbindungen zur Verwendung in elektronischen Vorrichtungen ist aktuell Gegenstand intensiver Forschung. Ziel ist hierbei die Entwicklung und Untersuchung von Verbindungen, welche bislang noch nicht in elektronischen Vorrichtungen eingesetzt wurden, sowie die Entwicklung von Verbindungen, welche ein verbessertes Eigenschaftsprofil der Vorrichtungen ermöglichen.

Unter dem Begriff elektronische Vorrichtung werden gemäß der vorliegenden Erfindung unter anderem organische integrierte Schaltungen (OICs), organische Feld-Effekt-Transistoren (OFETs), organische Dünnfilmtransistoren (OTFTs), organische lichtemittierende Transistoren (OLETs), organische Solarzellen (OSCs), organische optische Detektoren, organische Photorezeptoren, organische Feld-Quench-Devices (OFQDs), organische lichtemittierende elektrochemische Zellen (OLECs), organische Laserdioden (O-Laser) und organische Elektrolumineszenzvorrichtungen (OLEDs) verstanden.

Der Aufbau der oben genannten organischen Elektrolumineszenzvorrichtungen (OLEDs) ist dem Fachmann bekannt und unter anderem in US 4539507, US 5151629, EP 0676461 und WO 1998/27136 beschrieben.

Betreffend die Leistungsdaten der organischen Elektrolumineszenzvorrichtungen sind, insbesondere in Hinblick auf eine breite kommerzielle Verwendung, noch weitere Verbesserungen erforderlich. Von besonderer Bedeutung sind in diesem Zusammenhang die Lebensdauer, die Effizienz und die Betriebsspannung der organischen Elektrolumineszenzvorrichtungen sowie die realisierten Farbwerte. Insbesondere bei blau emittierenden Elektrolumineszenzvorrichtungen besteht Verbesserungspotential bezüglich der Lebensdauer der Vorrichtungen. Zudem ist es wünschenswert, dass die Verbindungen zur Verwendung als Funktionsmaterialien in elektronischen Vorrichtungen eine hohe thermische Stabilität und eine hohe Glasübergangstemperatur aufweisen und sich unzersetzt sublimieren lassen.

Auf dem Gebiet der elektronischen Vorrichtungen enthaltend organische Materialien besteht Bedarf an Matrixmaterialien, insbesondere an Matrixmaterialien für phosphoreszierende Emitter, die gleichzeitig zu guter Effizienz, hoher Lebensdauer und geringer Betriebsspannung der elektronischen Vorrichtungen führen. Gerade die Eigenschaften der Matrixmaterialien sind häufig limitierend für die Lebensdauer und die Effizienz der organischen Elektrolumineszenzvorrichtung. Bei Matrixmaterialien für phosphoreszierende Emitter ist es wünschenswert, dass diese ein hochliegendes T₁-Niveau (Triplettniveau) aufweisen. Besonders relevant ist dies bei Matrixmaterialien für blau emittierende phosphoreszierende Emitter.

Weiterhin ist die Bereitstellung neuer Elektronentransportmaterialien wünschenswert, da gerade auch die Eigenschaften des Elektronentransportmaterials einen wesentlichen Einfluss auf die oben genannten Eigenschaften der organischen Elektrolumineszenzvorrichtung ausüben. Insbesondere besteht Bedarf an Elektronentransportmaterialien, welche gleichzeitig zu guter Effizienz, hoher Lebensdauer und geringer Betriebsspannung führen.

Gemäß dem Stand der Technik werden häufig Carbazolderivate, z. B. Bis(carbazolyl)biphenyl, als Matrixmaterialien für phosphoreszierende Emitter verwendet. Ebenfalls werden in dieser Funktion Ketone (WO 2004/093207), Phosphinoxide, Sulfone (WO 2005/003253), Triazinverbindungen wie Triazinylspirobifluoren (vgl. WO 2010/015306) sowie Metallkomplexe, beispielsweise BAlq oder Bis[2-(2-benzothiazol)phenolat]-zink(11), verwendet.
Ebenfalls im Stand der Technik bekannt ist der Einsatz von Indeno-carbazolderivaten als Matrixmaterialien, welche anstelle der Indenyleinheit eine Spirobifluoreneinheit aufweisen und welche weiterhin an der Carbazoleinheit eine elektronenarme Heteroarylgruppe, beispielsweise eine Triazingruppe, tragen (WO 2010/136109).

KR20110002156 A offenbart Verbindungen 1-4-20 und 1-4-25, die eine elektronenreiche Heteroarylgruppe umfassen, die an eine Hälfte eines modifizierten Spirobifluoren-Grundkörpers gebundet ist, während an der anderen Hälfte des modifizierten Spirobifluoren-Grundkörpers eine elektronenaktive Gruppe ankondensiert ist. Verbindung 1-4-24 umfasst eine elektronenarme Heteroarylgruppe, welche über eine große kondensierte Arylgruppe (d.h. Anthracen) an eine Hälfte eines modifizierten Spirobifluoren-Grundkörpers gebundet ist, während an der anderen Hälfte des modifizierten Spirobifluoren-Grundkörpers eine elektronenaktive Gruppe ankondensiert ist.

Es besteht jedoch weiterhin Bedarf an neuen Verbindungen zur Verwendung als Funktionsmaterialien für elektronische Vorrichtungen. Insbesondere besteht Bedarf an Verbindungen zur Verwendung als Matrixmaterialien oder als Elektronentransportmaterialien in organischen Elektrolumineszenzvorrichtungen. Nochmals insbesondere besteht Bedarf an Verbindungen, mit denen eine Verbesserung der Leistungsdaten der elektronischen Vorrichtung erreicht werden kann.

Der vorliegenden Erfindung liegt somit die technische Aufgabe zu Grunde, Verbindungen bereitzustellen, welche sich zur Verwendung in elektronischen Vorrichtungen wie beispielsweise OLEDs eignen, und welche insbesondere als Matrixmaterialien für phosphoreszierende Emitter und/oder als Elektronentransportmaterialien eingesetzt werden können.

Die Verbindungen gemäß der vorliegenden Erfindung sind dadurch gekennzeichnet, dass mindestens eine elektronenarme Gruppe, bevorzugt eine elektronenarme Heteroarylgruppe, an einer Hälfte des Spirobifluorengrundgerüsts gebunden ist, während an der anderen Hälfte, welche mit der zuerst genannten Hälfte nicht konjugiert ist, eine ankondensierte Heteroarylgruppe vorliegt, bevorzugt eine ankondensierte Indolgruppe. In einer alternativen Ausführungsform sind die erfindungsgemäßen Verbindungen dadurch gekennzeichnet, dass eine Hälfte des Spirobifluorengrundgerüsts in ihrem Grundgerüst eine elektronenarme Brücke, bevorzugt eine Ketobrücke C=O, aufweist, während an der anderen Hälfte, welche mit der zuerst genannten Hälfte nicht konjugiert ist, eine ankondensierte Heteroarylgruppe vorliegt, bevorzugt eine ankondensierte Indolgruppe.

Gegenstand der Erfindung ist eine Verbindung einer Formel (I) oder (II) 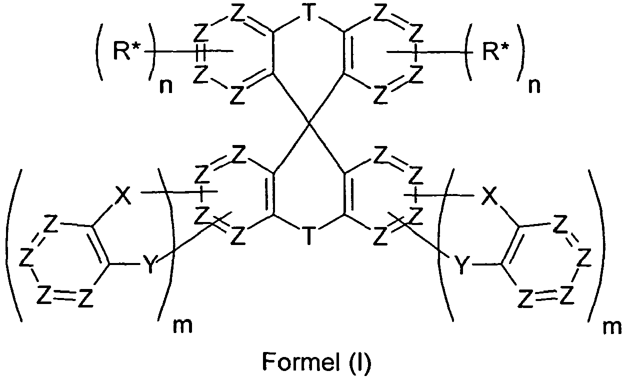 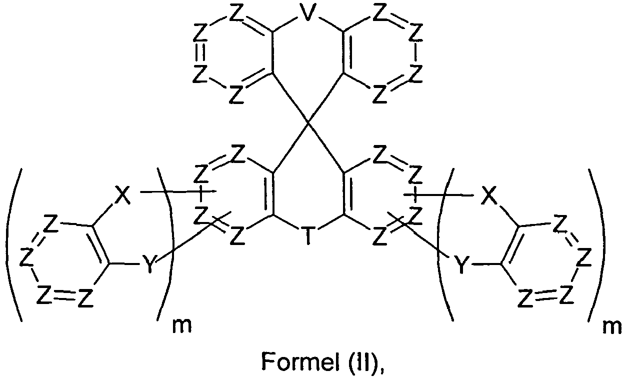 wobei für die auftretenden Symbole und Indices gilt:
R* ist bei jedem Auftreten gleich oder verschieden eine elektronenarme Heteroarylgruppe mit 5 bis 14 aromatischen Ringatomen oder eine Ketogruppe oder eine Phosphoroxidgruppe oder eine Schwefeloxidgruppe, welche jeweils direkt oder über eine Gruppe L gebunden sind und welche mit einem oder mehreren Resten R² substituiert sein können, oder -CN;
   - L: ist bei jedem Auftreten gleich oder verschieden eine divalente Gruppe der Formel -(Ar¹)ₖ-, wobei
   - Ar¹: bei jedem Auftreten gleich oder verschieden eine Arylen- oder Heteroarylengruppe mit 5 bis 10 aromatischen Ringatomen darstellt, welche mit einem oder mehreren Resten R² substituiert sein kann; und
   - k: bei jedem Auftreten gleich oder verschieden gewählt ist aus 1, 2, 3, 4 oder 5;
   - X, Y: sind bei jedem Auftreten gleich oder verschieden ausgewählt aus einer Einfachbindung, C(R¹)₂, NR¹, PR¹, P(O)R¹, O, S, SO und SO₂, wobei Fälle ausgeschlossen sind, in denen keine der beiden Gruppen X und Y eines Rings ausgewählt ist aus NR¹, PR¹, P(O)R¹, O, S, SO und SO₂;
   - V: ist ausgewählt aus einer Einfachbindung, CO, CS, P(O)R¹, SO und SO₂, wobei V nur dann eine Einfachbindung sein kann, wenn mindestens eine der Gruppen Z in den an V gebundenen Ringen gleich N ist;
   - T: ist bei jedem Auftreten gleich oder verschieden ausgewählt aus einer Einfachbindung, C(R¹)₂, CO, CS, Si(R¹)₂, NR¹, PR¹, P(O)R¹, O, S, SO und SO₂;
   - Z: ist bei jedem Auftreten gleich oder verschieden ausgewählt aus CR¹ und N, wenn keine Gruppe an Z gebunden ist, und ist gleich C, wenn eine Gruppe an Z gebunden ist;
   - R¹: ist bei jedem Auftreten gleich oder verschieden H, D, F, Cl, Br, I, B(OR²)₂, CHO, C(=O)R², CR²=C(R²)2, CN, C(=O)OR², C(=O)N(R²)₂, Si(R²)₃, N(R²)₂, NO₂, P(=O)(R²)₂, OSO₂R², OR², S(=O)R², S(=O)₂R², eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R² substituiert sein können und wobei eine oder mehrere CH₂-Gruppen in den oben genannten Gruppen durch -R²C=CR²-, -C=C-, Si(R²)_{2,} C=O, C=S, C=NR², -C(=O)O-, -C(=O)NR²-, NR², P(=O)(R²), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 30 aromatischen Ringatomen, das jeweils durch einen oder mehrere Reste R² substituiert sein kann, oder eine Aryloxy- oder Heteroaryloxygruppe mit 5 bis 30 aromatischen Ringatomen, die durch einen oder mehrere Reste R² substituiert sein kann, wobei zwei oder mehr Reste R¹ miteinander verknüpft sein können und einen Ring bilden können;
   - R²: ist bei jedem Auftreten gleich oder verschieden H, D, F, Cl, Br, I, B(OR³)_{2,} CHO, C(=O)R³, CR³=C(R³)₂, CN, C(=O)OR³, C(=O)N(R³)₂, Si(R³)_{3,} N(R³)_{2,} NO₂, P(=O)(R³)₂, OSO₂R³, OR³, S(=O)R³, S(=O)₂R³, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch -R³C=CR³-, -C≡C-, Si(R³)₂, C=O, C=S, C=NR³, -C(=O)O-, -C(=O)NR³-, NR³, P(=O)(R³), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 30 aromatischen Ringatomen, das jeweils durch einen oder mehrere Reste R³ substituiert sein kann, oder eine Aryloxy- oder Heteroaryloxygruppe mit 5 bis 30 aromatischen Ringatomen, die durch einen oder mehrere Reste R³ substituiert sein kann,
   - R³: ist bei jedem Auftreten gleich oder verschieden H, D, F oder ein aliphatischer, aromatischer oder heteroaromatischer organischer Rest mit 1 bis 20 C-Atomen, in dem auch ein oder mehrere H-Atome durch D oder F ersetzt sein können; dabei können zwei oder mehr Substituenten R³ miteinander verknüpft sein und einen Ring bilden;
   - m: ist bei jedem Auftreten gleich oder verschieden 0 oder 1, wobei nicht beide Indices m in einer Formel gleich 0 sein können;
   - n: ist bei jedem Auftreten gleich oder verschieden 0 oder 1, wobei nicht beide Indices n in einer Formel gleich 0 sein können,
   und wobei die Gruppen X und Y jeweils in benachbarten Positionen am Sechsring des Spirobifluorenderivats binden.

Es wird im Rahmen der vorliegenden Anmeldung die folgende Nummerierung der Positionen am modifizierten Spirobifluoren-Grundgerüst verwendet:

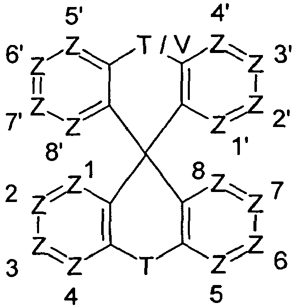

Unter einer elektronenarmen Heteroarylgruppe mit 5 bis 14 aromatischen Ringatomen als Ausführungsform von R* wird im Rahmen der vorliegenden Anmeldung bevorzugt Pyridin, Pyrimidin, Pyridazin, Pyrazin, Triazin und Benzimidazol verstanden.

Unter einer Heteroarylgruppe mit 5 bis 14 aromatischen Ringatomen als Ganz besonders bevorzugt ist Pyridin, Pyrimidin, Pyridazin, Pyrazin, Unter dem Begriff "elektronenarme Heteroarylgruppe" wird insbesondere eine Heteroarylgruppe umfassend mindestens einen heteroaromatischen Sechsring mit einem oder mehr Stickstoffatomen oder mindestens einen heteroaromatischen Fünfring mit zwei oder mehr Stickstoffatomen verstanden.

Unter einer Ketogruppe wird im Rahmen der vorliegenden Anmeldung eine Gruppe der folgenden Formel (K) verstanden 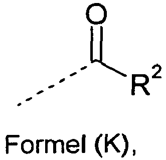 wobei die gestrichelte Bindung die Anbindungsposition der Ketogruppe darstellt und R² definiert ist wie oben. R² ist in diesem Zusammenhang bevorzugt ausgewählt aus einer Aryl- oder Heteroarylgruppe mit 5 bis 14 aromatischen Ringatomen, welche mit einem oder mehreren Resten R³ substituiert sein kann. Besonders bevorzugt ist R² ausgewählt aus einer Aryl- oder Heteroarylgruppe mit 5 bis 10 aromatischen Ringatomen, welche mit einem oder mehreren Resten R³ substituiert sein kann.

Unter einer Phosphoroxidgruppe wird im Rahmen der vorliegenden Anmeldung bevorzugt eine Gruppe der folgenden Formel (P) verstanden 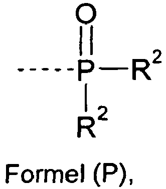 wobei die gestrichelte Bindung die Anbindungsstelle der Phosphoroxidgruppe darstellt und R² wie oben definiert ist. R² ist in diesem Zusammenhang bevorzugt ausgewählt aus einer Aryl- oder Heteroarylgruppe mit 5 bis 14 aromatischen Ringatomen, welche mit einem oder mehreren Resten R³ substituiert sein kann. Besonders bevorzugt ist R² ausgewählt aus einer Aryl- oder Heteroarylgruppe mit 5 bis 10 aromatischen Ringatomen, welche mit einem oder mehreren Resten R³ substituiert sein kann.

Unter einer Schwefeloxidgruppe wird im Rahmen der vorliegenden Anmeldung eine Gruppe der folgenden Formel (S) verstanden 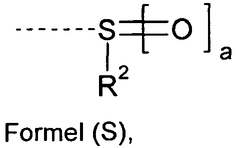 wobei die gestrichelte Bindung die Anbindungsstelle der Schwefeloxidgruppe darstellt,
a gleich 1 oder 2 sein kann,
und R² wie oben definiert ist. R² ist in diesem Zusammenhang bevorzugt ausgewählt aus einer Aryl- oder Heteroarylgruppe mit 5 bis 14 aromatischen Ringatomen, welche mit einem oder mehreren Resten R³ substituiert sein kann. Besonders bevorzugt ist R² ausgewählt aus einer Aryl- oder Heteroarylgruppe mit 5 bis 10 aromatischen Ringatomen, welche mit einem oder mehreren Resten R³ substituiert sein kann.

Eine Arylgruppe im Sinne dieser Erfindung enthält 6 bis 60 aromatische Ringatome; eine Heteroarylgruppe im Sinne dieser Erfindung enthält 5 bis 60 aromatische Ringatome, von denen mindestens eines ein Heteroatom darstellt. Die Heteroatome sind bevorzugt ausgewählt aus N, O und S. Dies stellt die grundlegende Definition dar. Werden in der Beschreibung der vorliegenden Erfindung andere Bevorzugungen angegeben, beispielsweise bezüglich der Zahl der aromatischen Ringatome oder der enthaltenden Heteroatome, so gelten diese.

Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin oder Thiophen, oder ein kondensierter (annellierter) aromatischer bzw. heteroaromatischer Polycyclus, beispielsweise Naphthalin, Phenanthren, Chinolin oder Carbazol verstanden. Ein kondensierter (annellierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Unter einer Aryloxygruppe gemäß der Definition der vorliegenden Erfindung wird eine Arylgruppe, wie oben definiert, verstanden, welche über ein Sauerstoffatom gebunden ist. Eine analoge Definition gilt für Heteroaryloxygruppen.

Ein aromatisches Ringsystem im Sinne dieser Erfindung enthält 6 bis 60 C-Atome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein sp³-hybridisiertes C-, Si-, N- oder O-Atom, ein sp²-hybridisiertes C- oder N-Atom oder ein sp-hybridisiertes C-Atom, verbunden sein können. So sollen beispielsweise auch Systeme wie 9,9'-Spirobifluoren, 9,9'-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Aryl- oder Heteroarylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Diphenyltriazin.

Unter einem aromatischen oder heteroaromatischen Ringsystem mit 5 - 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,+6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Kombinationen dieser Gruppen.

Im Rahmen der vorliegenden Erfindung werden unter einer geradkettigen Alkylgruppe mit 1 bis 40 C-Atomen bzw. einer verzweigten oder cyclischen Alkylgruppe mit 3 bis 40 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neo-Pentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer Alkoxy- oder Thioalkylgruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung verknüpft sind. Dies wird durch das folgende Schema verdeutlicht:

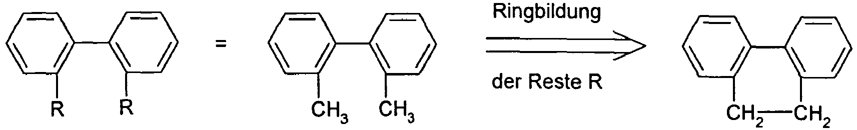

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

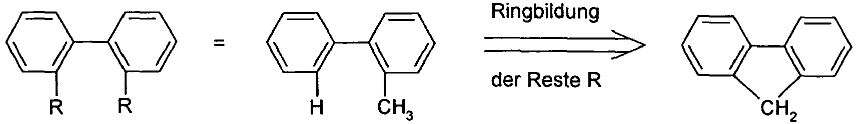

Gemäß einer bevorzugten Ausführungsform enthält die erfindungsgemäße Verbindung keine kondensierten Arylgruppen mit mehr als 18 aromatischen Ringatomen, besonders bevorzugt keine kondensierten Arylgruppen mit mehr als 16 aromatischen Ringatomen, ganz besonders bevorzugt keine kondensierten Arylgruppen mit mehr als 14 aromatischen Ringatomen, und nochmals stärker bevorzugt keine kondensierten Arylgruppen mit mehr als 10 aromatischen Ringatomen.

Weiterhin bevorzugt ist an die Gruppen X und Y keine Heteroarylgruppe mit 6 aromatischen Ringatomen gebunden. Besonders bevorzugt ist an die Gruppen X und Y keine Heteroarylgruppe, keine Ketogruppe, keine Phosphoroxidgruppe und keine Schwefeloxidgruppe gebunden.

Gemäß einer bevorzugten Ausführungsform stellt der Rest R* eine Gruppe -CN oder eine Gruppe der Formel (K), (P) oder (S), wie oben beschrieben, oder eine Gruppe der untenstehend aufgeführten Formeln (H-1) bis (H-10) dar 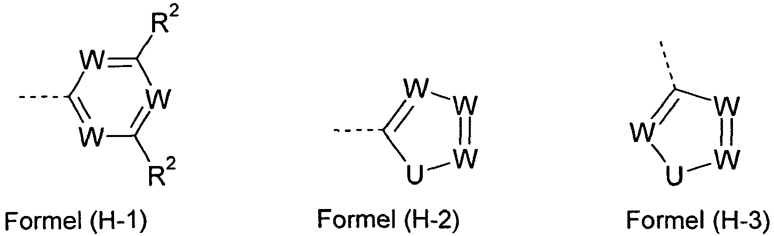 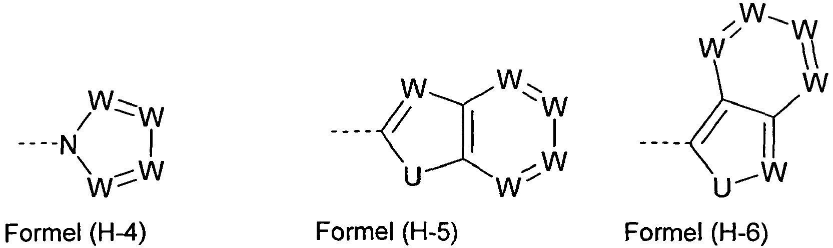 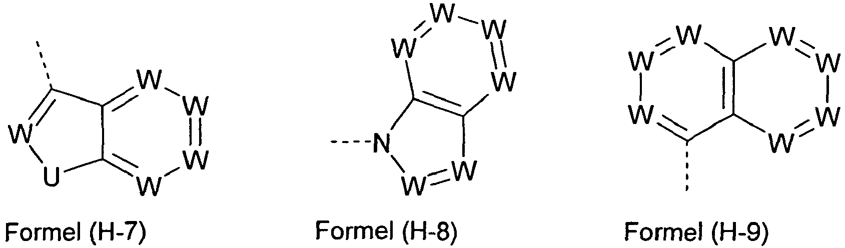 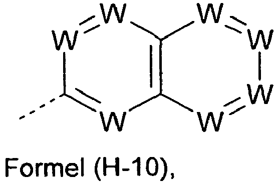 wobei die gestrichelte Bindung die Anbindungsposition markiert, R² wie oben definiert ist und
- W: bei jedem Auftreten gleich oder verschieden CR² oder N darstellt, und
- U: NR², O oder S darstellt, und
wobei mindestens eine Gruppe W pro Formel gleich N ist.

Ganz besonders bevorzugt ist R* ausgewählt aus Pyridin, Pyrimidin, Pyridazin, Pyrazin, Triazin und Benzimidazol, welche jeweils mit einem oder mehreren Resten R² substituiert sein können.

Gemäß einer bevorzugten Ausführungsform der Erfindung ist keine Gruppe L vorhanden.

Wenn eine Gruppe L der Formel -(Ar¹)ₖ- vorhanden ist, so ist Ar¹ bevorzugt Phenylen oder eine Heteroarylengruppe mit 5 bis 10 aromatischen Ringatomen, wobei die genannten Gruppen jeweils mit einem oder mehreren Resten R² substituiert sein können. Besonders bevorzugt ist Ar¹ ortho-, meta- oder para-Phenylen, welches jeweils mit einem oder mehreren Resten R² substituiert sein kann. Weiterhin bevorzugt ist der Index k in der Formel -(Ar¹)ₖ- bevorzugt gleich 1,2 oder 3, besonders bevorzugt gleich 1 oder 2 und ganz besonders bevorzugt gleich 1.

Es ist weiterhin bevorzugt, dass die Gruppe R* in der Position 2' oder in der Position 7' am modifizierten Spirobifluoren-Grundgerüst gebunden ist. Die Gruppen X und Y sind bevorzugt gleich oder verschieden ausgewählt aus einer Einfachbindung, C(R¹)₂, NR¹, O und S, wobei Fälle ausgeschlossen sind, in denen keine der beiden Gruppen X und Y eines Rings ausgewählt ist aus NR¹, O und S.

Bevorzugte Kombinationen von Gruppen X und Y in einem Ring sind in der folgenden Tabelle abgebildet:

| | X | Y |
|---|---|---|
| X-Y-1 | Einfachbindung | NR¹ |
| X-Y-2 | Einfachbindung | O |
| X-Y-3 | Einfachbindung | S |
| X-Y-4 | C(R¹)₂ | NR¹ |
| X-Y-5 | C(R¹)₂ | O |
| X-Y-6 | C(R¹)₂ | S |
| X-Y-7 | NR¹ | Einfachbindung |
| X-Y-8 | O | Einfachbindung |
| X-Y-9 | S | Einfachbindung |
| X-Y-10 | NR¹ | C(R¹)₂ |
| X-Y-11 | O | C(R¹)₂ |
| X-Y-12 | S | C(R¹)₂ |

Weiterhin bevorzugt ist es, wenn genau eine der beiden Gruppen X und Y eines Rings eine Einfachbindung darstellt, so dass ein Fünfring entsteht.

Besonders bevorzugt ist es, wenn eine der beiden Gruppen X und Y in einem Ring eine Einfachbindung darstellt und die andere der beiden Gruppen X und Y eine Gruppe NR¹ darstellt.

Weiterhin bevorzugt ist es, dass die Gruppen X und Y in den Positionen 2 und 3 und/oder in den Positionen 6 und 7 am modifizierten Spirobifluoren-Grundgerüst gebunden sind.

Gemäß einer bevorzugten Ausführungsform ist weiterhin die Gruppe V gleich CO.

Gemäß einer bevorzugten Ausführungsform ist weiterhin die Gruppe T bei jedem Auftreten gleich oder verschieden ausgewählt aus einer Einfachbindung, C(R¹)₂, O und S, besonders bevorzugt aus einer Einfachbindung und C(R¹)₂. Gemäß einer ganz besonders bevorzugten Ausführungsform stellt T eine Einfachbindung dar.

Weiterhin ist es bevorzugt, wenn keine, eine, zwei oder drei Gruppen Z pro aromatischem Sechsring gleich N sind, wobei nicht mehr als zwei benachbarte Gruppen Z gleichzeitig gleich N sind. Besonders bevorzugt ist nicht mehr als eine Gruppe Z pro aromatischem Sechsring gleich N, ganz besonders bevorzugt ist keine Gruppe Z gleich N.

Es ist weiterhin bevorzugt, dass R¹ bei jedem Auftreten gleich oder verschieden ausgewählt ist aus H, D, F, CN, Si(R²)₃, N(R²)₂ oder einer geradkettigen Alkyl- oder Alkoxygruppe mit 1 bis 20 C-Atomen oder einer verzweigten oder cyclischen Alkyl- oder Alkoxygruppe mit 3 bis 20 C-Atomen, wobei die oben genannten Gruppen jeweils mit einem oder mehreren Resten R² substituiert sein können und wobei in den oben genannten Gruppen eine oder mehrere CH₂-Gruppen durch -C=C-, -R²C=CR²-, Si(R²)₂, C=O, C=NR², -NR²-, -O-, -S-, -C(=O)O- oder -C(=O)NR²- ersetzt sein können, oder ein aromatisches oder heteroaromatisches Ringsystem mit 5 bis 18 aromatischen Ringatomen, das jeweils mit einem oder mehreren Resten R² substituiert sein kann, wobei zwei oder mehr Reste R¹ miteinander verknüpft sein können und einen Ring bilden können.

Es ist weiterhin bevorzugt, dass R² bei jedem Auftreten gleich oder verschieden ausgewählt ist aus H, D, F, CN, Si(R³)₃, N(R³)₂ oder einer geradkettigen Alkyl- oder Alkoxygruppe mit 1 bis 20 C-Atomen oder einer verzweigten oder cyclischen Alkyl- oder Alkoxygruppe mit 3 bis 20 C-Atomen, wobei die oben genannten Gruppen jeweils mit einem oder mehreren Resten R³ substituiert sein können und wobei in den oben genannten Gruppen eine oder mehrere CH₂-Gruppen durch -C=C-, -R³C=CR³-, Si(R³)₂, C=O, C=NR³, -NR³-, -O-, -S-, -C(=O)O- oder -C(=O)NR³- ersetzt sein können, oder ein aromatisches oder heteroaromatisches Ringsystem mit 5 bis 18 aromatischen Ringatomen, das jeweils mit einem oder mehreren Resten R³ substituiert sein kann, wobei zwei oder mehr Reste R² miteinander verknüpft sein können und einen Ring bilden können.

Gemäß einer bevorzugten Ausführungsform ist einer der beiden Indices m pro Formel gleich 1 und der andere ist gleich 0.

Gemäß einer weiteren bevorzugten Ausführungsform ist einer der beiden Indices n pro Formel gleich 1 und der andere ist gleich 0.

Bevorzugte Ausführungsformen der Verbindung gemäß Formel (I) entsprechen einer der folgenden Formeln (I-1) bis (I-12) 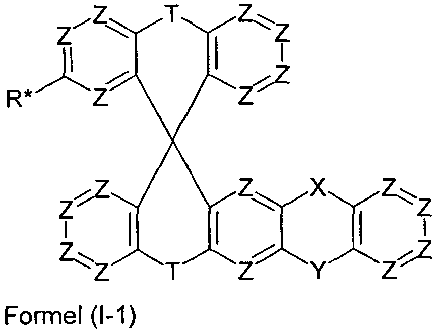 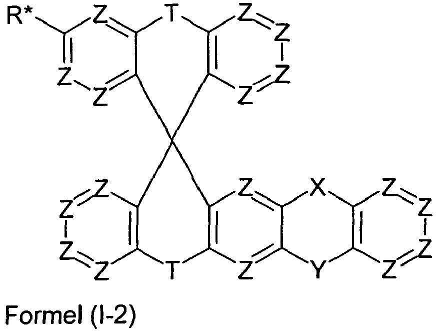 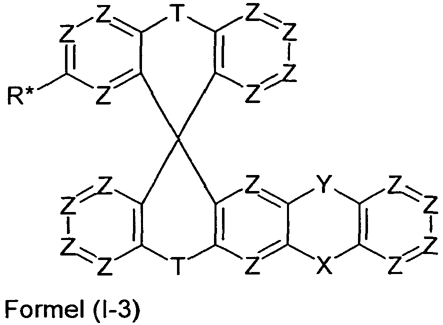 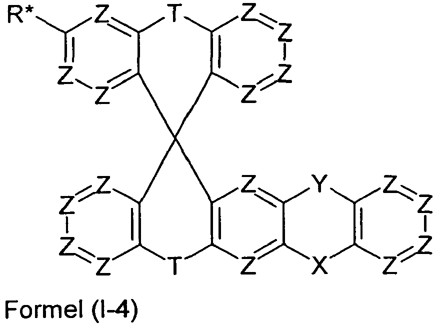 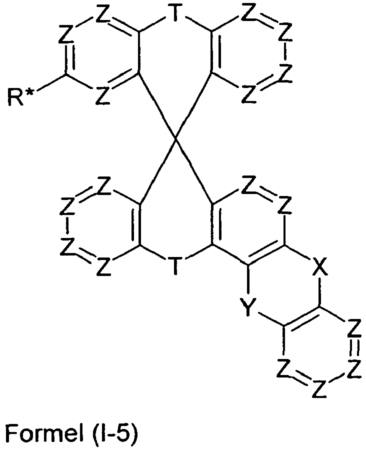 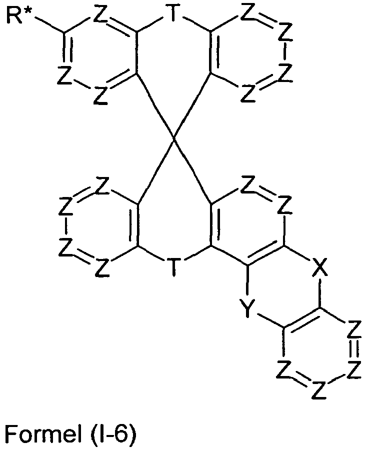 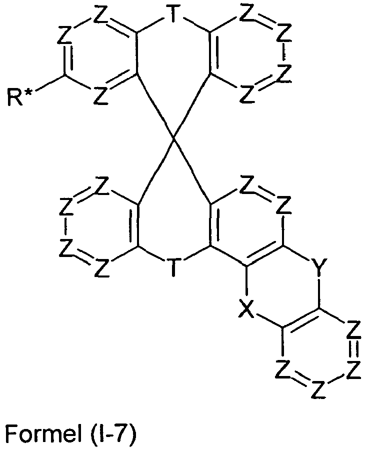 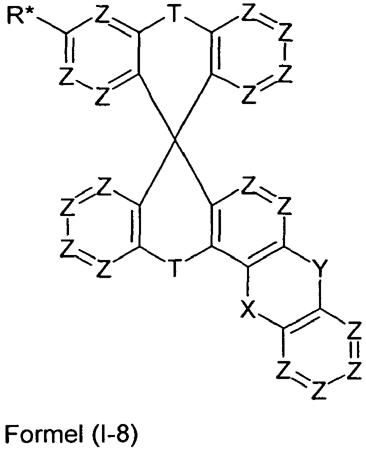 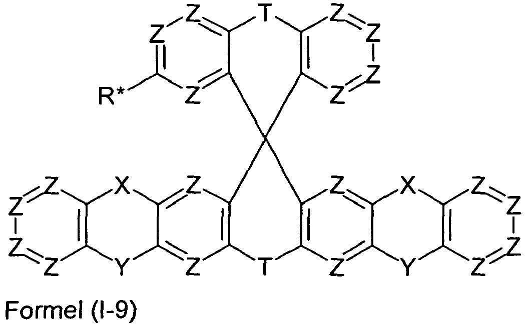 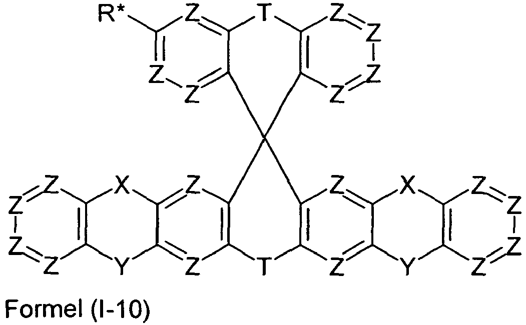 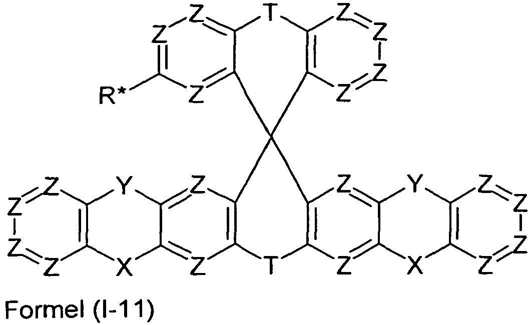 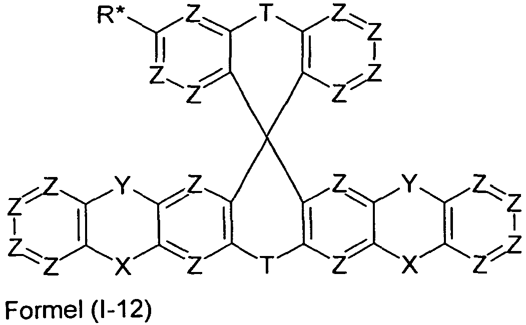 wobei die auftretenden Symbole wie obenstehend definiert sind und bevorzugt in ihren oben angegebenen bevorzugten Ausführungsformen vorliegen.

Besonders bevorzugt ist für die Formeln (I-1) bis (I-12) die Gruppe T eine Einfachbindung.

Weiterhin ist es bevorzugt, dass nicht mehr als eine Gruppe Z pro aromatischen Ring gleich N ist und die anderen Gruppen Z gleich CR¹ sind. Besonders bevorzugt ist keine Gruppe Z gleich N, so dass alle Gruppen Z gleich CR¹ sind.

Weiterhin bevorzugt ist an die Gruppen X und Y in den Formeln (I-1) bis (I-12) keine Heteroarylgruppe mit 6 aromatischen Ringatomen gebunden. Besonders bevorzugt ist an die Gruppen X und Y keine Heteroarylgruppe, keine Ketogruppe, keine Phosphoroxidgruppe und keine Schwefeloxidgruppe gebunden.

Weiterhin bevorzugt sind für die Formeln (I-1) bis (I-12) die in einer obenstehenden Tabelle angegebenen Kombinationen von Ausführungsformen von Gruppen X und Y.

Weiterhin sind die folgenden Kombinationen von Gruppen R* mit Grundgerüsten der Formeln (I-1) bis (I-12) bevorzugt:

| Formel | R* | L in R* | Grundgerüst |
|---|---|---|---|
| I-1-1 | Formel (K) | nicht vorhanden (n. v.) | Formel (I-1) |
| I-1-2 | siehe oben (s. o.) | m-Ph | s. o. |
| I-1-3 | s. o. | p-Ph | s. o. |
| I-1-4 | Formel (P) | n. v. | s. o. |
| I-1-5 | s. o. | m-Ph | s. o. |
| I-1-6 | s. o. | p-Ph | s. o. |
| I-1-7 | Formel (S) | n. v. | s. o. |
| I-1-8 | s. o. | m-Ph | s. o. |
| I-1-9 | s. o. | p-Ph | s. o. |
| I-1-10 | Formel (H-1) | n. v. | s. o. |
| I-1-11 | s. o. | m-Ph | s. o. |
| I-1-12 | s. o. | p-Ph | s. o. |
| I-1-13 | Formel (H-2) | n. v. | s. o. |
| I-1-14 | s. o. | m-Ph | s. o. |
| I-1-15 | s. o. | p-Ph | s. o. |
| I-1-16 | Formel (H-3) | n. v. | s. o. |
| I-1-17 | s. o. | m-Ph | s. o. |
| I-1-18 | s. o. | p-Ph | s. o. |
| I-1-19 | Formel (H-4) | n. v. | s. o. |
| I-1-20 | s. o. | m-Ph | s. o. |
| I-1-21 | s. o. | p-Ph | s. o. |
| I-1-22 | Formel (H-5) | n. v. | s. o. |
| 1-1-23 | s. o. | m-Ph | s. o. |
| I-1-24 | s. o. | p-Ph | s. o. |
| I-1-25 | Formel (H-6) | n. v. | s. o. |
| I-1-26 | s. o. | m-Ph | s. o. |
| I-1-27 | s. o. | p-Ph | s. o. |
| I-1-28 | Formel (H-7) | n. v. | s. o. |
| I-1-29 | s. o. | m-Ph | s. o. |
| I-1-30 | s. o. | p-Ph | s. o. |
| I-1-31 | Formel (H-8) | n. v. | s. o. |
| I-1-32 | s. o. | m-Ph | s. o. |
| I-1-33 | s. o. | p-Ph | s. o. |
| I-1-34 | Formel (H-9) | n. v. | s. o. |
| I-1-35 | s. o. | m-Ph | s. o. |
| I-1-36 | s. o. | p-Ph | s. o. |
| I-1-37 | Formel (H-10) | n. v. | s. o. |
| I-1-38 | s. o. | m-Ph | s. o. |
| I-1-39 | s. o. | p-Ph | s. o. |
| I-2-1 | Formel (K) | n. v. | Formel (I-2) |
| I-2-2 | s. o. | m-Ph | s. o. |
| I-2-3 | s. o. | p-Ph | s. o. |
| I-2-4 | Formel (P) | n. v. | s. o. |
| I-2-5 | s. o. | m-Ph | s. o. |
| I-2-6 | s. o. | p-Ph | s. o. |
| I-2-7 | Formel (S) | n. v. | s. o. |
| I-2-8 | s. o. | m-Ph | s. o. |
| I-2-9 | s. o. | p-Ph | s. o. |
| I-2-10 | Formel (H-1) | n. v. | s. o. |
| I-2-11 | s. o. | m-Ph | s. o. |
| I-2-12 | s. o. | p-Ph | s. o. |
| I-2-13 | Formel (H-2) | n. v. | s. o. |
| I-2-14 | s. o. | m-Ph | s. o. |
| 1-2-15 | s. o. | p-Ph | s. o. |
| I-2-16 | Formel (H-3) | n. v. | s. o. |
| I-2-17 | s. o. | m-Ph | s. o. |
| I-2-18 | s. o. | p-Ph | s. o. |
| I-2-19 | Formel (H-4) | n. v. | s. o. |
| I-2-20 | s. o. | m-Ph | s. o. |
| I-2-21 | s. o. | p-Ph | s. o. |
| I-2-22 | Formel (H-5) | n. v. | s. o. |
| I-2-23 | s. o. | m-Ph | s. o. |
| I-2-24 | s. o. | p-Ph | s. o. |
| I-2-25 | Formel (H-6) | n. v. | s. o. |
| I-2-26 | s. o. | m-Ph | s. o. |
| I-2-27 | s. o. | p-Ph | s. o. |
| I-2-28 | Formel (H-7) | n. v. | s. o. |
| I-2-29 | s. o. | m-Ph | s. o. |
| I-2-30 | s. o. | p-Ph | s. o. |
| I-2-31 | Formel (H-8) | n. v. | s. o. |
| I-2-32 | s. o. | m-Ph | s. o. |
| I-2-33 | s. o. | p-Ph | s. o. |
| I-2-34 | Formel (H-9) | n. v. | s. o. |
| I-2-35 | s. o. | m-Ph | s. o. |
| I-2-36 | s. o. | p-Ph | s. o. |
| I-2-37 | Formel (H-10) | n. v. | s. o. |
| I-2-38 | s. o. | m-Ph | s. o. |
| I-2-39 | s. o. | p-Ph | s. o. |
| I-3-1 | Formel (K) | n. v. | Formel (I-3) |
| I-3-2 | s. o. | m-Ph | s. o. |
| I-3-3 | s. o. | p-Ph | s. o. |
| I-3-4 | Formel (P) | n. v. | s. o. |
| I-3-5 | s. o. | m-Ph | s. o. |
| I-3-6 | s. o. | p-Ph | s. o. |
| I-3-7 | Formel (S) | n. v. | s. o. |
| I-3-8 | s. o. | m-Ph | s. o. |
| I-3-9 | s. o. | p-Ph | s. o. |
| I-3-10 | Formel (H-1) | n. v. | s. o. |
| I-3-11 | s. o. | m-Ph | s. o. |
| I-3-12 | s. o. | p-Ph | s. o. |
| I-3-13 | Formel (H-2) | n. v. | s. o. |
| I-3-14 | s. o. | m-Ph | s. o. |
| I-3-15 | s. o. | p-Ph | s. o. |
| I-3-16 | Formel (H-3) | n. v. | s. o. |
| I-3-17 | s. o. | m-Ph | s. o. |
| I-3-18 | s. o. | p-Ph | s. o. |
| I-3-19 | Formel (H-4) | n. v. | s. o. |
| I-3-20 | s. o. | m-Ph | s. o. |
| I-3-21 | s. o. | p-Ph | s. o. |
| I-3-22 | Formel (H-5) | n. v. | s. o. |
| I-3-23 | s. o. | m-Ph | s. o. |
| I-3-24 | s. o. | p-Ph | s. o. |
| I-3-25 | Formel (H-6) | n. v. | s. o. |
| I-3-26 | s. o. | m-Ph | s. o. |
| I-3-27 | s. o. | p-Ph | s. o. |
| I-3-28 | Formel (H-7) | n. v. | s. o. |
| I-3-29 | s. o. | m-Ph | s. o. |
| I-3-30 | s. o. | p-Ph | s. o. |
| I-3-31 | Formel (H-8) | n. v. | s. o. |
| I-3-32 | s. o. | m-Ph | s. o. |
| I-3-33 | s. o. | p-Ph | s. o. |
| I-3-34 | Formel (H-9) | n. v. | s. o. |
| I-3-35 | s. o. | m-Ph | s. o. |
| I-3-36 | s. o. | p-Ph | s. o. |
| I-3-37 | Formel (H-10) | n. v. | s. o. |
| I-3-38 | s. o. | m-Ph | s. o. |
| I-3-39 | s. o. | p-Ph | s. o. |
| I-4-1 | Formel (K) | n. v. | Formel (I-4) |
| I-4-2 | s. o. | m-Ph | s. o. |
| I-4-3 | s. o. | p-Ph | s. o. |
| I-4-4 | Formel (P) | n. v. | s. o. |
| I-4-5 | s. o. | m-Ph | s. o. |
| I-4-6 | s. o. | p-Ph | s. o. |
| I-4-7 | Formel (S) | n. v. | s. o. |
| I-4-8 | s. o. | m-Ph | s. o. |
| I-4-9 | s. o. | p-Ph | s. o. |
| I-4-10 | Formel (H-1) | n. v. | s. o. |
| I-4-11 | s. o. | m-Ph | s. o. |
| I-4-12 | s. o. | p-Ph | s. o. |
| I-4-13 | Formel (H-2) | n. v. | s. o. |
| I-4-14 | s. o. | m-Ph | s. o. |
| I-4-15 | s. o. | p-Ph | s. o. |
| I-4-16 | Formel (H-3) | n. v. | s. o. |
| I-4-17 | s. o. | m-Ph | s. o. |
| I-4-18 | s. o. | p-Ph | s. o. |
| I-4-19 | Formel (H-4) | n. v. | s. o. |
| I-4-20 | s. o. | m-Ph | s. o. |
| I-4-21 | s. o. | p-Ph | s. o. |
| I-4-22 | Formel (H-5) | n. v. | s. o. |
| I-4-23 | s. o. | m-Ph | s. o. |
| I-4-24 | s. o. | p-Ph | s. o. |
| I-4-25 | Formel (H-6) | n. v. | s. o. |
| I-4-26 | s. o. | m-Ph | s. o. |
| I-4-27 | s. o. | p-Ph | s. o. |
| I-4-28 | Formel (H-7) | n. v. | s. o. |
| I-4-29 | s. o. | m-Ph | s. o. |
| I-4-30 | s. o. | p-Ph | s. o. |
| I-4-31 | Formel (H-8) | n. v. | s. o. |
| I-4-32 | s. o. | m-Ph | s. o. |
| I-4-33 | s. o. | p-Ph | s. o. |
| I-4-34 | Formel (H-9) | n. v. | s. o. |
| I-4-35 | s. o. | m-Ph | s. o. |
| I-4-36 | s. o. | p-Ph | s. o. |
| I-4-37 | Formel (H-10) | n. v. | s. o. |
| I-4-38 | s. o. | m-Ph | s. o. |
| I-4-39 | s. o. | p-Ph | s. o. |
| I-5-1 | Formel (K) | n. v. | Formel (I-5) |
| I-5-2 | s. o. | m-Ph | s. o. |
| I-5-3 | s. o. | p-Ph | s. o. |
| I-5-4 | Formel (P) | n. v. | s. o. |
| I-5-5 | s. o. | m-Ph | s. o. |
| I-5-6 | s. o. | p-Ph | s. o. |
| I-5-7 | Formel (S) | n. v. | s. o. |
| I-5-8 | s. o. | m-Ph | s. o. |
| I-5-9 | s. o. | p-Ph | s. o. |
| I-5-10 | Formel (H-1) | n. v. | s. o. |
| I-5-11 | s. o. | m-Ph | s. o. |
| I-5-12 | s. o. | p-Ph | s. o. |
| I-5-13 | Formel (H-2) | n. v. | s. o. |
| I-5-14 | s. o. | m-Ph | s. o. |
| I-5-15 | s. o. | p-Ph | s. o. |
| I-5-16 | Formel (H-3) | n. v. | s. o. |
| I-5-17 | s. o. | m-Ph | s. o. |
| I-5-18 | s. o. | p-Ph | s. o. |
| I-5-19 | Formel (H-4) | n. v. | s. o. |
| I-5-20 | s. o. | m-Ph | s. o. |
| I-5-21 | s. o. | p-Ph | s. o. |
| I-5-22 | Formel (H-5) | n. v. | s. o. |
| I-5-23 | s. o. | m-Ph | s. o. |
| I-5-24 | s. o. | p-Ph | s. o. |
| I-5-25 | Formel (H-6) | n. v. | s. o. |
| I-5-26 | s. o. | m-Ph | s. o. |
| I-5-27 | s. o. | p-Ph | s. o. |
| I-5-28 | Formel (H-7) | n. v. | s. o. |
| I-5-29 | s. o. | m-Ph | s. o. |
| I-5-30 | s. o. | p-Ph | s. o. |
| I-5-31 | Formel (H-8) | n. v. | s. o. |
| I-5-32 | s. o. | m-Ph | s. o. |
| I-5-33 | s. o. | p-Ph | s. o. |
| I-5-34 | Formel (H-9) | n. v. | s. o. |
| I-5-35 | s. o. | m-Ph | s. o. |
| I-5-36 | s. o. | p-Ph | s. o. |
| I-5-37 | Formel (H-10) | n. v. | s. o. |
| I-5-38 | s. o. | m-Ph | s. o. |
| I-5-39 | s. o. | p-Ph | s. o. |
| I-6-1 | Formel (K) | n. v. | Formel (I-6) |
| I-6-2 | s. o. | m-Ph | s. o. |
| I-6-3 | s. o. | p-Ph | s. o. |
| I-6-4 | Formel (P) | n. v. | s. o. |
| I-6-5 | s. o. | m-Ph | s. o. |
| I-6-6 | s. o. | p-Ph | s. o. |
| I-6-7 | Formel (S) | n. v. | s. o. |
| I-6-8 | s. o. | m-Ph | s. o. |
| I-6-9 | s. o. | p-Ph | s. o. |
| I-6-10 | Formel (H-1) | n. v. | s. o. |
| I-6-11 | s. o. | m-Ph | s. o. |
| I-6-12 | s. o. | p-Ph | s. o. |
| I-6-13 | Formel (H-2) | n. v. | s. o. |
| I-6-14 | s. o. | m-Ph | s. o. |
| I-6-15 | s. o. | p-Ph | s. o. |
| I-6-16 | Formel (H-3) | n. v. | s. o. |
| I-6-17 | s. o. | m-Ph | s. o. |
| I-6-18 | s. o. | p-Ph | s. o. |
| I-6-19 | Formel (H-4) | n. v. | s. o. |
| I-6-20 | s. o. | m-Ph | s. o. |
| I-6-21 | s. o. | p-Ph | s. o. |
| I-6-22 | Formel (H-5) | n. v. | s. o. |
| I-6-23 | s. o. | m-Ph | s. o. |
| I-6-24 | s. o. | p-Ph | s. o. |
| I-6-25 | Formel (H-6) | n. v. | s. o. |
| I-6-26 | s. o. | m-Ph | s. o. |
| I-6-27 | s. o. | p-Ph | s. o. |
| I-6-28 | Formel (H-7) | n. v. | s. o. |
| I-6-29 | s. o. | m-Ph | s. o. |
| I-6-30 | s. o. | p-Ph | s. o. |
| I-6-31 | Formel (H-8) | n. v. | s. o. |
| I-6-32 | s. o. | m-Ph | s. o. |
| I-6-33 | s. o. | p-Ph | s. o. |
| I-6-34 | Formel (H-9) | n. v. | s. o. |
| I-6-35 | s. o. | m-Ph | s. o. |
| I-6-36 | s. o. | p-Ph | s. o. |
| I-6-37 | Formel (H-10) | n. v. | s. o. |
| I-6-38 | s. o. | m-Ph | s. o. |
| I-6-39 | s. o. | p-Ph | s. o. |
| I-7-1 | Formel (K) | n. v. | Formel (I-7) |
| I-7-2 | s. o. | m-Ph | s. o. |
| I-7-3 | s. o. | p-Ph | s. o. |
| I-7-4 | Formel (P) | n. v. | s. o. |
| I-7-5 | s. o. | m-Ph | s. o. |
| I-7-6 | s. o. | p-Ph | s. o. |
| I-7-7 | Formel (S) | n. v. | s. o. |
| I-7-8 | s. o. | m-Ph | s. o. |
| I-7-9 | s. o. | p-Ph | s. o. |
| I-7-10 | Formel (H-1) | n. v. | s. o. |
| I-7-11 | s. o. | m-Ph | s. o. |
| I-7-12 | s. o. | p-Ph | s. o. |
| I-7-13 | Formel (H-2) | n. v. | s. o. |
| I-7-14 | s. o. | m-Ph | s. o. |
| I-7-15 | s. o. | p-Ph | s. o. |
| I-7-16 | Formel (H-3) | n. v. | s. o. |
| I-7-17 | s. o. | m-Ph | s. o. |
| I-7-18 | s. o. | p-Ph | s. o. |
| I-7-19 | Formel (H-4) | n. v. | s. o. |
| I-7-20 | s. o. | m-Ph | s. o. |
| I-7-21 | s. o. | p-Ph | s. o. |
| I-7-22 | Formel (H-5) | n. v. | s. o. |
| I-7-23 | s. o. | m-Ph | s. o. |
| I-7-24 | s. o. | p-Ph | s. o. |
| I-7-25 | Formel (H-6) | n. v. | s. o. |
| I-7-26 | s. o. | m-Ph | s. o. |
| I-7-27 | s. o. | p-Ph | s. o. |
| I-7-28 | Formel (H-7) | n. v. | s. o. |
| I-7-29 | s. o. | m-Ph | s. o. |
| I-7-30 | s. o. | p-Ph | s. o. |
| I-7-31 | Formel (H-8) | n. v. | s. o. |
| I-7-32 | s. o. | m-Ph | s. o. |
| I-7-33 | s. o. | p-Ph | s. o. |
| I-7-34 | Formel (H-9) | n. v. | s. o. |
| I-7-35 | s. o. | m-Ph | s. o. |
| I-7-36 | s. o. | p-Ph | s. o. |
| I-7-37 | Formel (H-10) | n. v. | s. o. |
| I-7-38 | s. o. | m-Ph | s. o. |
| I-7-39 | s. o. | p-Ph | s. o. |
| I-8-1 | Formel (K) | n. v. | Formel (I-8) |
| I-8-2 | s. o. | m-Ph | s. o. |
| I-8-3 | s. o. | p-Ph | s. o. |
| I-8-4 | Formel (P) | n. v. | s. o. |
| I-8-5 | s. o. | m-Ph | s. o. |
| I-8-6 | s. o. | p-Ph | s. o. |
| I-8-7 | Formel (S) | n. v. | s. o. |
| I-8-8 | s. o. | m-Ph | s. o. |
| I-8-9 | s. o. | p-Ph | s. o. |
| I-8-10 | Formel (H-1) | n. v. | s. o. |
| I-8-11 | s. o. | m-Ph | s. o. |
| I-8-12 | s. o. | p-Ph | s. o. |
| I-8-13 | Formel (H-2) | n. v. | s. o. |
| I-8-14 | s. o. | m-Ph | s. o. |
| I-8-15 | s. o. | p-Ph | s. o. |
| I-8-16 | Formel (H-3) | n. v. | s. o. |
| I-8-17 | s. o. | m-Ph | s. o. |
| I-8-18 | s. o. | p-Ph | s. o. |
| I-8-19 | Formel (H-4) | n. v. | s. o. |
| I-8-20 | s. o. | m-Ph | s. o. |
| I-8-21 | s. o. | p-Ph | s. o. |
| I-8-22 | Formel (H-5) | n. v. | s. o. |
| I-8-23 | s. o. | m-Ph | s. o. |
| I-8-24 | s. o. | p-Ph | s. o. |
| I-8-25 | Formel (H-6) | n. v. | s. o. |
| I-8-26 | s. o. | m-Ph | s. o. |
| I-8-27 | s. o. | p-Ph | s. o. |
| I-8-28 | Formel (H-7) | n. v. | s. o. |
| I-8-29 | s. o. | m-Ph | s. o. |
| I-8-30 | s. o. | p-Ph | s. o. |
| I-8-31 | Formel (H-8) | n. v. | s. o. |
| I-8-32 | s. o. | m-Ph | s. o. |
| I-8-33 | s. o. | p-Ph | s. o. |
| I-8-34 | Formel (H-9) | n. v. | s. o. |
| I-8-35 | s. o. | m-Ph | s. o. |
| I-8-36 | s. o. | p-Ph | s. o. |
| I-8-37 | Formel (H-10) | n. v. | s. o. |
| I-8-38 | s. o. | m-Ph | s. o. |
| I-8-39 | s. o. | p-Ph | s. o. |
| I-9-1 | Formel (K) | n. v. | Formel (I-9) |
| I-9-2 | s. o. | m-Ph | s. o. |
| I-9-3 | s. o. | p-Ph | s. o. |
| I-9-4 | Formel (P) | n. v. | s. o. |
| I-9-5 | s. o. | m-Ph | s. o. |
| I-9-6 | s. o. | p-Ph | s. o. |
| I-9-7 | Formel (S) | n. v. | s. o. |
| I-9-8 | s. o. | m-Ph | s. o. |
| I-9-9 | s. o. | p-Ph | s. o. |
| I-9-10 | Formel (H-1) | n. v. | s. o. |
| I-9-11 | s. o. | m-Ph | s. o. |
| I-9-12 | s. o. | p-Ph | s. o. |
| I-9-13 | Formel (H-2) | n. v. | s. o. |
| I-9-14 | s. o. | m-Ph | s. o. |
| I-9-15 | s. o. | p-Ph | s. o. |
| I-9-16 | Formel (H-3) | n. v. | s. o. |
| I-9-17 | s. o. | m-Ph | s. o. |
| I-9-18 | s. o. | p-Ph | s. o. |
| I-9-19 | Formel (H-4) | n. v. | s. o. |
| I-9-20 | s. o. | m-Ph | s. o. |
| I-9-21 | s. o. | p-Ph | s. o. |
| I-9-22 | Formel (H-5) | n. v. | s. o. |
| I-9-23 | s. o. | m-Ph | s. o. |
| I-9-24 | s. o. | p-Ph | s. o. |
| I-9-25 | Formel (H-6) | n. v. | s. o. |
| I-9-26 | s. o. | m-Ph | s. o. |
| I-9-27 | s. o. | p-Ph | s. o. |
| I-9-28 | Formel (H-7) | n. v. | s. o. |
| I-9-29 | s. o. | m-Ph | s. o. |
| I-9-30 | S. O. | p-Ph | S. O. |
| I-9-31 | Formel (H-8) | n. v. | s. o. |
| I-9-32 | s. o. | m-Ph | s. o. |
| I-9-33 | s. o. | p-Ph | s. o. |
| I-9-34 | Formel (H-9) | n. v. | s. o. |
| I-9-35 | s. o. | m-Ph | s. o. |
| I-9-36 | s. o. | p-Ph | s. o. |
| I-9-37 | Formel (H-10) | n. v. | s. o. |
| I-9-38 | s. o. | m-Ph | s. o. |
| I-9-39 | s. o. | p-Ph | s. o. |
| I-10-1 | Formel (K) | n. v. | Formel (I-10) |
| I-10-2 | s. o. | m-Ph | s. o. |
| I-10-3 | s. o. | p-Ph | s. o. |
| I-10-4 | Formel (P) | n. v. | s. o. |
| I-10-5 | s. o. | m-Ph | s. o. |
| I-10-6 | s. o. | p-Ph | s. o. |
| I-10-7 | Formel (S) | n. v. | s. o. |
| I-10-8 | s. o. | m-Ph | s. o. |
| I-10-9 | s. o. | p-Ph | s. o. |
| I-10-10 | Formel (H-1) | n. v. | s. o. |
| I-10-11 | s. o. | m-Ph | s. o. |
| I-10-12 | s. o. | p-Ph | s. o. |
| I-10-13 | Formel (H-2) | n. v. | s. o. |
| I-10-14 | s. o. | m-Ph | s. o. |
| I-10-15 | s. o. | p-Ph | s. o. |
| I-10-16 | Formel (H-3) | n. v. | s. o. |
| I-10-17 | s. o. | m-Ph | s. o. |
| I-10-18 | s. o. | p-Ph | s. o. |
| I-10-19 | Formel (H-4) | n. v. | s. o. |
| I-10-20 | s. o. | m-Ph | s. o. |
| I-10-21 | s. o. | p-Ph | s. o. |
| I-10-22 | Formel (H-5) | n. v. | s. o. |
| I-10-23 | s. o. | m-Ph | s. o. |
| I-10-24 | s. o. | p-Ph | s. o. |
| I-10-25 | Formel (H-6) | n. v. | s. o. |
| I-10-26 | s. o. | m-Ph | s. o. |
| I-10-27 | s. o. | p-Ph | s. o. |
| I-10-28 | Formel (H-7) | n. v. | s. o. |
| I-10-29 | s. o. | m-Ph | s. o. |
| I-10-30 | s. o. | p-Ph | s. o. |
| I-10-31 | Formel (H-8) | n. v. | s. o. |
| I-10-32 | s. o. | m-Ph | s. o. |
| I-10-33 | s. o. | p-Ph | s. o. |
| I-10-34 | Formel (H-9) | n. v. | s. o. |
| I-10-35 | s. o. | m-Ph | s. o. |
| I-10-36 | s. o. | p-Ph | s. o. |
| I-10-37 | Formel (H-10) | n. v. | s. o. |
| I-10-38 | s. o. | m-Ph | s. o. |
| I-10-39 | s. o. | p-Ph | s. o. |
| I-11-1 | Formel (K) | n. v. | Formel (I-11) |
| I-11-2 | s. o. | m-Ph | s. o. |
| I-11-3 | s. o. | p-Ph | s. o. |
| I-11-4 | Formel (P) | n. v. | s. o. |
| I-11-5 | s. o. | m-Ph | s. o. |
| I-11-6 | s. o. | p-Ph | s. o. |
| I-11-7 | Formel (S) | n. v. | s. o. |
| I-11-8 | s. o. | m-Ph | s. o. |
| I-11-9 | s. o. | p-Ph | s. o. |
| I-11-10 | Formel (H-1) | n. v. | s. o. |
| I-11-11 | s. o. | m-Ph | s. o. |
| I-11-12 | s. o. | p-Ph | s. o. |
| I-11-13 | Formel (H-2) | n. v. | s. o. |
| I-11-14 | s. o. | m-Ph | s. o. |
| I-11-15 | s. o. | p-Ph | s. o. |
| I-11-16 | Formel (H-3) | n. v. | s. o. |
| I-11-17 | s. o. | m-Ph | s. o. |
| I-11-18 | s. o. | p-Ph | s. o. |
| I-11-19 | Formel (H-4) | n. v. | s. o. |
| I-11-20 | s. o. | m-Ph | s. o. |
| I-11-21 | s. o. | p-Ph | s. o. |
| I-11-22 | Formel (H-5) | n. v. | s. o. |
| I-11-23 | s. o. | m-Ph | s. o. |
| I-11-24 | s. o. | p-Ph | s. o. |
| I-11-25 | Formel (H-6) | n. v. | s. o. |
| I-11-26 | s. o. | m-Ph | s. o. |
| I-11-27 | s. o. | p-Ph | s. o. |
| I-11-28 | Formel (H-7) | n. v. | s. o. |
| I-11-29 | s. o. | m-Ph | s. o. |
| I-11-30 | s. o. | p-Ph | s. o. |
| I-11-31 | Formel (H-8) | n. v. | s. o. |
| I-11-32 | s. o. | m-Ph | s. o. |
| I-11-33 | s. o. | p-Ph | s. o. |
| I-11-34 | Formel (H-9) | n. v. | s. o. |
| I-11-35 | s. o. | m-Ph | s. o. |
| I-11-36 | s. o. | p-Ph | s. o. |
| I-11-37 | Formel (H-10) | n. v. | s. o. |
| I-11-38 | s. o. | m-Ph | s. o. |
| I-11-39 | s. o. | p-Ph | s. o. |
| I-12-1 | Formel (K) | n. v. | Formel (I-12) |
| I-12-2 | s. o. | m-Ph | s. o. |
| I-12-3 | s. o. | p-Ph | s. o. |
| I-12-4 | Formel (P) | n. v. | s. o. |
| I-12-5 | s. o. | m-Ph | s. o. |
| I-12-6 | s. o. | p-Ph | s. o. |
| I-12-7 | Formel (S) | n. v. | s. o. |
| I-12-8 | s. o. | m-Ph | s. o. |
| I-12-9 | s. o. | p-Ph | s. o. |
| I-12-10 | Formel (H-1) | n. v. | s. o. |
| I-12-11 | s. o. | m-Ph | s. o. |
| I-12-12 | s. o. | p-Ph | s. o. |
| I-12-13 | Formel (H-2) | n. v. | s. o. |
| I-12-14 | s. o. | m-Ph | s. o. |
| I-12-15 | s. o. | p-Ph | s. o. |
| I-12-16 | Formel (H-3) | n. v. | s. o. |
| I-12-17 | s. o. | m-Ph | s. o. |
| I-12-18 | s. o. | p-Ph | s. o. |
| I-12-19 | Formel (H-4) | n. v. | s. o. |
| I-12-20 | s. o. | m-Ph | s. o. |
| I-12-21 | s. o. | p-Ph | s. o. |
| I-12-22 | Formel (H-5) | n. v. | s. o. |
| I-12-23 | s. o. | m-Ph | s. o. |
| I-12-24 | s. o. | p-Ph | s. o. |
| I-12-25 | Formel (H-6) | n. v. | s. o. |
| I-12-26 | s. o. | m-Ph | s. o. |
| I-12-27 | s. o. | p-Ph | s. o. |
| I-12-28 | Formel (H-7) | n. v. | s. o. |
| I-12-29 | s. o. | m-Ph | s. o. |
| I-12-30 | s. o. | p-Ph | s. o. |
| I-12-31 | Formel (H-8) | n. v. | s. o. |
| I-12-32 | s. o. | m-Ph | s. o. |
| I-12-33 | s. o. | p-Ph | s. o. |
| I-12-34 | Formel (H-9) | n. v. | s. o. |
| I-12-35 | s. o. | m-Ph | s. o. |
| I-12-36 | s. o. | p-Ph | s. o. |
| I-12-37 | Formel (H-10) | n. v. | s. o. |
| I-12-38 | s. o. | m-Ph | s. o. |
| I-12-39 | s. o. | p-Ph | s. o. |

Dabei sind die angegebenen Formeln und die darin auftretenden Symbole wie oben definiert. Weiterhin steht p-Ph für para-Phenylen und m-Ph für meta-Phenylen.

Besonders bevorzugte Ausführungsformen der Formel (I) sind die im Folgenden angegebenen Formeln (I-1-A) bis (I-12-A) 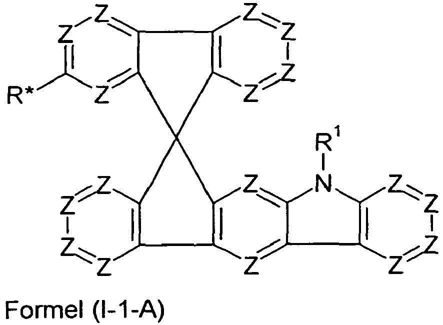 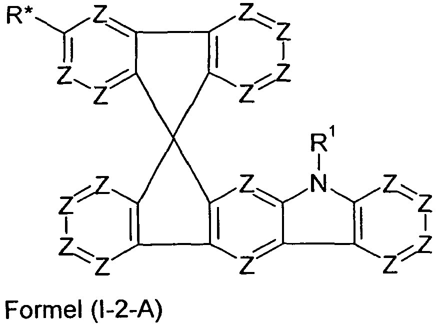 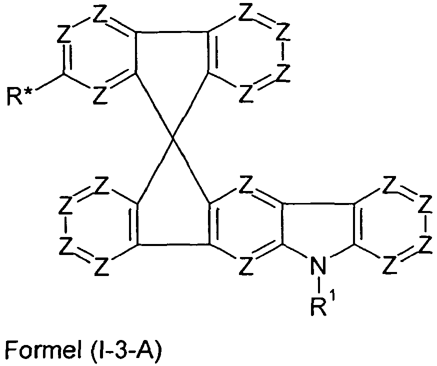 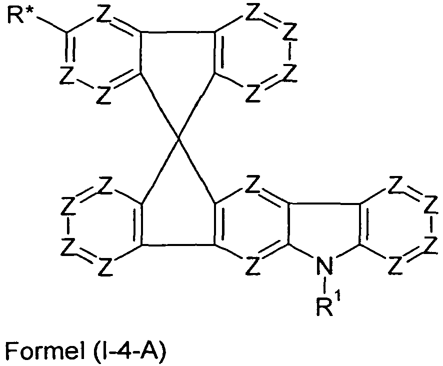 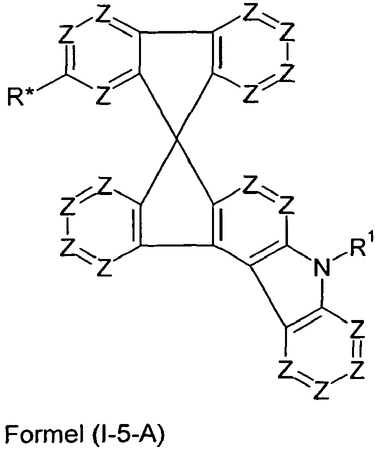 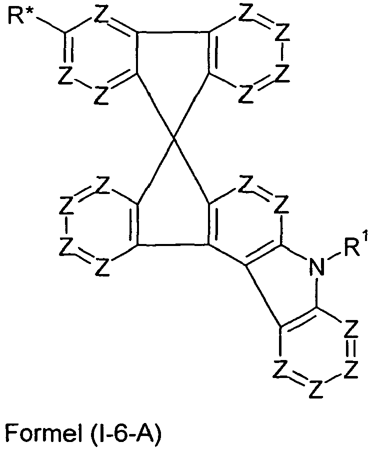 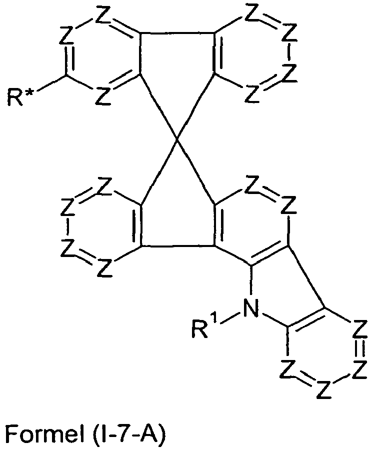 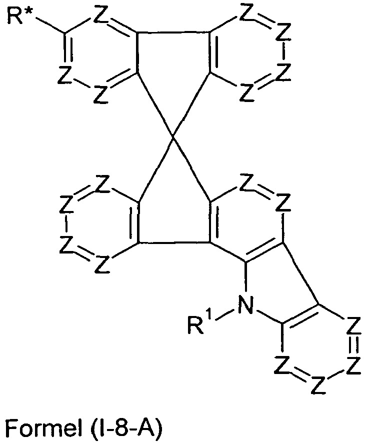 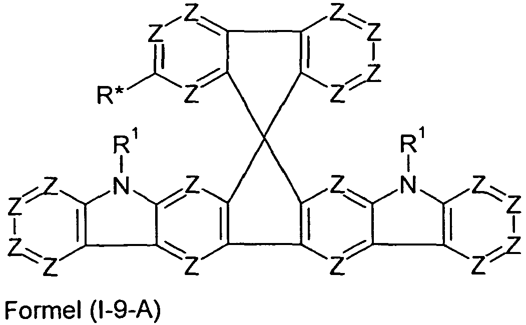 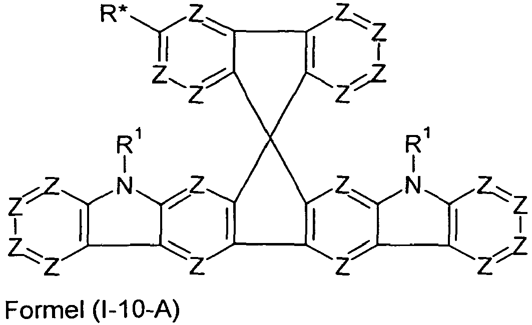 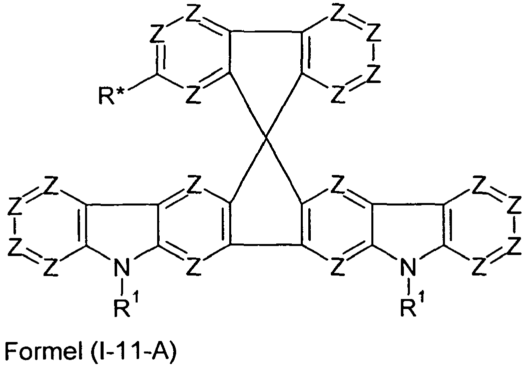 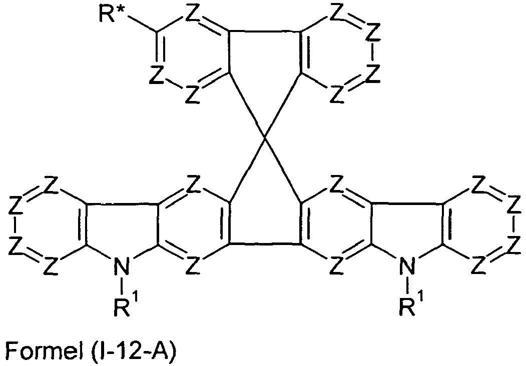 wobei die auftretenden Symbole wie obenstehend definiert sind und bevorzugt in den oben angegebenen bevorzugten Ausführungsformen vorliegen.

Weiterhin ist es bevorzugt, dass nicht mehr als eine Gruppe Z pro aromatischen Ring gleich N ist und die anderen Gruppen Z gleich CR¹ sind. Besonders bevorzugt ist keine Gruppe Z gleich N, so dass alle Gruppen Z gleich CR¹ sind.

Weiterhin bevorzugt stellt der Rest R¹, der an das Stickstoffatom gebunden ist, in den Formeln (I-1-A) bis (I-12-A) keine Heteroarylgruppe mit 6 aromatischen Ringatomen dar. Besonders bevorzugt ist dieser Rest R¹ keine Heteroarylgruppe, keine Ketogruppe, keine Phosphoroxidgruppe und keine Schwefeloxidgruppe.

Die in einer obenstehenden Tabelle angegebenen Kombinationen von Gruppen R* mit Grundgerüsten der Formeln (I-1) bis (I-12) sind identisch bevorzugt für Grundgerüste der Formeln (I-1-A) bis (I-12-A).

Bevorzugte Ausführungsformen der Verbindung gemäß Formel (II) entsprechen einer der folgenden Formeln (II-1) bis (II-6) 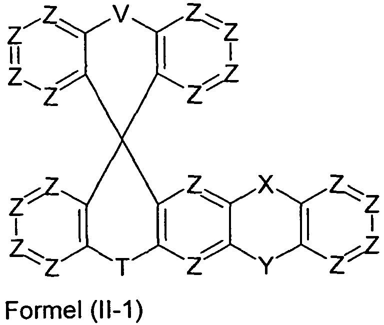 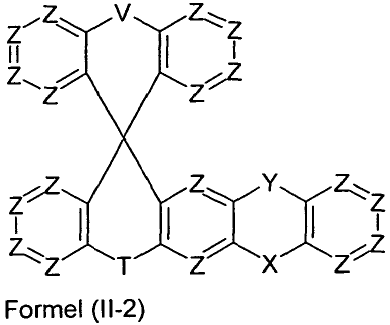 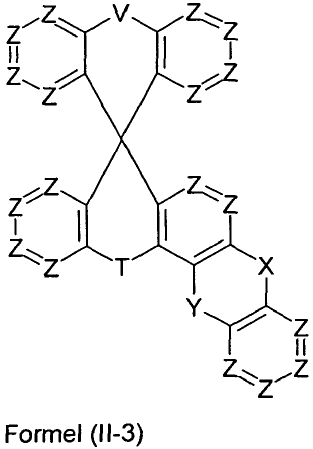 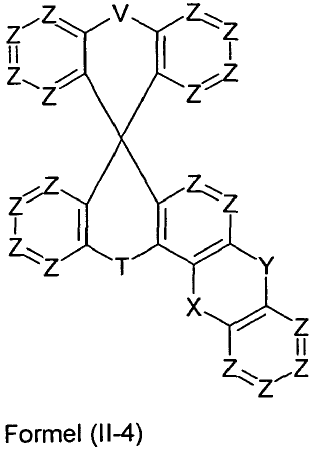 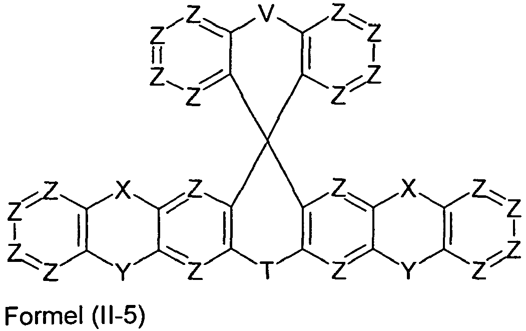 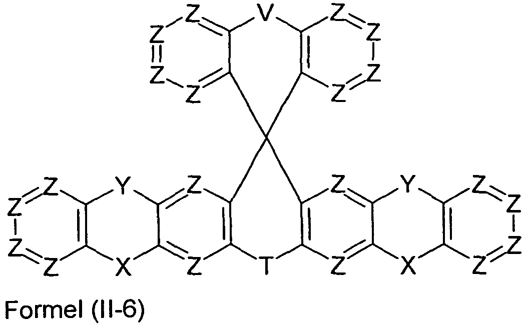 wobei die auftretenden Symbole wie obenstehend definiert sind und bevorzugt in ihren oben angegebenen bevorzugten Ausführungsformen vorliegen.

Besonders bevorzugt ist für die Formeln (II-1) bis (II-6) die Gruppe T eine Einfachbindung oder eine Gruppe C(R¹)₂.

Weiterhin ist es bevorzugt, dass nicht mehr als eine Gruppe Z pro aromatischen Ring gleich N ist und die anderen Gruppen Z gleich CR¹ sind. Besonders bevorzugt ist keine Gruppe Z gleich N, so dass alle Gruppen Z gleich CR¹ sind.

Weiterhin bevorzugt ist an die Gruppen X und Y in den Formeln (II-1) bis (II-6) keine Heteroarylgruppe mit 6 aromatischen Ringatomen gebunden. Besonders bevorzugt ist an die Gruppen X und Y keine Heteroarylgruppe, keine Ketogruppe, keine Phosphoroxidgruppe und keine Schwefeloxidgruppe gebunden.

Weiterhin bevorzugt sind für die Formeln (II-1) bis (II-6) die in einer obenstehenden Tabelle angegebenen Kombinationen von Ausführungsformen von Gruppen X und Y.

Weiterhin sind die folgenden Kombinationen von Gruppen R* mit Grundgerüsten der Formeln (II-1) bis (II-6) bevorzugt:

| Formel | R* | L in R* | Grundgerüst |
|---|---|---|---|
| II-1-1 | Formel (K) | nicht vorhanden (n. v.) | Formel (II-1) |
| II-1-2 | siehe oben (s. o.) | m-Ph | s. o. |
| II-1-3 | s. o. | p-Ph | s. o. |
| II-1-4 | Formel (P) | n. v. | s. o. |
| II-1-5 | s. o. | m-Ph | s. o. |
| II-1-6 | s. o. | p-Ph | s. o. |
| II-1-7 | Formel (S) | n. v. | s. o. |
| II-1-8 | s. o. | m-Ph | s. o. |
| II-1-9 | s. o. | p-Ph | s. o. |
| II-1-10 | Formel (H-1) | n. v. | s. o. |
| II-1-11 | s. o. | m-Ph | s. o. |
| II-1-12 | s. o. | p-Ph | s. o. |
| II-1-13 | Formel (H-2) | n. v. | s. o. |
| II-1-14 | s. o. | m-Ph | s. o. |
| II-1-15 | s. o. | p-Ph | s. o. |
| II-1-16 | Formel (H-3) | n. v. | s. o. |
| II-1-17 | s. o. | m-Ph | s. o. |
| II-1-18 | s. o. | p-Ph | s. o. |
| II-1-19 | Formel (H-4) | n. v. | s. o. |
| II-1-20 | s. o. | m-Ph | s. o. |
| II-1-21 | s. o. | p-Ph | s. o. |
| II-1-22 | Formel (H-5) | n. v. | s. o. |
| II-1-23 | s. o. | m-Ph | s. o. |
| II-1-24 | s. o. | p-Ph | s. o. |
| II-1-25 | Formel (H-6) | n. v. | s. o. |
| II-1-26 | s. o. | m-Ph | s. o. |
| II-1-27 | s. o. | p-Ph | s. o. |
| II-1-28 | Formel (H-7) | n. v. | s. o. |
| II-1-29 | s. o. | m-Ph | s. o. |
| II-1-30 | s. o. | p-Ph | s. o. |
| II-1-31 | Formel (H-8) | n. v. | s. o. |
| II-1-32 | s. o. | m-Ph | s. o. |
| II-1-33 | s. o. | p-Ph | s. o. |
| II-1-34 | Formel (H-9) | n. v. | s. o. |
| II-1-35 | s. o. | m-Ph | s. o. |
| II-1-36 | s. o. | p-Ph | s. o. |
| II-1-37 | Formel (H-10) | n. v. | s. o. |
| II-1-38 | s. o. | m-Ph | s. o. |
| II-1-39 | s. o. | p-Ph | s. o. |
| II-2-1 | Formel (K) | n. v. | Formel (II-2) |
| II-2-2 | s. o. | m-Ph | s. o. |
| II-2-3 | s. o. | p-Ph | s. o. |
| II-2-4 | Formel (P) | n. v. | s. o. |
| II-2-5 | s. o. | m-Ph | s. o. |
| II-2-6 | s. o. | p-Ph | s. o. |
| II-2-7 | Formel (S) | n. v. | s. o. |
| II-2-8 | s. o. | m-Ph | s. o. |
| II-2-9 | s. o. | p-Ph | s. o. |
| II-2-10 | Formel (H-1) | n. v. | s. o. |
| II-2-11 | s. o. | m-Ph | s. o. |
| II-2-12 | s. o. | p-Ph | s. o. |
| II-2-13 | Formel (H-2) | n. v. | s. o. |
| II-2-14 | s. o. | m-Ph | s. o. |
| II-2-15 | s. o. | p-Ph | s. o. |
| II-2-16 | Formel (H-3) | n. v. | s. o. |
| II-2-17 | s. o. | m-Ph | s. o. |
| II-2-18 | s. o. | p-Ph | s. o. |
| II-2-19 | Formel (H-4) | n. v. | s. o. |
| II-2-20 | s. o. | m-Ph | s. o. |
| II-2-21 | s. o. | p-Ph | s. o. |
| II-2-22 | Formel (H-5) | n. v. | s. o. |
| II-2-23 | s. o. | m-Ph | s. o. |
| II-2-24 | s. o. | p-Ph | s. o. |
| II-2-25 | Formel (H-6) | n. v. | s. o. |
| II-2-26 | s. o. | m-Ph | s. o. |
| II-2-27 | s. o. | p-Ph | s. o. |
| II-2-28 | Formel (H-7) | n. v. | s. o. |
| II-2-29 | s. o. | m-Ph | s. o. |
| II-2-30 | s. o. | p-Ph | s. o. |
| II-2-31 | Formel (H-8) | n. v. | s. o. |
| II-2-32 | s. o. | m-Ph | s. o. |
| II-2-33 | s. o. | p-Ph | s. o. |
| II-2-34 | Formel (H-9) | n. v. | s. o. |
| II-2-35 | s. o. | m-Ph | s. o. |
| II-2-36 | s. o. | p-Ph | s. o. |
| II-2-37 | Formel (H-10) | n. v. | s. o. |
| II-2-38 | s. o. | m-Ph | s. o. |
| II-2-39 | s. o. | p-Ph | s. o. |
| II-3-1 | Formel (K) | n. v. | Formel (II-3) |
| II-3-2 | s. o. | m-Ph | s. o. |
| II-3-3 | s. o. | p-Ph | s. o. |
| II-3-4 | Formel (P) | n. v. | s. o. |
| II-3-5 | s. o. | m-Ph | s. o. |
| II-3-6 | s. o. | p-Ph | s. o. |
| II-3-7 | Formel (S) | n. v. | s. o. |
| II-3-8 | s. o. | m-Ph | s. o. |
| II-3-9 | s. o. | p-Ph | s. o. |
| II-3-10 | Formel (H-1) | n. v. | s. o. |
| II-3-11 | s. o. | m-Ph | s. o. |
| II-3-12 | s. o. | p-Ph | s. o. |
| II-3-13 | Formel (H-2) | n. v. | s. o. |
| II-3-14 | s. o. | m-Ph | s. o. |
| II-3-15 | s. o. | p-Ph | s. o. |
| II-3-16 | Formel (H-3) | n. v. | s. o. |
| II-3-17 | s. o. | m-Ph | s. o. |
| II-3-18 | s. o. | p-Ph | s. o. |
| II-3-19 | Formel (H-4) | n. v. | s. o. |
| II-3-20 | s. o. | m-Ph | s. o. |
| II-3-21 | s. o. | p-Ph | s. o. |
| II-3-22 | Formel (H-5) | n. v. | s. o. |
| II-3-23 | s. o. | m-Ph | s. o. |
| II-3-24 | s. o. | p-Ph | s. o. |
| II-3-25 | Formel (H-6) | n. v. | s. o. |
| II-3-26 | s. o. | m-Ph | s. o. |
| II-3-27 | s. o. | p-Ph | s. o. |
| II-3-28 | Formel (H-7) | n. v. | s. o. |
| II-3-29 | s. o. | m-Ph | s. o. |
| II-3-30 | s. o. | p-Ph | s. o. |
| II-3-31 | Formel (H-8) | n. v. | s. o. |
| II-3-32 | s. o. | m-Ph | s. o. |
| II-3-33 | s. o. | p-Ph | s. o. |
| II-3-34 | Formel (H-9) | n. v. | s. o. |
| II-3-35 | s. o. | m-Ph | s. o. |
| II-3-36 | s. o. | p-Ph | s. o. |
| II-3-37 | Formel (H-10) | n. v. | s. o. |
| II-3-38 | s. o. | m-Ph | s. o. |
| II-3-39 | s. o. | p-Ph | s. o. |
| II-4-1 | Formel (K) | n. v. | Formel (II-4) |
| II-4-2 | s. o. | m-Ph | s. o. |
| II-4-3 | s. o. | p-Ph | s. o. |
| II-4-4 | Formel (P) | n. v. | s. o. |
| II-4-5 | s. o. | m-Ph | s. o. |
| II-4-6 | s. o. | p-Ph | s. o. |
| II-4-7 | Formel (S) | n. v. | s. o. |
| II-4-8 | s. o. | m-Ph | s. o. |
| II-4-9 | s. o. | p-Ph | s. o. |
| II-4-10 | Formel (H-1) | n. v. | s. o. |
| II-4-11 | s. o. | m-Ph | s. o. |
| II-4-12 | s. o. | p-Ph | s. o. |
| II-4-13 | Formel (H-2) | n. v. | s. o. |
| II-4-14 | s. o. | m-Ph | s. o. |
| II-4-15 | s. o. | p-Ph | s. o. |
| II-4-16 | Formel (H-3) | n. v. | s. o. |
| II-4-17 | s. o. | m-Ph | s. o. |
| II-4-18 | s. o. | p-Ph | s. o. |
| II-4-19 | Formel (H-4) | n. v. | s. o. |
| II-4-20 | s. o. | m-Ph | s. o. |
| II-4-21 | s. o. | p-Ph | s. o. |
| II-4-22 | Formel (H-5) | n. v. | s. o. |
| II-4-23 | s. o. | m-Ph | s. o. |
| II-4-24 | s. o. | p-Ph | s. o. |
| II-4-25 | Formel (H-6) | n. v. | s. o. |
| II-4-26 | s. o. | m-Ph | s. o. |
| II-4-27 | s. o. | p-Ph | s. o. |
| II-4-28 | Formel (H-7) | n. v. | s. o. |
| II-4-29 | s. o. | m-Ph | s. o. |
| II-4-30 | s. o. | p-Ph | s. o. |
| II-4-31 | Formel (H-8) | n. v. | s. o. |
| II-4-32 | s. o. | m-Ph | s. o. |
| II-4-33 | s. o. | p-Ph | s. o. |
| II-4-34 | Formel (H-9) | n. v. | s. o. |
| II-4-35 | s. o. | m-Ph | s. o. |
| II-4-36 | s. o. | p-Ph | s. o. |
| II-4-37 | Formel (H-10) | n. v. | s. o. |
| II-4-38 | s. o. | m-Ph | s. o. |
| II-4-39 | s. o. | p-Ph | s. o. |
| II-5-1 | Formel (K) | n. v. | Formel (II-5) |
| II-5-2 | s. o. | m-Ph | s. o. |
| II-5-3 | s. o. | p-Ph | s. o. |
| II-5-4 | Formel (P) | n. v. | s. o. |
| II-5-5 | s. o. | m-Ph | s. o. |
| II-5-6 | s. o. | p-Ph | s. o. |
| II-5-7 | Formel (S) | n. v. | s. o. |
| II-5-8 | s. o. | m-Ph | s. o. |
| II-5-9 | s. o. | p-Ph | s. o. |
| II-5-10 | Formel (H-1) | n. v. | s. o. |
| II-5-11 | s. o. | m-Ph | s. o. |
| II-5-12 | s. o. | p-Ph | s. o. |
| II-5-13 | Formel (H-2) | n. v. | s. o. |
| II-5-14 | s. o. | m-Ph | s. o. |
| II-5-15 | s. o. | p-Ph | s. o. |
| II-5-16 | Formel (H-3) | n. v. | s. o. |
| II-5-17 | s. o. | m-Ph | s. o. |
| II-5-18 | s. o. | p-Ph | s. o. |
| II-5-19 | Formel (H-4) | n. v. | s. o. |
| II-5-20 | s. o. | m-Ph | s. o. |
| II-5-21 | s. o. | p-Ph | s. o. |
| II-5-22 | Formel (H-5) | n. v. | s. o. |
| II-5-23 | s. o. | m-Ph | s. o. |
| II-5-24 | s. o. | p-Ph | s. o. |
| II-5-25 | Formel (H-6) | n. v. | s. o. |
| II-5-26 | s. o. | m-Ph | s. o. |
| II-5-27 | s. o. | p-Ph | s. o. |
| II-5-28 | Formel (H-7) | n. v. | s. o. |
| II-5-29 | s. o. | m-Ph | s. o. |
| II-5-30 | s. o. | p-Ph | s. o. |
| II-5-31 | Formel (H-8) | n. v. | s. o. |
| II-5-32 | s. o. | m-Ph | s. o. |
| II-5-33 | s. o. | p-Ph | s. o. |
| II-5-34 | Formel (H-9) | n. v. | s. o. |
| II-5-35 | s. o. | m-Ph | s. o. |
| II-5-36 | s. o. | p-Ph | s. o. |
| II-5-37 | Formel (H-10) | n. v. | s.o. |
| II-5-38 | s. o. | m-Ph | s. o. |
| II-5-39 | s. o. | p-Ph | s. o. |
| II-6-1 | Formel (K) | n. v. | Formel (II-6) |
| II-6-2 | s. o. | m-Ph | s. o. |
| II-6-3 | s. o. | p-Ph | s. o. |
| II-6-4 | Formel (P) | n. v. | s. o. |
| II-6-5 | s. o. | m-Ph | s. o. |
| II-6-6 | s. o. | p-Ph | s. o. |
| II-6-7 | Formel (S) | n. v. | s. o. |
| II-6-8 | s. o. | m-Ph | s. o. |
| II-6-9 | s. o. | p-Ph | s. o. |
| II-6-10 | Formel (H-1) | n. v. | s. o. |
| II-6-11 | s. o. | m-Ph | s. o. |
| II-6-12 | s. o. | p-Ph | s. o. |
| II-6-13 | Formel (H-2) | n. v. | s. o. |
| II-6-14 | s. o. | m-Ph | s. o. |
| II-6-15 | s. o. | p-Ph | s. o. |
| II-6-16 | Formel (H-3) | n. v. | s. o. |
| II-6-17 | s. o. | m-Ph | s. o. |
| II-6-18 | s. o. | p-Ph | s. o. |
| II-6-19 | Formel (H-4) | n. v. | s. o. |
| II-6-20 | s. o. | m-Ph | s. o. |
| II-6-21 | s. o. | p-Ph | s. o. |
| II-6-22 | Formel (H-5) | n. v. | s. o. |
| II-6-23 | s. o. | m-Ph | s. o. |
| II-6-24 | s. o. | p-Ph | s. o. |
| II-6-25 | Formel (H-6) | n. v. | s. o. |
| II-6-26 | s. o. | m-Ph | s. o. |
| II-6-27 | s. o. | p-Ph | s. o. |
| II-6-28 | Formel (H-7) | n. v. | s. o. |
| II-6-29 | s. o. | m-Ph | s. o. |
| II-6-30 | s. o. | p-Ph | s. o. |
| II-6-31 | Formel (H-8) | n. v. | s. o. |
| II-6-32 | s. o. | m-Ph | s. o. |
| II-6-33 | s. o. | p-Ph | s. o. |
| II-6-34 | Formel (H-9) | n. v. | s. o. |
| II-6-35 | s. o. | m-Ph | s. o. |
| II-6-36 | s. o. | p-Ph | s. o. |
| II-6-37 | Formel (H-10) | n. v. | s. o. |
| II-6-38 | s. o. | m-Ph | s. o. |
| II-6-39 | s. o. | p-Ph | s. o. |

Dabei sind die angegebenen Formeln und die darin auftretenden Symbole wie oben definiert. Weiterhin steht p-Ph für para-Phenylen und m-Ph für meta-Phenylen.

Besonders bevorzugte Ausführungsformen der Formel (I) sind die im Folgenden angegebenen Formeln (II-1-A) bis (II-6-A) 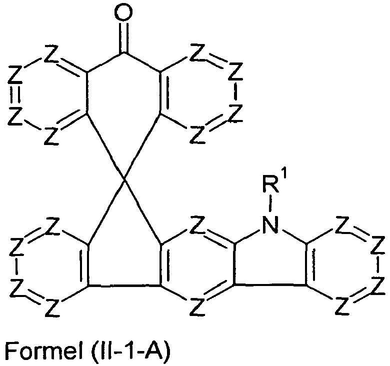 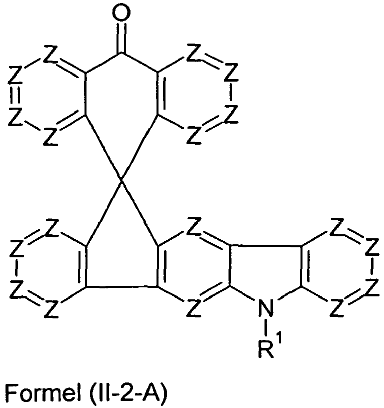 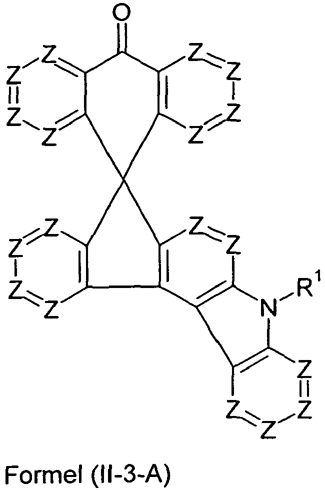 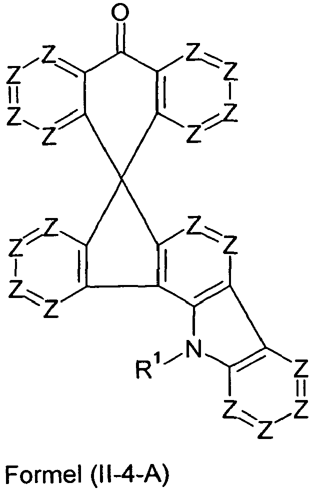 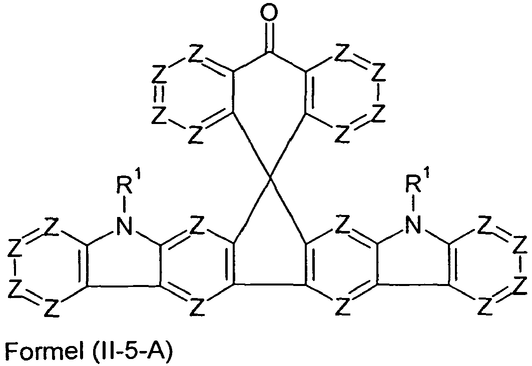 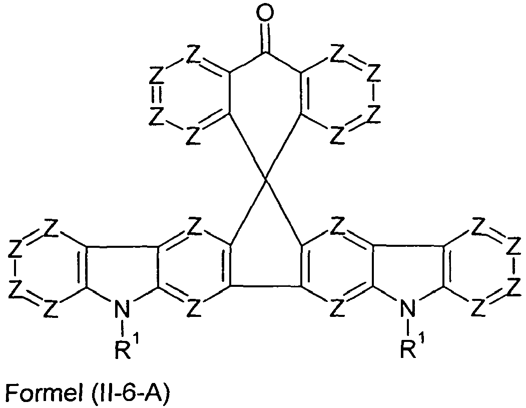 wobei die auftretenden Symbole wie obenstehend definiert sind und bevorzugt in den oben angegebenen bevorzugten Ausführungsformen vorliegen.

Weiterhin ist es bevorzugt, dass nicht mehr als eine Gruppe Z pro aromatischen Ring gleich N ist und die anderen Gruppen Z gleich CR¹ sind. Besonders bevorzugt ist keine Gruppe Z gleich N, so dass alle Gruppen Z gleich CR¹ sind.

Weiterhin bevorzugt stellt der Rest R¹, der an das Stickstoffatom gebunden ist, in den Formeln (II-1-A) bis (II-6-A) keine Heteroarylgruppe mit 6 aromatischen Ringatomen dar. Besonders bevorzugt ist dieser Rest R¹ keine Heteroarylgruppe, keine Ketogruppe, keine Phosphoroxidgruppe und keine Schwefeloxidgruppe.

Die in einer obenstehenden Tabelle angegebenen Kombinationen von Gruppen R* mit Grundgerüsten der Formeln (II-1) bis (II-6) sind identisch bevorzugt für Grundgerüste der Formeln (II-1-A) bis (II-6-A).

Beispiele für Verbindungen gemäß Formel (I) oder (II) werden in der folgenden Tabelle gegeben:

| | |
|---|---|
| 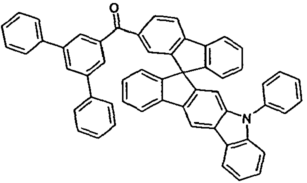 | 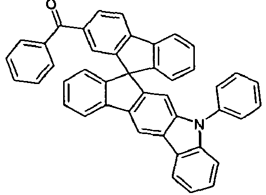 |
| 1 | 2 |
| 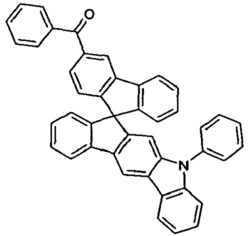 | 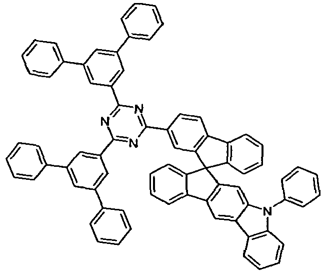 |
| 3 | 4 |
| 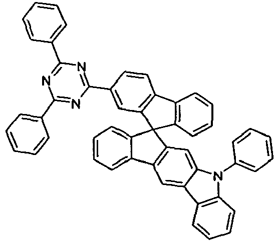 | 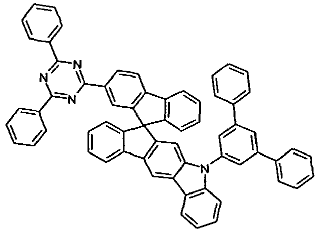 |
| 5 | 6 |
| 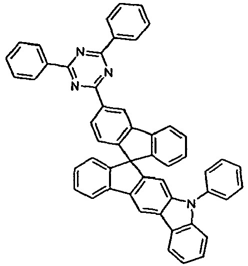 | 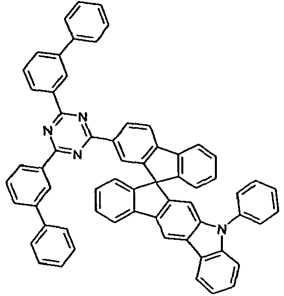 |
| 7 | 8 |
| 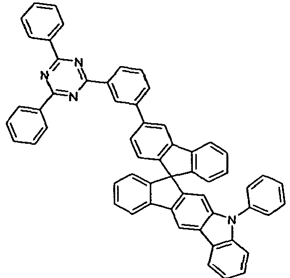 | 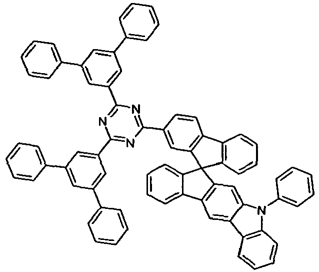 |
| 9 | 10 |
| 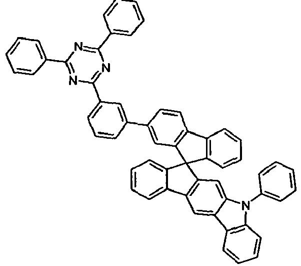 | 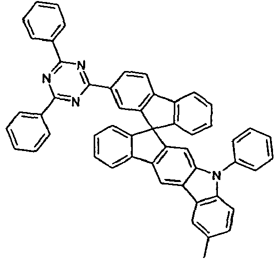 |
| 11 | 12 |
| 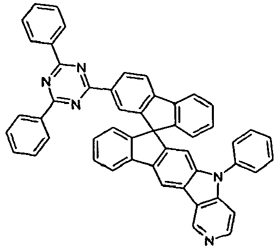 | 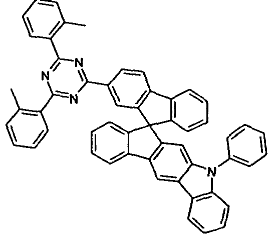 |
| 13 | 14 |
| 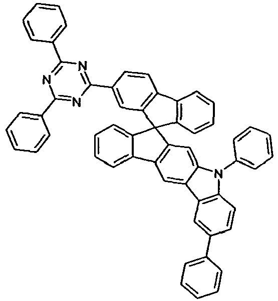 | 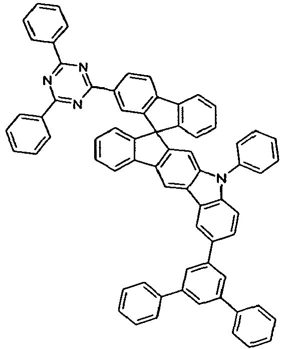 |
| 15 | 16 |
| 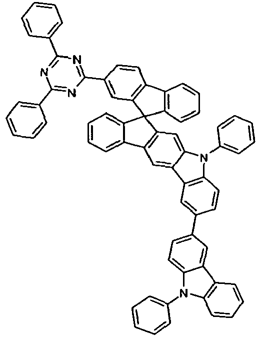 | 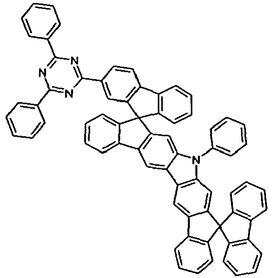 |
| 17 | 18 |
| 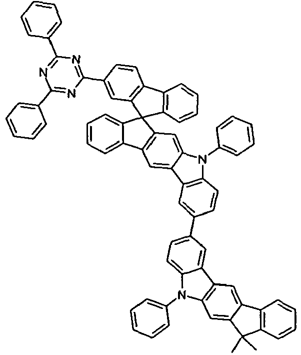 | 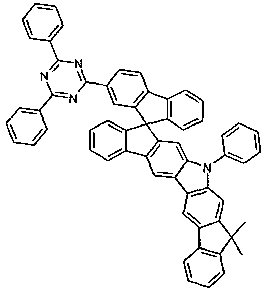 |
| 19 | 20 |
| 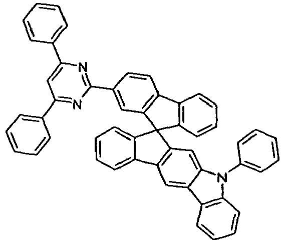 | 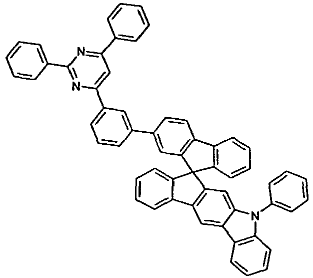 |
| 21 | 22 |
| 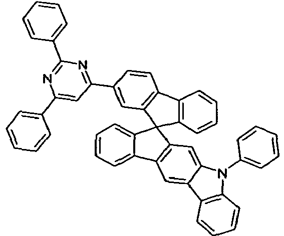 | 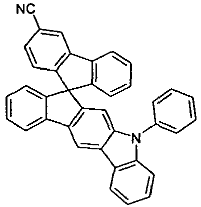 |
| 23 | 24 |
| 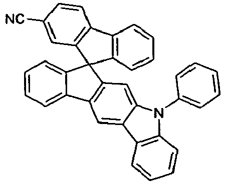 | 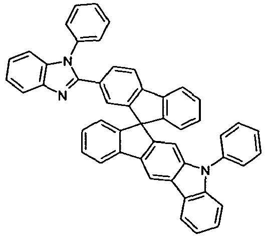 |
| 25 | 26 |
| 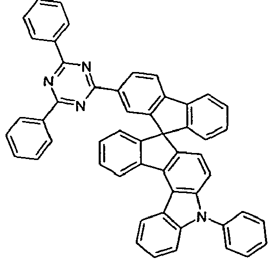 | 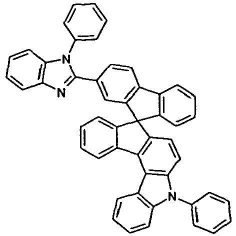 |
| 27 | 28 |
| 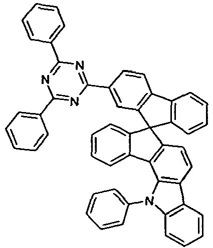 | 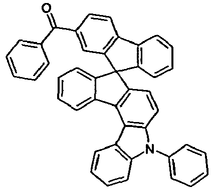 |
| 29 | 30 |
| 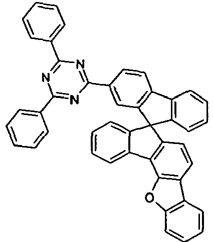 | 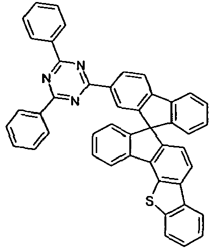 |
| 31 | 32 |
| 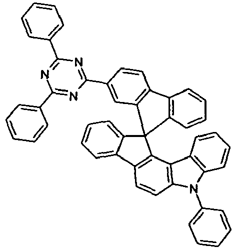 | 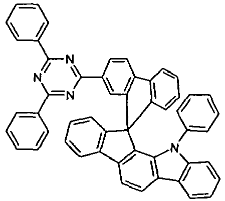 |
| 33 | 34 |
| 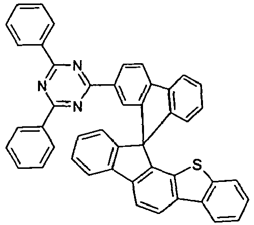 | 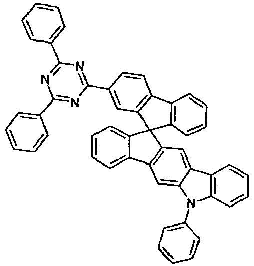 |
| 35 | 36 |
| 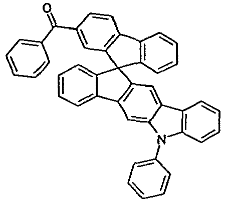 | 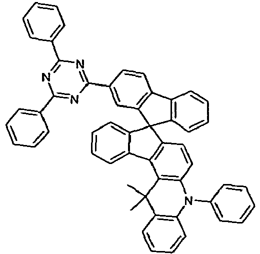 |
| 37 | 38 |
| 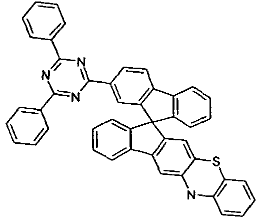 | 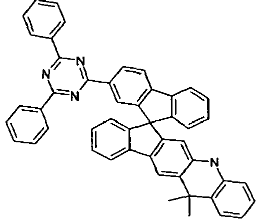 |
| 39 | 40 |
| 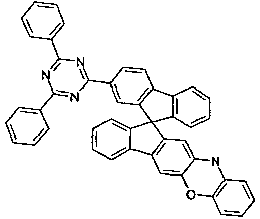 | 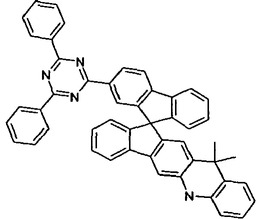 |
| 41 | 42 |
| 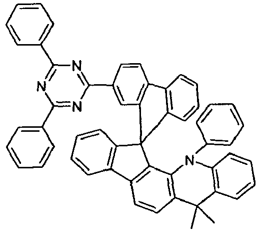 | 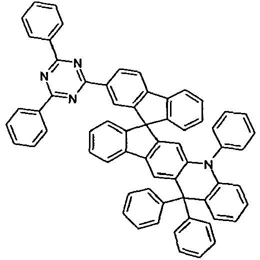 |
| 43 | 44 |
| 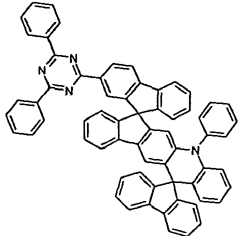 | 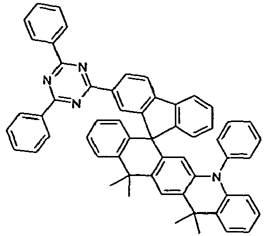 |
| 45 | 46 |
| 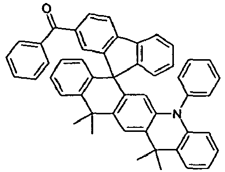 | 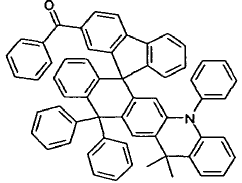 |
| 47 | 48 |
| 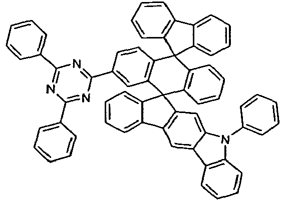 | 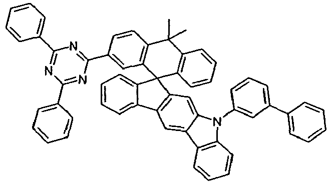 |
| 49 | 50 |
| 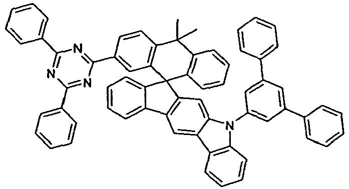 | 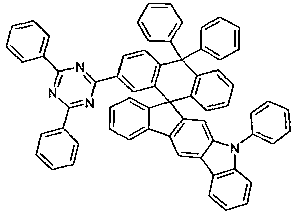 |
| 51 | 52 |
| 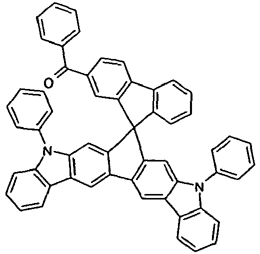 | 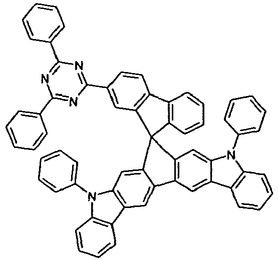 |
| 53 | 54 |
| 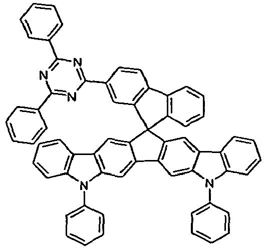 | 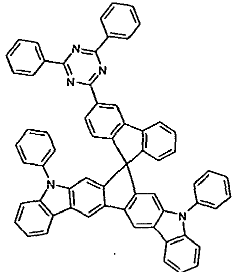 |
| 55 | 56 |
| 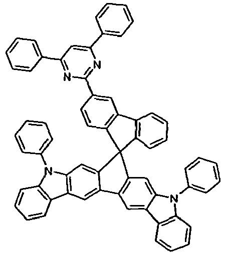 | 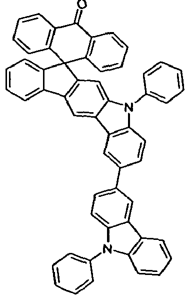 |
| 57 | 58 |
| 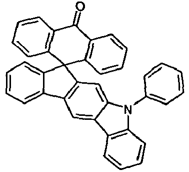 | 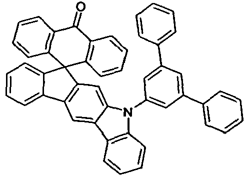 |
| 59 | 60 |
| 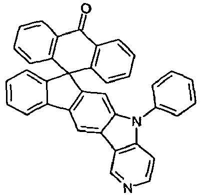 | 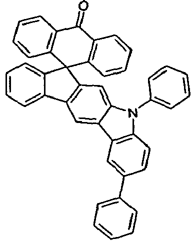 |
| 61 | 62 |
| 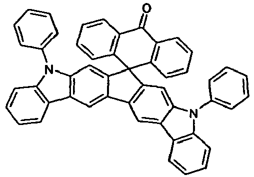 | 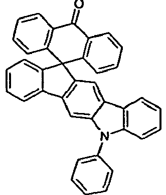 |
| 63 | 64 |
| 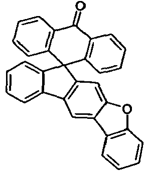 | 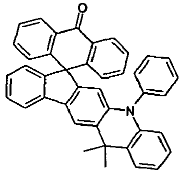 |
| 65 | 66 |
| 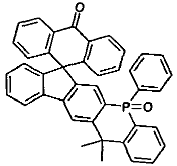 | 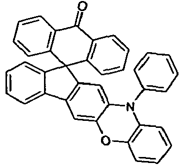 |
| 67 | 68 |
| 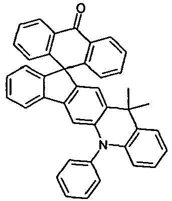 | 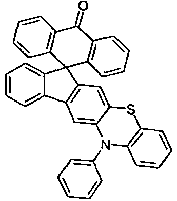 |
| 69 | 70 |
| 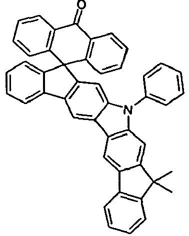 | 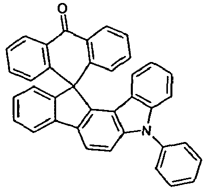 |
| 71 | 72 |
| 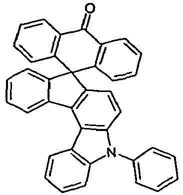 | 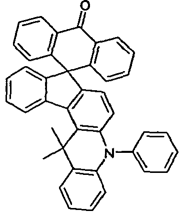 |
| 73 | 74 |
| 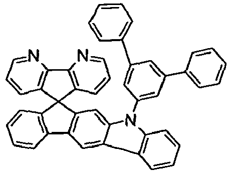 | 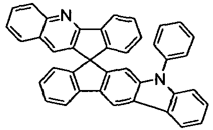 |
| 75 | 76 |

Die Synthese der erfindungsgemäßen Verbindungen kann gemäß im Stand der Technik bekannten Verfahren und Reaktionstypen, beispielsweise Halogenierung, Buchwald-Kupplung und Suzuki-Kupplung erfolgen.

Zwei bevorzugte Syntheseverfahren zur Herstellung der erfindungsgemäßen Verbindungen sollen im Folgenden vorgestellt werden.

Der Fachmann ist zur Synthese der erfindungsgemäßen Verbindungen nicht auf die an dieser Stelle offenbarten Verfahren angewiesen, sondern kann im Rahmen seines Fachwissens auf dem Gebiet der organischen Synthese alternative Synthesewege entwickeln und anwenden.

Gemäß dem in Schema 1 gezeigten Syntheseweg wird von einer Spirobifluorenverbindung ausgegangen, welche eine reaktive Gruppe, beispielsweise eine Halogengruppe, und einen Rest R* trägt. Der Rest R* stellt eine elektronenarme Gruppe, beispielsweise eine Triazingruppe, ein Keton oder ein Phosphinoxid dar. Ausgangsverbindungen dieser Art sind vielfach in einem oder wenigen Schritten aus kommerziell erhältlichen Verbindungen herstellbar. Explizite Synthesewege für solche Verbindungen werden in den Ausführungsbeispielen gezeigt.

In den zwei folgenden Schritten wird nun über eine Buchwald-Kupplung und anschließende Palladium-katalysierte Zyklisierung die ankondensierte Indol-Gruppe eingeführt. Anstelle dieser Reaktionssequenz können über abweichende Reaktionen auch andere Heteroarylgruppen ankondensiert werden, beispielsweise die in den Tabellenbeispielen wiedergegebenen Strukturen. In einem dritten Schritt wird die freie NH-Funktion über eine Buchwald-Kupplung aryliert.

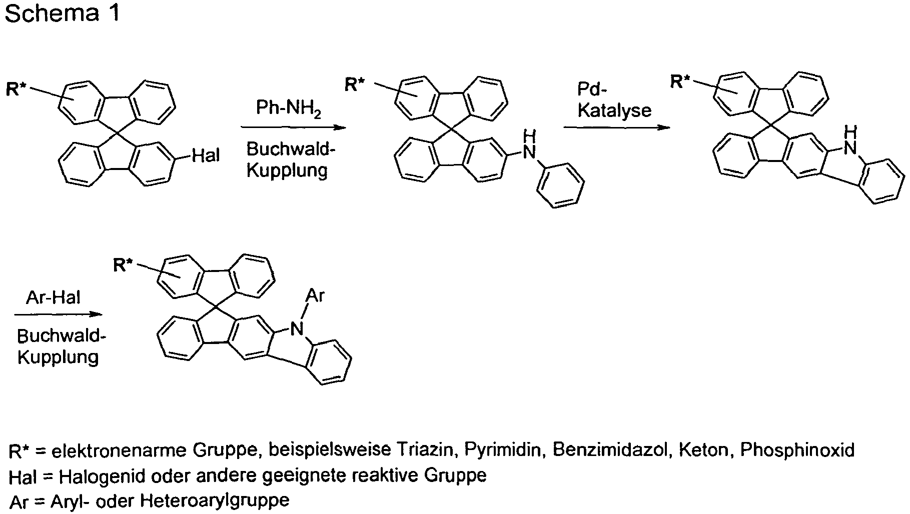

Gemäß dem in Schema 2 gezeigten Syntheseweg können erfindungsgemäße Verbindungen hergestellt werden, welche eine modifizierte Spirobifluorengruppe tragen, beispielsweise eine durch eine Keto-Brücke modifizierte Spirobifluorengruppe. Hierzu wird von einem Fluorenderivat ausgegangen, welches eine reaktive Gruppe trägt. Über die oben beschriebene Sequenz von Buchwald-Kupplung und Zyklisierung kann erneut eine Indolgruppe ankondensiert werden. Diese wird, wie ebenfalls bereits oben beschrieben, mittels einer Buchwald-Kupplung aryliert. In einem letzten Schritt wird über eine Zyklisierungsreaktion aus der Fluorenongruppe die modifizierte Spirobifluorengruppe hergestellt. Es ist alternativ möglich, eine andere Ausgangsverbindung als die in Schema 2 beschriebene in der Zyklisierungsreaktion einzusetzen. Einige solcher Ausgangsverbindungen sind kommerziell erhältlich. Hierzu sind entsprechende Synthesebeispiele für erfindungsgemäße Verbindungen in den Ausführungsbeispielen beschrieben.

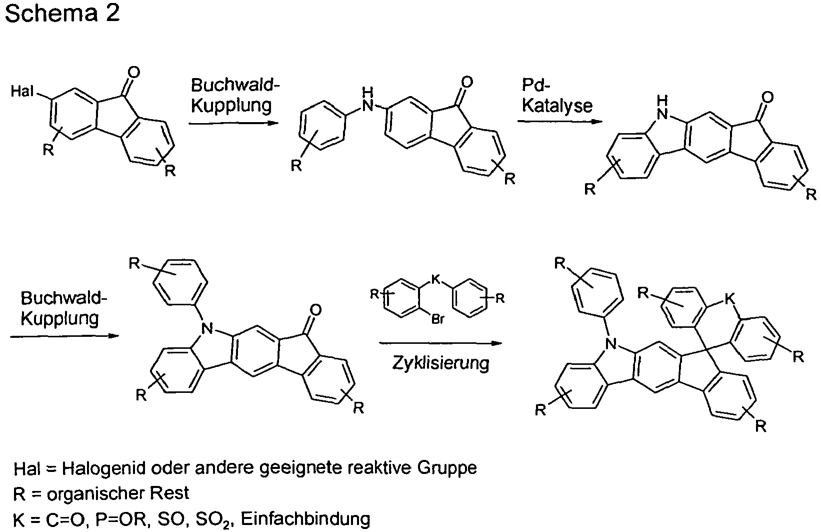

Gegenstand der Erfindung ist somit ein Verfahren zur Herstellung von erfindungsgemäßen Verbindungen, dadurch gekennzeichnet, dass
- an eine Spirobifluorengruppe, welche mit einer elektronenarmen Gruppe substituiert ist, eine Heteroarylgruppe ankondensiert wird, oder dass
- eine Zyklisierungsreaktion durchgeführt wird, über die eine modifizierte Spirobifluorengruppe erhalten wird.

Bevorzugt ist die oben genannte elektronenarme Gruppe eine Triazingruppe, eine Pyrimidingruppe, eine Benzimidazolgruppe, eine Ketongruppe oder eine Phosphinoxidgruppe.

Weiterhin bevorzugt enthält die modifizierte Spirobifluorengruppe eine Ketogruppe, eine Phosphinoxidgruppe, eine Sulfoxidgruppe oder eine Sulfongruppe.

Die oben beschriebenen erfindungsgemäßen Verbindungen, insbesondere Verbindungen, welche mit reaktiven Abgangsgruppen, wie Brom, Iod, Chlor, Boronsäure oder Boronsäureester, substituiert sind, können als Monomere zur Erzeugung entsprechender Oligomere, Dendrimere oder Polymere Verwendung finden. Geeignete reaktive Abgangsgruppen sind beispielsweise Brom, Iod, Chlor, Boronsäuren, Boronsäureester, Amine, Alkenyl- oder Alkinylgruppen mit endständiger C-C-Doppelbindung bzw. C-C-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterer Gegenstand der Erfindung sind daher Oligomere, Polymere oder Dendrimere enthaltend eine oder mehrere Verbindungen gemäß Formel (I) oder (II), wobei die Bindung(en) zum Polymer, Oligomer oder Dendrimer an beliebigen, mit R¹ oder R² substituierten Positionen lokalisiert sein können. Je nach Verknüpfung der Verbindung gemäß Formel (I) oder (II) ist die Verbindung Bestandteil einer Seitenkette des Oligomers oder Polymers oder Bestandteil der Hauptkette. Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist. Die erfindungsgemäßen Polymere, Oligomere oder Dendrimere können konjugiert, teilkonjugiert oder nichtkonjugiert sein. Die erfindungsgemäßen Oligomere oder Polymere können linear, verzweigt oder dendritisch sein. In den linear verknüpften Strukturen können die Einheiten gemäß Formel (I) oder (II) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten gemäß Formel (I) oder (II) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Für die Wiederholeinheiten gemäß Formel (I) oder (II) in Oligomeren, Dendrimeren und Polymeren gelten dieselben Bevorzugungen wie oben für Verbindungen gemäß Formel (I) oder (II) beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 2000/22026), Spirobifluorenen (z. B. gemäß EP 707020, EP 894107 oder WO 2006/061181), Paraphenylenen (z. B. gemäß WO 1992/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689 oder WO 2007/006383), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 2007/068325) oder phosphoreszierende Metallkomplexe (z. B. gemäß WO 2006/003000), und/oder Ladungstransporteinheiten, insbesondere solche basierend auf Triarylaminen.

Die erfindungsgemäßen Polymere, Oligomere und Dendrimere weisen vorteilhafte Eigenschaften, insbesondere hohe Lebensdauern, hohe Effizienzen und gute Farbkoordinaten auf.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (I) oder (II) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind folgende:
- (A): SUZUKI-Polymerisation;
- (B): YAMAMOTO-Polymerisation;
- (C): STILLE-Polymerisation; und
- (D): HARTWIG-BUCHWALD-Polymerisation.

Wie die Polymerisation nach diesen Methoden durchgeführt werden kann und wie die Polymere dann vom Reaktionsmedium abgetrennt und aufgereinigt werden können, ist dem Fachmann bekannt und in der Literatur, beispielsweise in WO 2003/048225, WO 2004/037887 und WO 2004/037887, im Detail beschrieben.

Gegenstand der vorliegenden Erfindung ist somit auch ein Verfahren zur Herstellung der erfindungsgemäßen Polymere, Oligomere und Dendrimere, das dadurch gekennzeichnet ist, dass sie durch Polymerisation gemäß SUZUKI, Polymerisation gemäß YAMAMOTO, Polymerisation gemäß STILLE oder Polymerisation gemäß HARTWIG-BUCHWALD hergestellt werden. Die erfindungsgemäßen Dendrimere können gemäß dem Fachmann bekannten Verfahren oder in Analogie dazu hergestellt werden. Geeignete Verfahren sind in der Literatur beschrieben, wie z. B. in Frechet, Jean M. J.; Hawker, Craig J., "Hyperbranched polyphenylene and hyperbranched polyesters: new soluble, three-dimensional, reactive polymers", Reactive & Functional Polymers (1995), 26(1-3), 127-36; Janssen, H. M.; Meijer, E. W., "The synthesis and characterization of dendritic molecules", Materials Science and Technology (1999), 20 (Synthesis of Polymers), 403-458; Tomalia, Donald A., "Dendrimer molecules", Scientific American (1995), 272(5), 62-6; WO 2002/067343 A1 und WO 2005/026144 A1.

Für die Verarbeitung der Verbindungen gemäß Formel (I) oder (II) aus flüssiger Phase, beispielsweise durch Spin-Coating oder durch Druckverfahren, sind Formulierungen der Verbindungen erforderlich. Diese Formulierungen können beispielsweise Lösungen, Dispersionen oder Miniemulsionen sein.

Gegenstand der Erfindung ist daher weiterhin eine Formulierung, insbesondere eine Lösung, Dispersion oder Miniemulsion, enthaltend mindestens eine Verbindung gemäß Formel (I) oder (II) oder mindestens ein Polymer, Oligomer oder Dendrimer enthaltend mindestens eine Einheit gemäß Formel (I) oder (II) sowie mindestens ein Lösungsmittel, bevorzugt ein organisches Lösungsmittel. Wie solche Lösungen hergestellt werden können, ist dem Fachmann bekannt und beispielsweise in den Anmeldungen WO 2002/072714 und WO 2003/019694 und der darin zitierten Literatur beschrieben.

Die Verbindungen gemäß Formel (I) oder (II) eignen sich für den Einsatz in elektronischen Vorrichtungen, insbesondere in organischen Elektrolumineszenzvorrichtungen (OLEDs). Die Verbindungen können, unter anderem abhängig von der Substitution, in unterschiedlichen Funktionen und/oder Schichten eingesetzt werden. Bevorzugt werden die Verbindungen als Hostmaterialien für phosphoreszierende Emitter und/oder als Elektronentransportmaterialien in einer Elektronentransportschicht und/oder als Lochblockiermaterialien in einer Lochblockierschicht eingesetzt.

Ein weiterer Gegenstand der Erfindung ist die Verwendung der Verbindungen gemäß Formel (I) oder (II) in elektronischen Vorrichtungen. Dabei sind die elektronischen Vorrichtungen bevorzugt ausgewählt aus der Gruppe bestehend aus organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und besonders bevorzugt ausgewählt aus organischen Elektrolumineszenzvorrichtungen (OLEDs).

Gegenstand der Erfindung ist weiterhin eine elektronische Vorrichtung, enthaltend Anode, Kathode sowie mindestens eine organische Schicht, wobei die organische Schicht mindestens eine Verbindung der Formel (I) oder (II) enthält. Dabei ist die elektronische Vorrichtung bevorzugt ausgewählt aus den oben genannten Vorrichtungen und besonders bevorzugt eine organische Elektrolumineszenzvorrichtung (OLED).

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer), Auskopplungsschichten und/oder organischen oder anorganischen p/n-Übergängen. Es sei aber darauf hingewiesen, dass nicht notwendigerweise jede dieser Schichten vorhanden sein muss und die Wahl der Schichten immer von den verwendeten Verbindungen abhängt und insbesondere auch von der Tatsache, ob es sich um eine fluoreszierende oder phosphoreszierende Elektrolumineszenzvorrichtung handelt. Die in den jeweiligen Schichten und Funktionen bevorzugt eingesetzten Verbindungen werden in späteren Abschnitten explizit offenbart.

Es ist erfindungsgemäß bevorzugt, wenn die Verbindung gemäß Formel (I) oder (II) in einer elektronischen Vorrichtung enthaltend einen oder mehrere phosphoreszierende Dotanden eingesetzt wird. Dabei kann die Verbindung in unterschiedlichen Schichten, bevorzugt in einer Elektronentransportschicht, einer Lochtransportschicht, einer Lochinjektionsschicht oder in der emittierenden Schicht verwendet werden. Die Verbindung gemäß Formel (I) oder (II) kann aber auch erfindungsgemäß in einer elektronischen Vorrichtung, enthaltend einen oder mehrere fluoreszierende Dotanden und keine phosphoreszierenden Dotanden, eingesetzt werden.

Vom Begriff phosphoreszierende Dotanden sind typischerweise Verbindungen umfasst, bei denen die Lichtemission durch einen spinverbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Dotanden eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende Dotanden Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Erfindung alle lumineszierenden Iridium-, Platin- oder Kupferkomplexe als phosphoreszierende Verbindungen angesehen.

Beispiele der oben beschriebenen phosphoreszierenden Dotanden können den Anmeldungen WO 2000/70655, WO 2001/41512, WO 2002/02714, WO 2002/15645, EP 1191613, EP 1191612, EP 1191614, WO 2005/033244, WO 2005/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind, zur Verwendung in den erfindungsgemäßen Vorrichtungen. Auch kann der Fachmann ohne erfinderisches Zutun weitere phosphoreszierende Komplexe in Kombination mit den erfindungsgemäßen Verbindungen in OLEDs einsetzen. Weitere Beispiele für geeignete phosphoreszierende Dotanden können der in einem späteren Abschnitt folgenden Tabelle entnommen werden.

In einer bevorzugten Ausführungsform der vorliegenden Erfindung werden die Verbindungen der Formel (I) oder (II) als Matrixmaterial in Kombination mit einem oder mehreren Dotanden, vorzugsweise phosphoreszierenden Dotanden, eingesetzt.

Unter einem Dotanden wird in einem System enthaltend ein Matrixmaterial und einen Dotanden diejenige Komponente verstanden, deren Anteil in der Mischung der kleinere ist. Entsprechend wird unter einem Matrixmaterial in einem System enthaltend ein Matrixmaterial und einen Dotanden diejenige Komponente verstanden, deren Anteil in der Mischung der größere ist.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%. Entsprechend beträgt der Anteil des Dotanden zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere Dotanden enthalten. Auch in diesem Fall sind die Dotanden im Allgemeinen diejenigen Materialien, deren Anteil im System der kleinere ist und die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Dotanden.

In einer weiteren bevorzugten Ausführungsform der Erfindung werden die Verbindungen gemäß Formel (I) oder (II) als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Die Mixed-Matrix-Systeme können einen oder mehrere Dotanden umfassen. Die Dotandverbindung bzw. die Dotandverbindungen zusammen haben erfindungsgemäß einen Anteil von 0.1 bis 50.0 Vol.-% an der Gesamtmischung und bevorzugt einen Anteil von 0.5 bis 20.0 Vol.-% an der Gesamtmischung. Entsprechend haben die Matrixkomponenten zusammen einen Anteil von 50.0 bis 99.9 Vol-% an der Gesamtmischung und bevorzugt einen Anteil von 80.0 bis 99.5 Vol.-% an der Gesamtmischung.

Besonders geeignete Matrixmaterialien, welche in Kombination mit den erfindungsgemäßen Verbindungen als Matrixkomponenten eines Mixed-Matrix-Systems eingesetzt werden können, sind allgemein die in folgenden Abschnitten aufgeführten bevorzugten Matrixmaterialien. Dabei sind, je nachdem ob ein fluoreszierender oder ein phosphoreszierender Dotand in der emittierenden Schicht vorliegt, jeweils die unten angegebenen bevorzugten Matrixmaterialien für fluoreszierende Dotanden oder die unten angegebenen bevorzugten Matrixmaterialien für phosphoreszierende Dotanden bevorzugt.

Bevorzugte phosphoreszierende Dotanden zur Verwendung in Mixed-Matrix-Systemen enthaltend die erfindungsgemäßen Verbindungen sind die in einer folgenden Tabelle aufgeführten phosphoreszierenden Dotanden.

In einer weiteren bevorzugten Ausführungsform der Erfindung wird die Verbindung gemäß Formel (I) oder (II) als Elektronentransportmaterial in einer Elektronentransport- oder Elektroneninjektionsschicht eingesetzt. Dabei kann die emittierende Schicht fluoreszierende und/oder phosphoreszierende Emitter enthalten. Wenn die Verbindung als Elektronentransportmaterial eingesetzt wird, kann es bevorzugt sein, wenn sie dotiert ist, beispielsweise mit Alkalimetallkomplexen, wie z. B. Liq (Lithiumhydroxychinolinat). Auch eignet sich die Kombination der erfindungsgemäßen Verbindung in einer Elektronentransportschicht mit einer Elektroneninjektionsschicht. Als Materialien für die Elektroneninjektionsschicht eignen sich beispielsweise Alkali- oder Erdalkalifluoride, wie z. B. LiF.

In nochmals einer weiteren bevorzugten Ausführungsform der Erfindung wird die Verbindung gemäß Formel (I) oder (II) als Lochblockiermaterial in einer Lochblockierschicht eingesetzt. Unter einer Lochblockierschicht wird eine Schicht verstanden, die auf Kathodenseite direkt an eine emittierende Schicht angrenzt.

Die erfindungsgemäße organische Elektrolumineszenzvorrichtung kann auch mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues oder gelbes oder orangefarbenes oder rotes Licht emittieren, wobei die verschiedenen Farben in dieser Ausführungsform der Erfindung zusammen weißes Licht ergeben. Insbesondere bevorzugt sind Dreischichtsysteme, also Systeme mit drei emittierenden Schichten, wobei eine oder mehrere dieser Schichten eine Verbindung gemäß Formel (I) oder (II) enthalten kann und wobei die drei Schichten blaue, grüne und orangefarbene oder rote Emission zeigen (für den prinzipiellen Aufbau siehe z. B.
WO 2005/011013). Ebenso eignen sich in solchen Systemen für weiße Emission Emitter, welche breitbandige Emissionsbanden aufweisen und dadurch weiße Emission zeigen. Alternativ und/oder zusätzlich können die erfindungsgemäßen Verbindungen in solchen Systemen auch in einer Lochtransportschicht oder Elektronentransportschicht oder in einer anderen Schicht vorhanden sein.

Im Folgenden werden die in den erfindungsgemäßen elektronischen Vorrichtungen bevorzugt eingesetzten weiteren Funktionsmaterialien aufgeführt.

Die in der folgenden Tabelle aufgeführten Verbindungen stellen besonders geeignete phosphoreszierende Dotanden dar.

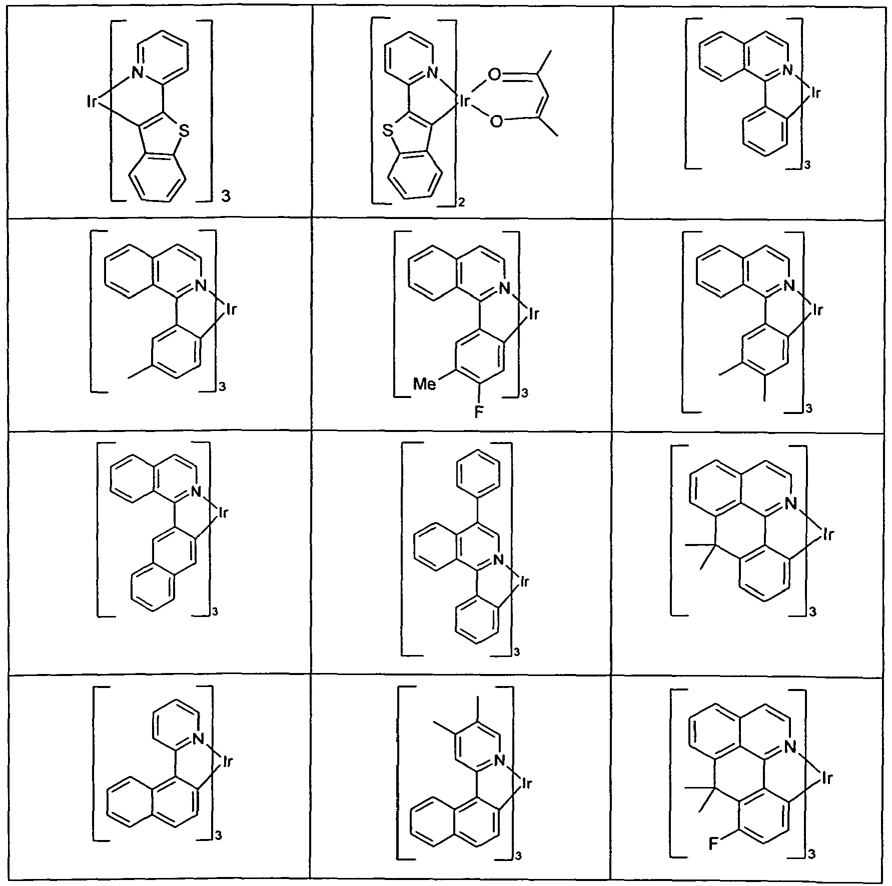

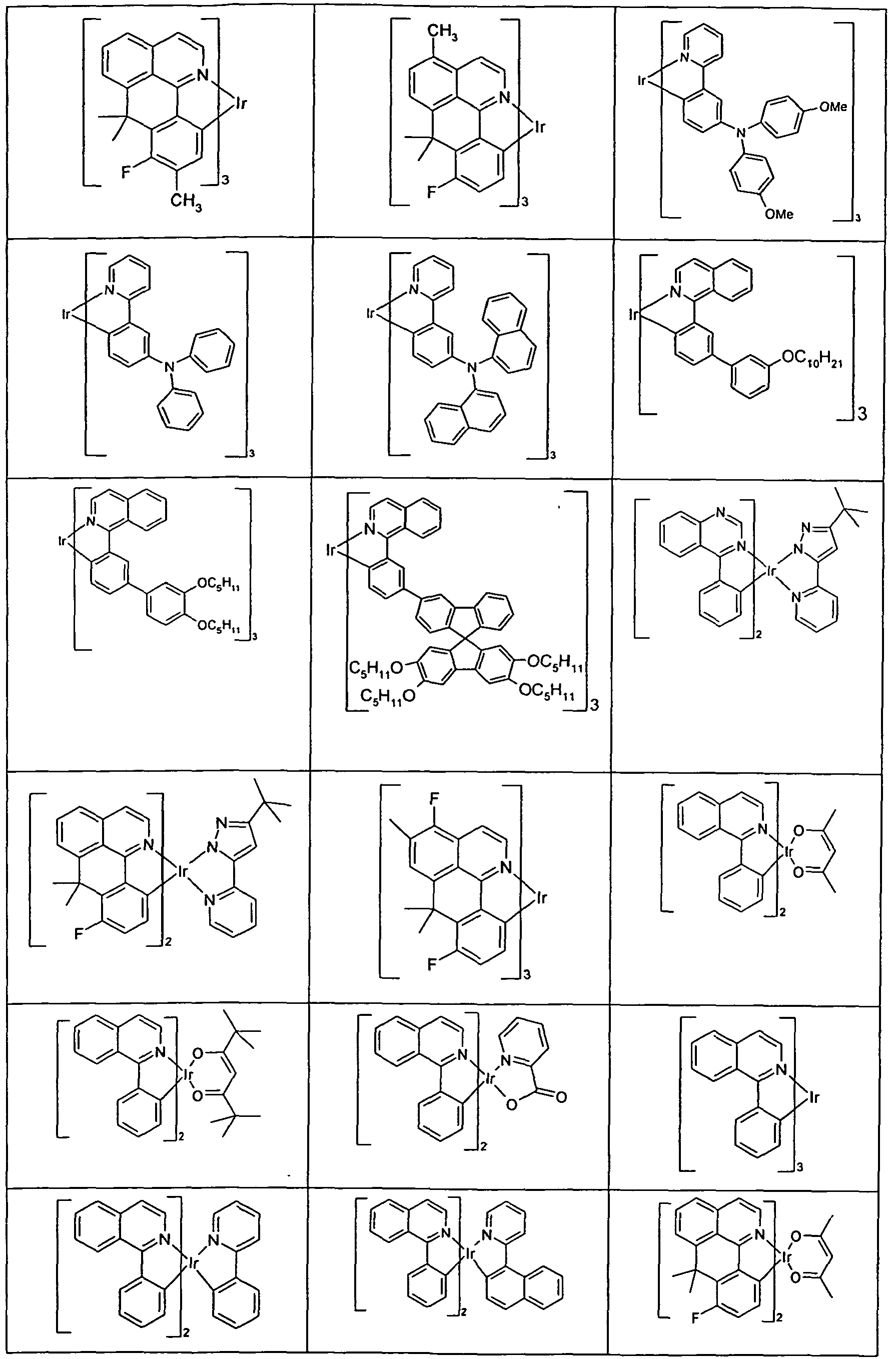

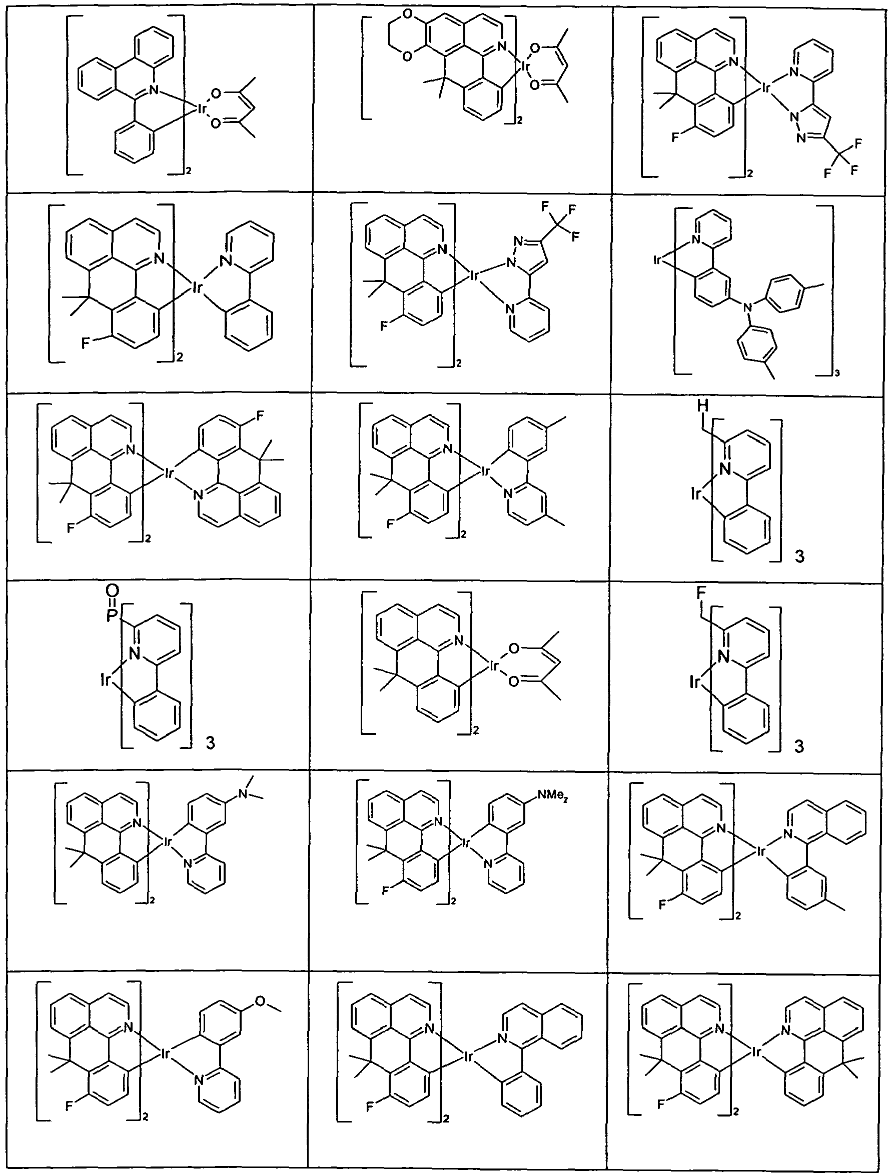

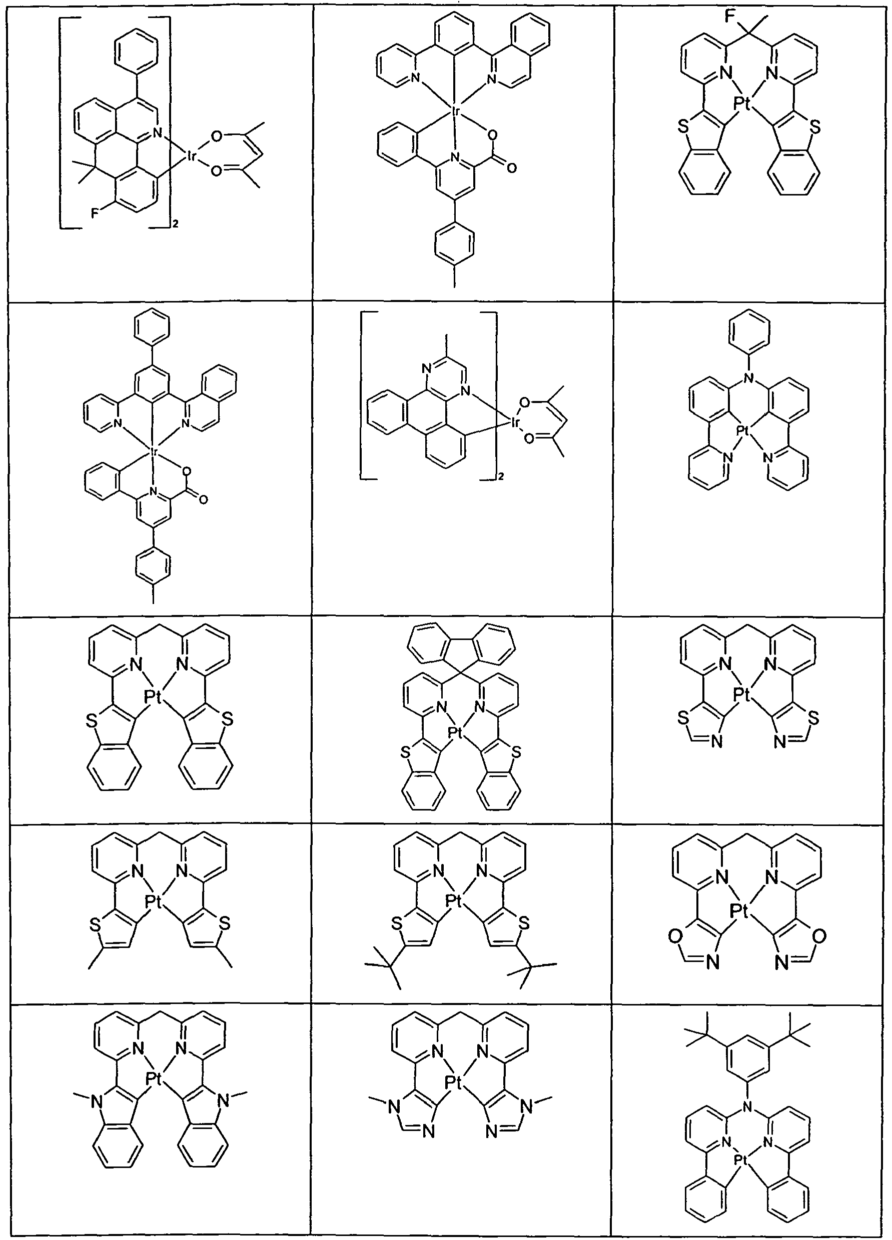

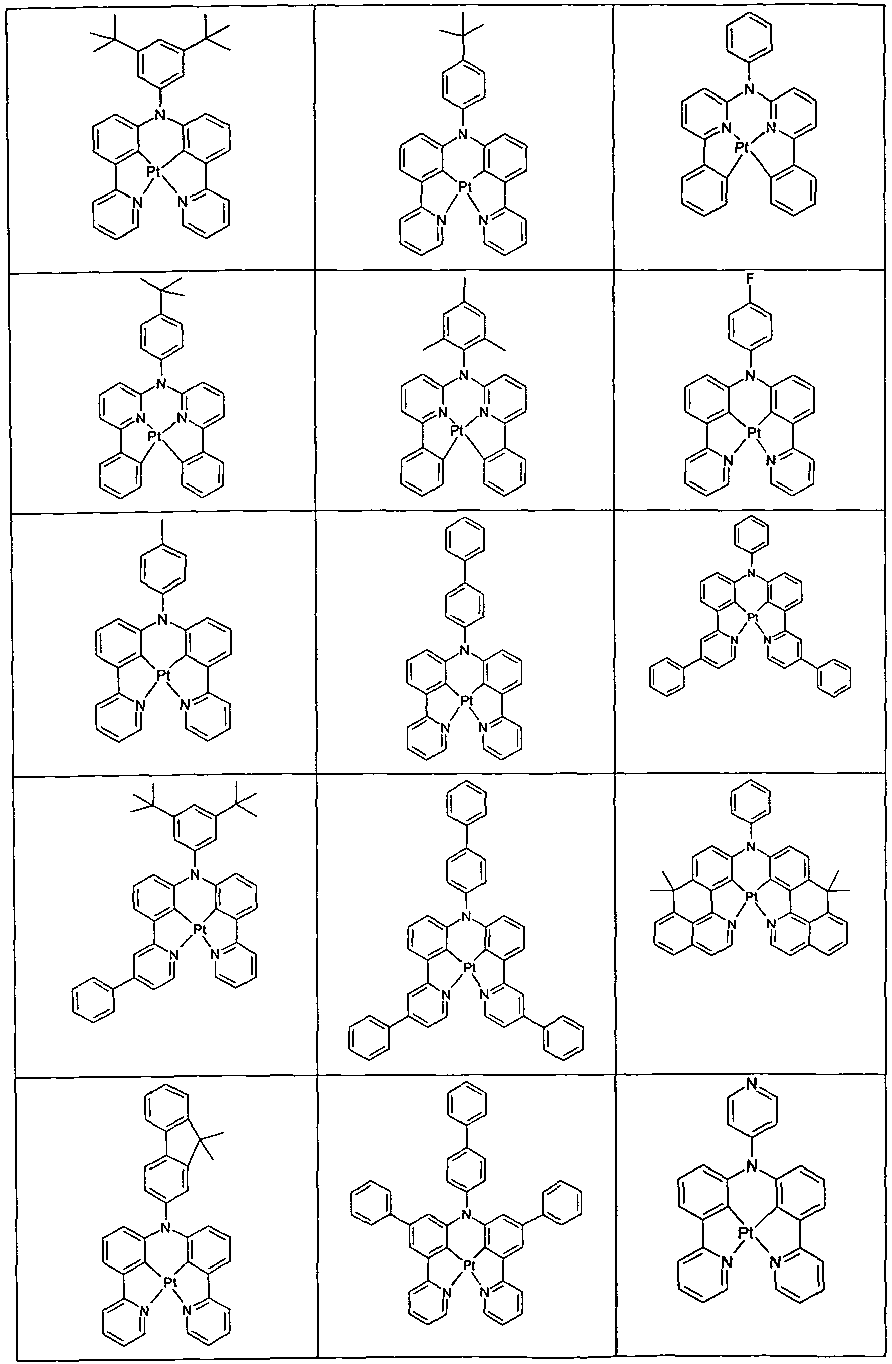

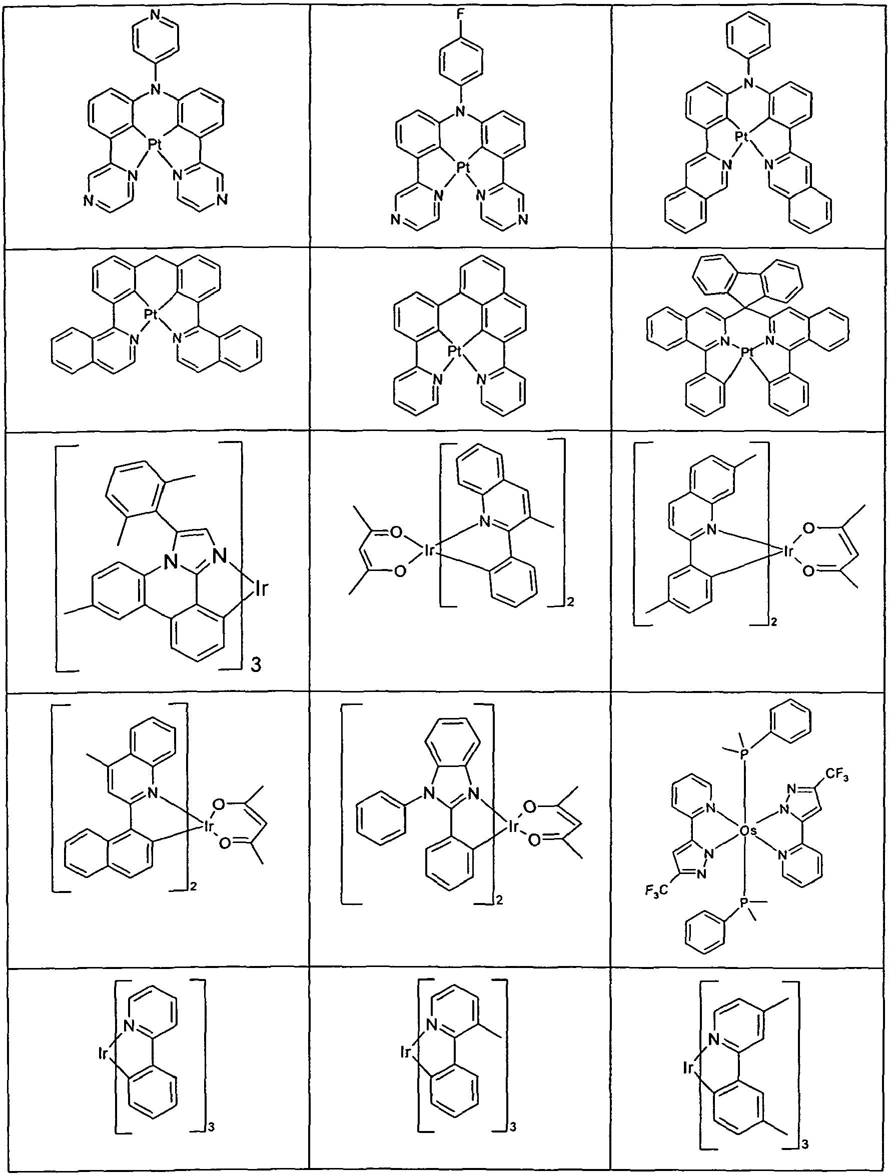

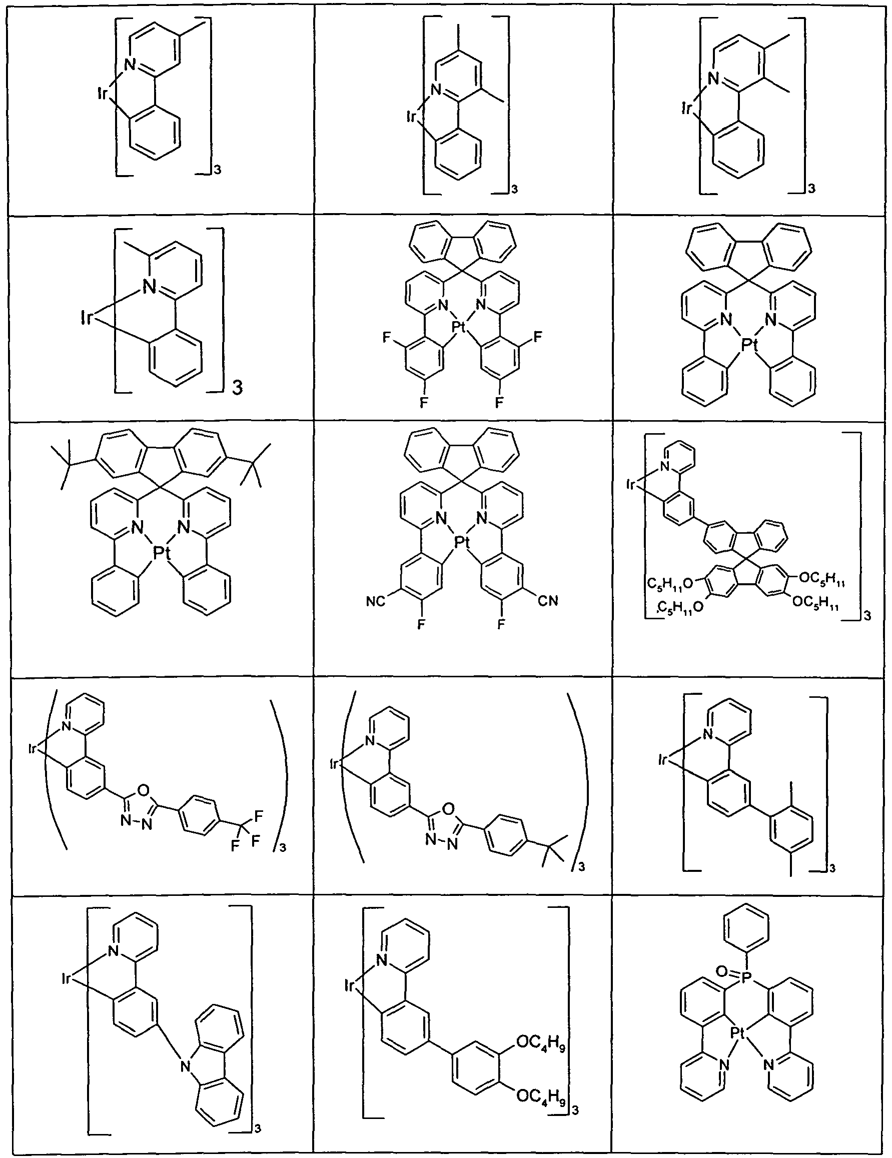

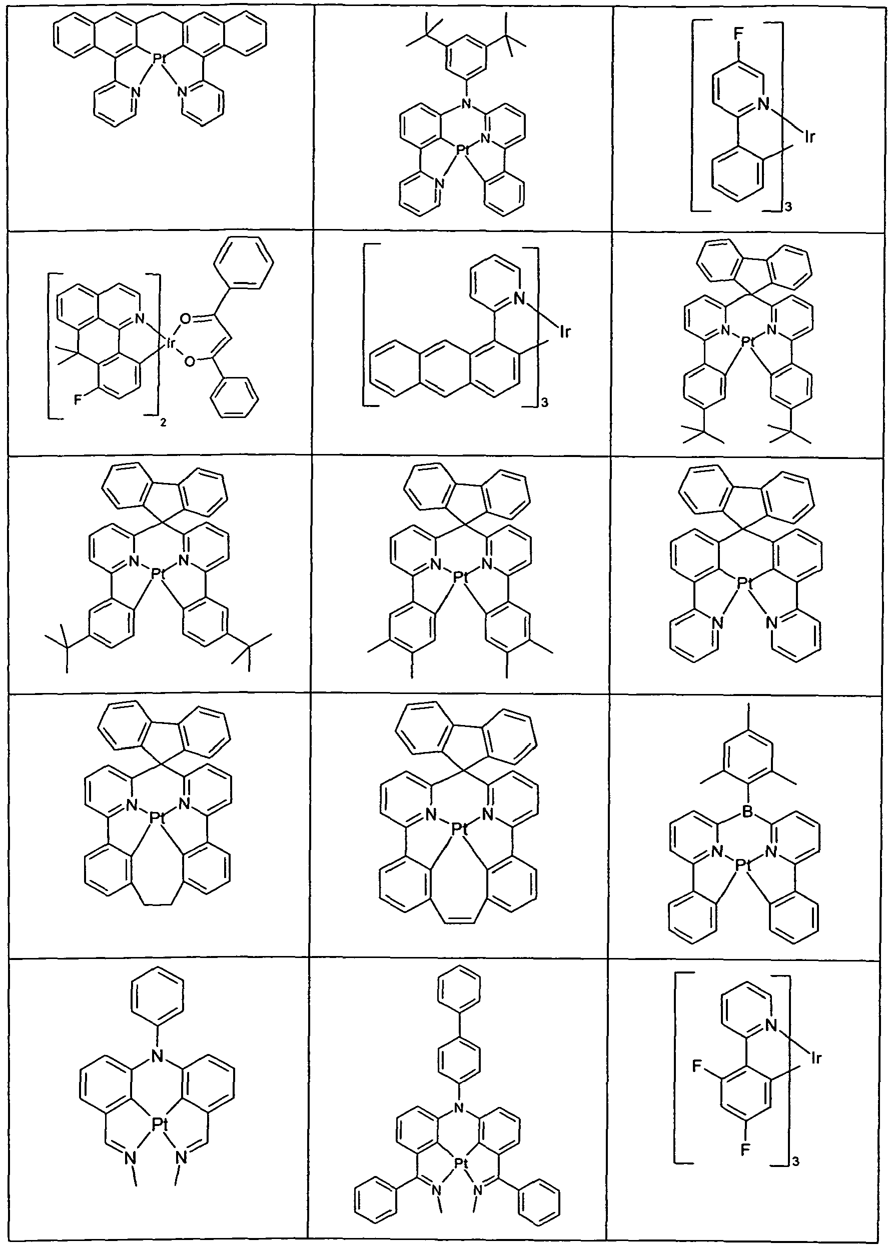

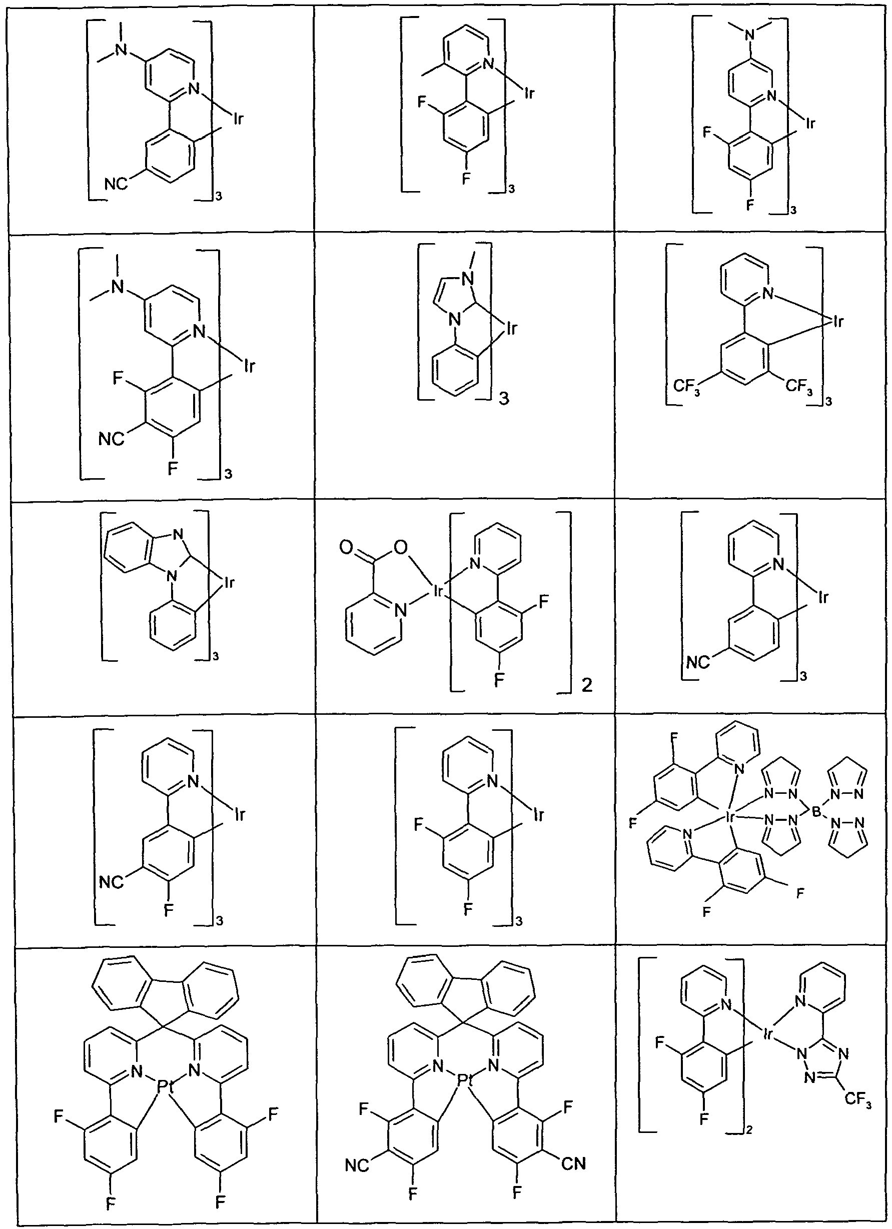

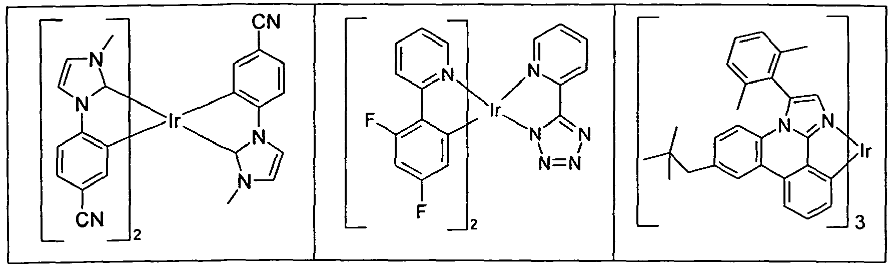

Bevorzugte fluoreszierende Dotanden sind ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind.

Weitere bevorzugte fluoreszierende Dotanden sind gewählt aus Indenofluorenaminen bzw. -diaminen, beispielsweise gemäß WO 2006/122630, Benzoindenofluorenaminen bzw. -diaminen, beispielsweise gemäß WO 2008/006449, und Dibenzoindenofluorenaminen bzw. -diaminen, beispielsweise gemäß WO 2007/140847. Beispiele für fluoreszierende Dotanden aus der Klasse der Styrylamine sind substituierte oder unsubstituierte Tristilbenamine oder die fluoreszierenden Dotanden, die in WO 2006/000388, WO 2006/058737, WO 2006/000389,

WO 2007/065549 und WO 2007/115610 beschrieben sind. Weiterhin bevorzugt sind die in WO 2010/012328 offenbarten kondensierten Kohlenwasserstoffe. Geeignete fluoreszierende Dotanden sind weiterhin die in JP 2006/001973, WO 2004/047499, WO 2006/098080, WO 2007/065678, US 2005/0260442 und WO 2004/092111 offenbarten Derivate.

Als Matrixmaterialien, bevorzugt für fluoreszierende Dotanden, kommen Materialien verschiedener Stoffklassen in Frage. Bevorzugte Matrixmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß
WO 2005/084081 und WO 2005/084082), der Atropisomere (z. B. gemäß
WO 2006/048268), der Boronsäurederivate (z. B. gemäß
WO 2006/117052) oder der Benzanthracene (z. B. gemäß
WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind.

Bevorzugte Matrixmaterialien für phosphoreszierende Dotanden sind neben den erfindungsgemäßen Verbindungen aromatische Amine, insbesondere Triarylamine, z. B. gemäß US 2005/0069729, Carbazolderivate (z. B. CBP, N,N-Biscarbazolylbiphenyl) oder Verbindungen gemäß WO 2005/039246, US 2005/0069729, JP 2004/288381, EP 1205527 oder WO 2008/086851, verbrückte Carbazolderivate, z. B. gemäß den Anmeldungen WO 2011/088877 und WO 2011/128017, Indenocarbazolderivate, z. B. gemäß WO 2010/136109 und WO 2011/000455, Azacarbazolderivate, z. B. gemäß EP 1617710, EP 1617711, EP 1731584, JP 2005/347160, Indolocarbazolderivate, z. B. gemäß WO 2007/063754 oder WO 2008/056746, Ketone, z. B. gemäß WO 2004/093207 oder WO 2010/006680, Phosphinoxide, Sulfoxide und Sulfone, z. B. gemäß WO 2005/003253, Oligophenylene, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Aluminiumkomplexe, z. B. BAIq, Diazasilol- und Tetraazasilol-Derivate, z. B. gemäß WO 2010/054729, Diazaphosphol-Derivate, z. B. gemäß WO 2010/054730 und Aluminiumkomplexe, z. B. BAIQ.

Geeignete Ladungstransportmaterialien, wie sie in der Lochinjektions- bzw. Lochtransportschicht oder in der Elektronentransportschicht der erfindungsgemäßen organischen Elektrolumineszenzvorrichtung verwendet werden können, sind neben den erfindungsgemäßen Verbindungen beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Als Kathode sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Ba/Ag oder Mg/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink-Oxid (IZO). Bevorzugt sind weiterhin leitfähige, dotierte organische Materialien, insbesondere leitfähige dotierte Polymere.

Die Vorrichtung wird entsprechend (je nach Anwendung) strukturiert, kontaktiert und schließlich versiegelt, da sich die Lebensdauer der erfindungsgemäßen Vorrichtungen bei Anwesenheit von Wasser und/oder Luft verkürzt.

In einer bevorzugten Ausführungsform ist die erfindungsgemäße organische Elektrolumineszenzvorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar. Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (I) oder (II) nötig. Hohe Löslichkeit lässt sich durch geeignete Substitution der Verbindungen erreichen.

Weiterhin bevorzugt ist es, dass zur Herstellung einer erfindungsgemäßen organischen Elektrolumineszenzvorrichtung eine oder mehrere Schichten aus Lösung und eine oder mehrere Schichten durch ein Sublimationsverfahren aufgetragen werden.

Die organischen Elektrolumineszenzvorrichtungen enthaltend eine oder mehrere erfindungsgemäße Verbindungen können in Displays, als Lichtquellen in Beleuchtungsanwendungen sowie als Lichtquellen in medizinischen und/oder kosmetischen Anwendungen (z.B. Lichttherapie) eingesetzt werden.

Die erfindungsgemäßen Verbindungen und die erfindungsgemäßen organischen Elektrolumineszenzvorrichtungen zeichnen sich durch folgende überraschende Vorteile gegenüber dem Stand der Technik aus:
1. Die erfindungsgemäßen Verbindungen eignen sich sehr gut für den Einsatz in einer Elektronentransportschicht einer organischen Elektrolumineszenzvorrichtung.
2. Die erfindungsgemäßen Verbindungen, eingesetzt als Matrixmaterial für fluoreszierende oder phosphoreszierende Emitter, führen zu sehr hohen Effizienzen sowie zu langen Lebensdauern. Dies gilt insbesondere, wenn die Verbindungen als Matrixmaterial für einen phosphoreszierenden Emitter eingesetzt werden.
3. Die erfindungsgemäßen Verbindungen eignen sich nicht nur als Matrix für rot und grün phosphoreszierende Verbindungen, sondern auch für blau phosphoreszierende Verbindungen.
4. Die erfindungsgemäßen Verbindungen, eingesetzt in organischen Elektrolumineszenzvorrichtungen, führen zu hohen Effizienzen und zu steilen Strom-Spannungs-Kurven mit niedrigen Einsatz- und Betriebsspannungen.
5. Die erfindungsgemäßen Verbindungen weisen eine hohe Temperaturstabilität sowie eine hohe Oxidationsstabilität in Lösung auf und sind somit gut prozessierbar.

Die Erfindung wird durch die nachfolgenden Anwendungsbeispiele näher erläutert, wobei die Erfindung nicht auf den Umfang der Beispiele beschränkt ist.

### Ausführungsbeispiele

### 1. Synthese der Intermediate A bis D

Synthese des Intermediats A: 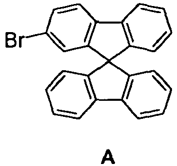 10.3 mmol 9,9'-Spirobifluoren werden in 30 ml CH₂Cl₂ gelöst und vor Lichteinfall geschützt. 10.3 mmol NBS werden innerhalb 30 min unter Rühren portionsweise zugegeben. Nach 24 h wird die Mischung mit Wasser versetzt. Die organische Phase wird nach dem Trocknen über Na₂SO₄ am Rotationsverdampfer eingeengt. Der Rückstand wird mit MeOH umkristallisiert.

Analog werden folgende Verbindungen erhalten:

| **Bsp**. | **Edukt** 1 | **Produkt** | **Ausbeute** |
|---|---|---|---|
| B | 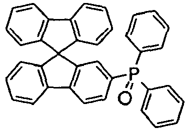 | 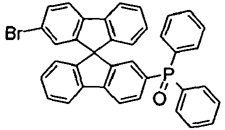 | 44 % |
| | **1125547-88-7** | | |
| C | 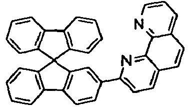 | 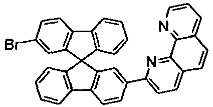 | 38 % |
| | **1174660-93-5** | | |
| D | 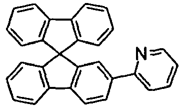 | 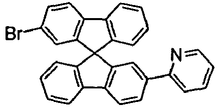 | 42 % |
| | **1092539-80-4** | | |

### 2. Synthese der erfindungsgemäßen Verbindungen 1 bis 8

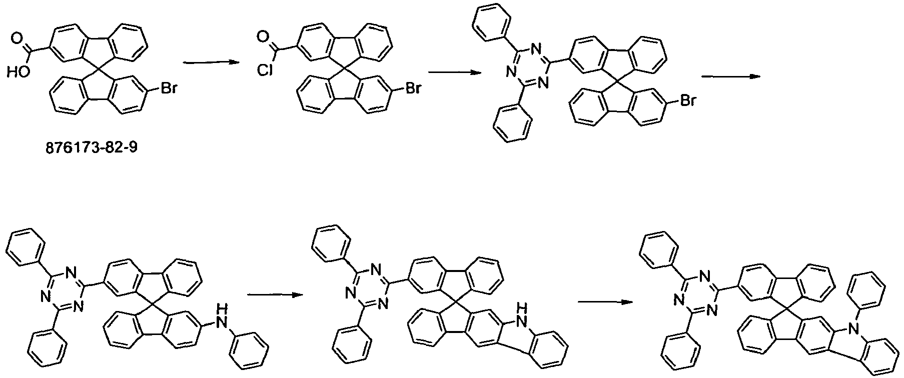

### 2'-Brom-9H,9'H-[9,9']bifluoreny/-2-canbonsäurechlorid

20 ml (274mmol) Thionylchlorid wird mit 0.16 ml DMF bei Raumtemperatur vorgelegt. Dann werden 9 g (20 mmol) 2'-Bromo-9H,9'H-[9,9']bifluorenyl-2-carbonsäure hinzugefügt. Die Reaktionsmischung wird 1 h bei 60 °C gerührt. Danach wird das restliche Thionylchlorid abdestilliert und aus Toluol umkristallisiert.
Ausbeute: 9.1 g (19 mmol), 98% d. Th., Reinheit nach ¹H-NMR ca. 97%.

*2*-*(2*'-*Bromo*-*9H*,*9'H-[9,9']bifluorenyl-*2*-yl)-4,6-diphenyl-[1,3,5]triazin* 42 g (89 mmol) 2'-Bromo-9H,9'H-[9,9']bifluorenyl-carbonsäurechlorid, 11.90 g (89 mmol) Aluminiumtrichlorid und 1.9 ml (27 mmol) Thionylchlorid werden in 260 ml Dichlorbenzol suspendiert. Dann werden 19.3 ml (187 mmol) Benzonitril langsam hinzugefügt. Die Reaktionsmischung wird 1 h bei 100 °C gerührt. 9.55 g (179 mmol) Ammoniumchlorid werden hinzugefügt und der Ansatz wird 16 h lang bei 100 °C gerührt. Nach Abkühlen auf Raumtemperatur wird die Reaktionslösung auf 3.5 L Methanol gegeben und 45 min lang gerührt. Der ausgefallene Feststoff wird abfiltriert und aus Toluol umkristallisiert.
Ausbeute: 51 g (80 mmol), 90% d. Th., Reinheit nach ¹H-NMR ca. 97%.

### [2'-(4,6-Diphenyl-[1,3,5]triazin-2-yl)-9H,9'H-[9,9']bifluorenyl-2-y/]-phenyl-amin

117 g (183 mmol) 2-(2'-Bromo-9H,9'H-[9,9']bifluorenyl-2-yl)-4,6-diphenyl-[1,3,5]triazin, 20 ml Anilin (220 mmol), 1.5 g DPPF (2.7 mmol), 0.5 g Palladium(II)acetat und 45 g Natrium-tert-butylat (486 mmol) werden in 1.5 L Toluol 18 h unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Der verbleibende Rückstand wird aus Heptan/Essigester umkristallisiert.
Ausbeute: 106 g (162 mmol), 89% d. Th., Reinheit nach ¹H-NMR ca. 97%.

### 12-[2-(4,6-Diphenyl-[1,3,5]triazin-2-yl)-9H-fluoren-9-yl]-10,12-dihydro-10-aza-indeno[2,1-b]fluoren

22.8 g (35 mmol) [2'-(4,6-Diphenyl-[1,3,5]triazin-2-yl)-9H,9'H-[9,9']bifluorenyl-2-yl]-phenyl-amin, 0.4 g Palladium(II)acetat (1.78 mmol) und 0.5 g Kaliumcarbonat (3.62 mmol) werden mit 35 ml Pivalinsäure versetzt und das Gemisch bei 120 °C für 9 h gerührt. Nach dieser Zeit erfolgt die Zugabe von 0.4 g Palladium(II)acetat (1.78 mmol) und das Gemisch wird weiter bei 120 °C für 9 h gerührt. Dann werden 200 ml Dichlormethan und 0.1 M Na₂CO₃-Lösung zugegeben. Das Gemisch wird zwischen Wasser und Dichlormethan verteilt, die wässrige Phase dreimal mit Dichlormethan extrahiert, die vereinigten organische Phasen über Na₂SO₄ getrocknet und einrotiert. Der Rückstand wird aus Toluol/Heptan umkristallisiert.
Ausbeute: 18.2 g (28 mmol), 80% d. Th., Reinheit nach ¹H-NMR ca. 97%.

### 12-[2-(4,6-Diphenyl-[1,3,5]triazin-2-yl)-9H-fluoren-9-yl]-10-phenyl-10,12-dihydro-10-aza-indeno[2,1-b]fluoren (Beispiel 1)

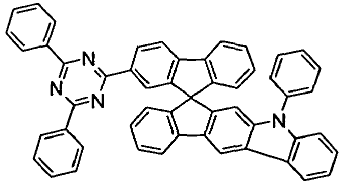

69 g (106 mmol) 12-[2-(4,6-Diphenyl-[1,3,5]triazin-2-yl)-9H-fluoren-9-yl]-10,12-dihydro-10-aza-indeno[2,1-b]fluoren, 17.8 g (114 mmol) Brombenzol und 30.5 g NaOtBu werden in 1.5 L p-Xylol suspendiert. Zu dieser Suspension werden 0.5 g (2.11 mmol) Pd(OAc)₂ und 1.6 mL einer 1 M Tri-tert-butylphosphin-Lösung gegeben. Die Reaktionsmischung wird 16 h unter Rückfluss erhitzt. Nach Erkalten wird die organische Phase abgetrennt, dreimal mit 200 ml Wasser gewaschen und anschließend zur Trockne eingeengt. Der Rückstand wird mit Toluol heiß extrahiert, aus Toluol umkristallisiert und abschließend im Hochvakuum sublimiert, Reinheit beträgt 99,9%.
Ausbeute: 74 g (102 mmol), 97% d. Th.

Analog werden folgende Verbindungen erhalten:

| **Bsp.** | **Edukt 1** | **Edukt 2** | **Produkt** | **Ausbeute** |
|---|---|---|---|---|
| 2 | 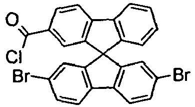 | 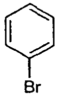 | 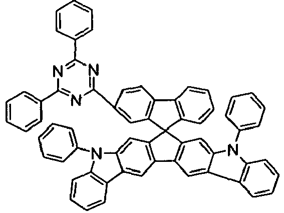 | 59 % |
| | **1233200-68-4** | | | |
| 3 | 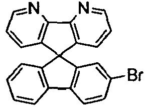 | 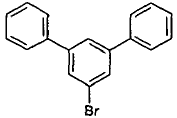 | 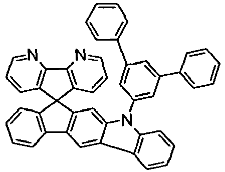 | 66 % |
| | **905853-26-1** | **103068-20-8** Es erfolgen nur die drei letzten Schritte im Schema | | |
| 4 | 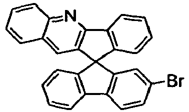 | 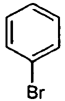 | 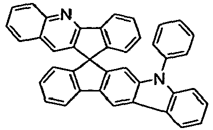 | 69 % |
| | **1262330-86-8** | Es erfolgen nur die drei letzten Schritte im Schema | | |
| 5 | 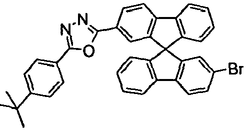 | 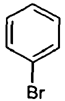 | 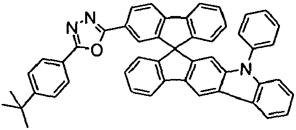 | 54 % |
| | **876173-84-1** | Es erfolgen nur die drei letzten Schritte im Schema | | |
| 6 | 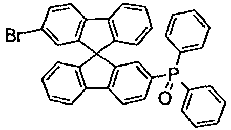 | 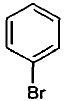 | 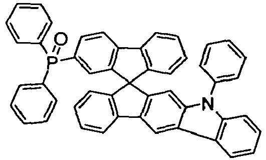 | 45 % |
| | **B** | Es erfolgen nur die drei letzten Schritte im Schema | | |
| 7 | 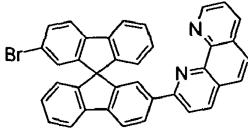 | 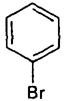 | 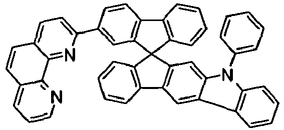 | 56 % |
| | **C** | Es erfolgen nur die drei letzten Schritte im Schema | | |
| 8 | 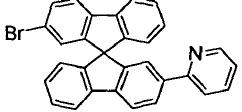 | 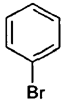 | 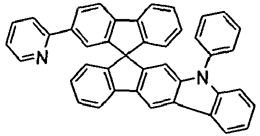 | 49 % |
| | **D** | Es erfolgen nur die drei letzten Schritte im Schema | | |

### 3. Synthese der erfindungsgemäßen Verbindungen 9-13

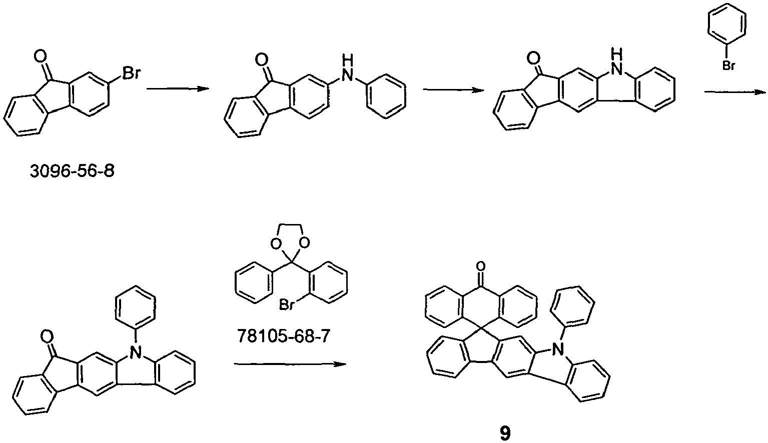

### 2-Phenylamino-fluoren-9-on

47g (183 mmol) 2-Bromfluorenon, 20 ml Anilin (220 mmol), 1.5 g DPPF (2.7 mmol), 0.5 g Palladium(II)acetat und 45 g Natrium-tert-butylat (486 mmol) werden in 1.5 L Toluol 18 h unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Der verbleibende Rückstand wird aus Heptan/Essigester umkristallisiert.
Ausbeute: 43 g (159 mmol), 87% d. Th., Reinheit nach ¹H-NMR ca. 97%.

### 10H-10-Aza-indeno[2,1-b]fluoren-12-on

9.5 g (35 mmol) 2-Phenylamino-fluoren-9-on, 0.4 g Palladium(II)acetat (1.78 mmol) und 0.5 g Kaliumcarbonat (3.62 mmol) werden in 35 ml Pivalinsäure versetzt und das Gemisch bei 120 °C für 9 h gerührt. Nach dieser Zeit erfolgt die Zugabe von 0.4 g Palladium(II)acetat (1.78 mmol) und das Gemisch wird weiter bei 120 °C für 9 h gerührt. Dann werden 200 ml Dichlormethan und 0.1 M Na₂CO₃-Lösung zugegeben. Das Gemisch wird zwischen Wasser und Dichlormethan verteilt, die wässrige Phase dreimal mit Dichlormethan extrahiert, die vereinigten organischen Phasen über Na₂SO₄ getrocknet und einrotiert. Der Rückstand wird aus Toluol/Heptan umkristallisiert.
Ausbeute: 7.4 g (27 mmol), 79 % d. Th., Reinheit nach ¹H-NMR ca. 97%.

### 10-Phenyl-10H-10-aza-indeno[2,1-b]fluoren-12-on

28.5 g (106 mmol) 10H-10-Aza-indeno[2,1-b]fluoren-12-on, 17.8 g (114 mmol) Brombenzol und 30.5 g NaOtBu werden in 1.5 L p-Xylol suspendiert. Zu dieser Suspension werden 0.5 g (2.11 mmol) Pd(OAc)₂ und 1.6 ml einer 1 M Tri-tert-butylphosphin-Lösung gegeben. Die Reaktionsmischung wird 16 h unter Rückfluss erhitzt. Nach Erkalten wird die organische Phase abgetrennt, dreimal mit 200 ml Wasser gewaschen und anschließend zur Trockene eingeengt. Der Rückstand wird mit Toluol heiß extrahiert, aus Toluol umkristallisiert und abschließend im Hochvakuum sublimiert, Reinheit beträgt 99.9%.
Ausbeute: 32 g (95 mmol), 90% d. Th., Reinheit nach ¹H-NMR ca. 97%.

### Synthese der Spiroverbindung (Beispiel 9)

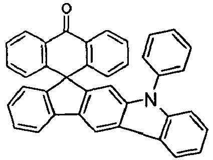

Zu einer Lösung aus 43.6 g (143 mmol) 2-(-2-Bromophenyl)-2-phenyl-1,3-Dioxolan, gelöst in 200 ml wasserfreiem THF, werden bei -78 °C 65 ml (160 mmol) n-Butyllithium (2.5 M in Hexan) innerhalb von 30 Minuten zugegeben und dann auf 0 °C gebracht. Mit einer Spritze wird die lithiierte Verbindung in einen Tropftrichter überführt und langsam bei 0 °C in eine Suspension aus 48 g (140 mmol) 10-Phenyl-10H-10-aza-indeno[2,1-b]fluoren-12-on in 30 ml wasserfreiem THF getropft. Die Lösung wird auf Raumtemperatur gebracht und 4 h bei dieser Temperatur gehalten, dann wird mit gesättigter Ammoniumchlorid-Lösung versetzt. Die wässrige Phase wird mit CH₂Cl₂ (3 x 15 ml) extrahiert und die organischen Phasen über wasserfreiem Natriumsulfat getrocknet. Nach Entfernen des Lösemittels wird eine rötliche Flüssigkeit erhalten, welche eine Mischung mehrerer Isomere ist. Die Flüssigkeit wird in 100 ml Eisessig gelöst und unter Rückfluss erhitzt, dann werden wenige Tropfen konzentrierte HCl zugegeben und noch eine weitere Minute unter Rückfluss erhitzt. Es wird Wasser zugegeben, bis sich eine Trübung bildet, dann wird die Mischung auf Raumtemperatur gekühlt und filtriert. Die saure wässrige Phase wird mit CH₂Cl₂ extrahiert und über wasserfreiem Natriumsulfat getrocknet. Anschließend wird das Lösemittel am Rotationsverdampfer entfernt. Ausbeute: 34 g (68 mmol), 50% d. Th., Reinheit nach ¹H-NMR ca. 93%.

Analog werden folgende Verbindungen erhalten:

| **Bsp.** | **Edukt 1** | **Edukt 2** | **Produkt** | **Ausbeute** |
|---|---|---|---|---|
| 10 | 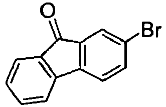 | 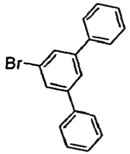 | 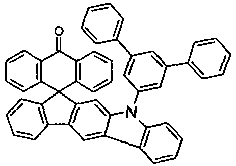 | 72 % |
| | **3096-56-8** | | | |
| 11 | 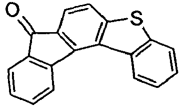 | es erfolgt nur der letzte Umsetzungsschritt im Schema | 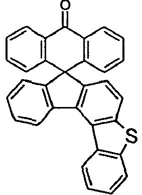 | 68 % |
| | **354816-92-5** | | | |
| 12 | 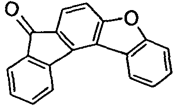 | es erfolgt nur der letzte Umsetzungsschritt im Schema | 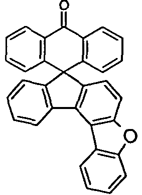 | 72 % |
| | **354816-91-4** | | | |
| 13 | 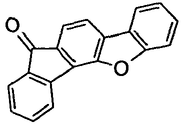 | es erfolgt nur der letzte Umsetzungsschritt im Schema | 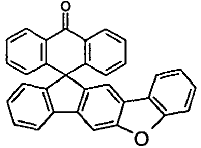 | 70 % |
| | **121073-95-8** | | | |

### 4. Synthese der erfindungsgemäßen Verbindungen 14-17

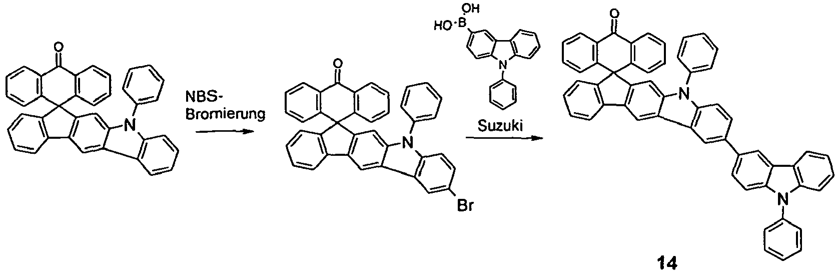

### NBS-Bromierung:

Es werden 20.4 g (40.18 mmol) des Indenocarbazol-Derivats (Beispiel 9) in 450 mL Acetonitril suspendiert und bei -20°C portionsweise mit 7.15 g (40.18 mmol) N-Bromsuccinimid versetzt, so dass die Reaktionstemperatur nicht über -20°C steigt. Es wird für 18 h nachgerührt. Dabei lässt man die Temperatur auf RT kommen. Die Reaktionsmischung wird anschließend einrotiert, mit Dichlormethan gelöst und mit Wasser gewaschen. Es wird getrocknet, eingeengt und anschließend aus Toluol bis auf eine Reinheit von 99.0% umkristallisiert. Man erhält 19 g (81%) des Produkts als weißen Feststoff.

### Suzuki-Reaktion (Synthese der Verbindung 14):

12 g (42 mmol) (9-Phenyl-carbazol-3yl)boronsäure und 30 g (52.4 mmol) des in 2-Position bromierten Indenocarbazol-Derivats (CAS 854952-58-2) werden in einem entgasten Gemisch aus 135 mL Wasser, 315 mL Dioxan und 315 mL Toluol gelöst und mit 5.33 g (50.31 mmol) Na₂CO₃ versetzt. Die Reaktionsmischung wird entgast und es wird mit 0.96 g (0.84 mmol) Pd-Tetrakis-Triphenylphosphin-Katalysator versetzt. Es wird für 18 h unter Rückfluss erhitzt. Nach dem Abkühlen wird mit Dichlormethan versetzt (heterogenes Gemisch), die Wasserphase wird abgetrennt und die organische Phase wird mit Toluol azeotrop eingeengt. Das Reaktionsprodukt wird aus DMSO kristallisiert und man erhält 29 g (67%) des Produkts mit einer Reinheit von 99.98% als weißen Feststoff.

Analog werden folgende Verbindungen erhalten:

| **Bsp.** | **Edukt 1** | **Edukt 2** | **Produkt** | **Ausbeute** |
|---|---|---|---|---|
| **15** | 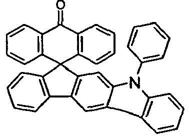 | 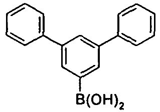 | 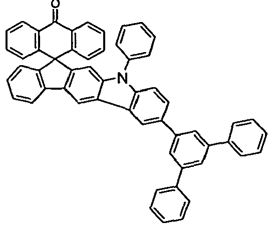 | 79 % |
| | | **128388-54-5** | | |
| **16** | 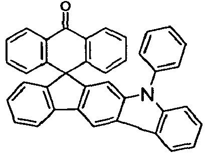 | 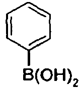 | 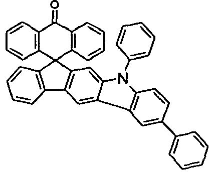 | 81 % |
| **17** | 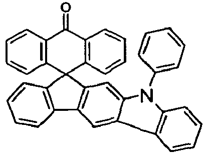 | 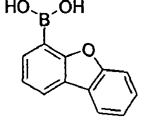 | 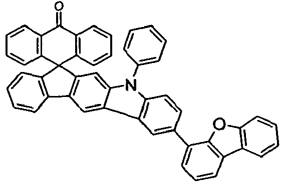 | 77 % |
| | | **100124-06-9** | | |

### 5. Device-Beispiele: Herstellung der OLEDs

Die Herstellung von erfindungsgemäßen OLEDs sowie OLEDs nach dem Stand der Technik erfolgt nach einem allgemeinen Verfahren gemäß WO 04/058911, das auf die hier beschriebenen Gegebenheiten (Schichtdickenvariation, Materialien) angepasst wird.

In den folgenden Beispielen V1-E19 (siehe Tabellen 1 und 2) werden die Daten verschiedener OLEDs vorgestellt. Glasplättchen, die mit strukturiertem ITO (Indium Zinn Oxid) der Dicke 50 nm beschichtet sind, werden zur verbesserten Prozessierung mit 20 nm PEDOT:PSS beschichtet (Poly(3,4-ethylenedioxythiophene)poly(styrenesulfonate), bezogen als CLEVIOS™ P VP Al 4083 von Heraeus Clevios Deutschland, aus wässriger Lösung aufgeschleudert, nach dem Aufschleudern 10 min bei 180° an Luft getrocknet). Diese beschichteten Glasplättchen bilden die Substrate, auf welche die OLEDs aufgebracht werden. Die OLEDs haben prinzipiell folgenden Schichtaufbau: Substrat / Optionale Lochinjektionsschicht (HIL) / Lochtransportschicht (HTL) / Optionale Zwischenschicht (IL) / Elektronenblockerschicht (EBL) / Emissionsschicht (EML) / Optionale Lochblockierschicht (HBL) / Elektronentransportschicht (ETL) / Optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 3 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie ST1:2:TEG1 (65%:25%:10%) bedeutet hierbei, dass das Material ST1 in einem Volumenanteil von 65%, das Material 2 in einem Anteil von 25% und TEG1 in einem Anteil von 10% in der Schicht vorliegt. Analog kann auch die Elektronentransportschicht aus einer Mischung aus zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik, sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe U1000 in Tabelle 2 bezeichnet die Spannung, die für eine Leuchtdichte von 1000 cd/m² benötigt wird. SE1000 und LE1000 bezeichnen die Strom- bzw. Leistungseffizienz, die bei 1000 cd/m² erreicht werden. EQE1000 schließlich bezeichnet die externe Quanteneffizienz bei einer Betriebsleuchtdichte von 1000 cd/m². Als Lebensdauer LD wird die Zeit definiert, nach der die Leuchtdichte bei einem Betrieb mit konstantem Strom von der Startleuchtdichte L0 auf einen gewissen Anteil L1 absinkt. Eine Angabe von L0 = 4000 cd/m² und L1 = 80% in Tabelle 2 bedeutet, dass die in Spalte LD angegebene Lebensdauer der Zeit entspricht, nach der die Anfangsleuchtdichte von 4000 cd/m² auf 3200 cd/m² absinkt.

Die Daten der verschiedenen OLEDs sind in Tabelle 2 zusammengefasst. Die Beispiele V1-V5 sind Vergleichsbeispiele gemäß dem Stand der Technik, die Beispiele E1-E19 zeigen Daten von OLEDs mit erfindungsgemäßen Materialien.

Im folgenden werden einige der Beispiele näher erläutert, um die Vorteile der erfindungsgemäßen Verbindungen zu verdeutlichen. Es sei jedoch darauf hingewiesen, dass dies nur eine Auswahl der in Tabelle 2 gezeigten Daten darstellt.

### Verwendung von erfindungsgemäßen Verbindungen als Matrixmaterialien für phosphoreszierende Dotanden

Bei Verwendung von Materialien gemäß dem Stand der Technik, welche zwischen der Spiro-Einheit und der elektronenziehenden Einheit ein Anthracen enthalten, erreicht man bei grüner Emission 8.1% und bei roter Emission 6.7% externe Quanteneffizienz. Die Lebensdauer bei einer Startleuchtdichte von 10000 cd/m² (grün, Abfall auf 70%) bzw. 4000 cd/m² (rot, Abfall auf 80%) liegt deutlich unter 100 h (Beispiele V1, V2). Diese Werte sind für phosphoreszente Dotanden sehr gering, was z.B. das Beispiel E2 mit erfindungsgemäßen Materialien zeigt: Es lassen sich mit den erfindungsgemäßen Verbindungen externe Quanteneffizienzen über 17% und Lebensdauern von über 250 h erreichen.

Ähnliche Verhältnisse ergeben sich, wenn man das Material S1 in Kombination mit einem zweiten Material in einem Mixed Matrix System verwendet (vgl. Beispiele V3, V4, E3, E5, E13, E14, E19).

Mit Materialien gemäß dem Stand der Technik, bei welchen die elektronenarme Gruppe (z. B. ein Triazin) an der Carbazoleinheit der Verbindung gebunden ist, erhält man bereits gute Leistungsdaten. Mit einer solchen Verbindung IC4 beispielsweise erreicht man eine externe Quanteneffizienz von fast 15%, eine Leistungseffizienz von 44 Im/W und eine Lebensdauer von 220 h. Bindet die elektronenarme Gruppe wie bei den erfindungsgemäßen Materialien jedoch an die Spirobifluoreneinheit, so erhält man eine deutliche Verbesserung der Leistungseffizienz um 25%, die externe Quanteneffizienz steigt um etwa 10%, die Lebensdauer um etwa 30% und die Betriebsspannung verbessert sich um 0.4 V (Beispiele V5, E1).

**Tabelle 1: Aufbau der OLEDs**

| Bsp. | HIL | HTL | IL | EBL | EML | HBL | ETL | EIL |
|---|---|---|---|---|---|---|---|---|
| | Dicke | Dicke | Dicke | Dicke | Dicke | Dicke | Dicke | Dicke |
| V1 | --- | SpA1 | HATCN | SpMA1 | S1:TEG1 (90%:10%) | --- | ST2:LiQ (50%:50%) | --- |
| | | 70nm | 5nm | 90nm | 30nm | | 40nm | |
| V2 | --- | SpA1 | HATCN | SpMA1 | S1:TER1 | IC1 | ST2:LiQ (50%:50%) | --- |
| | | 90nm | 5nm | 130nm | (92%:8%) 40nm | 10nm | 30nm | |
| V3 | --- | SpA1 | HATCN | SpMA1 | ST1:S1:TEG1 | IC1 | ST2:LiQ (50%:50%) | --- |
| | | 70nm | 5nm | 90nm | (60%:30%:10%) 30nm | 10nm | 30nm | |
| V4 | --- | SpA1 | HATCN | SpMA1 | S1:IC3:TEG1 | IC1 | ST2:LiQ (50%:50%) | --- |
| | | 70nm | 5nm | 90nm | (30%:60:%10%) 30nm | 10nm | 30nm | |
| V5 | --- | SpA1 | HATCN | SpMA1 | IC4:TEG1 (90%:10%) | --- | ST2:LiQ (50%:50%) | --- |
| | | 70nm | 5nm | 90nm | 30nm | | 40nm | |
| E1 | --- | SpA1 | HATCN | SpMA1 | 1:TEG1 (90%:10%) | --- | ST2:LiQ (50%:50%) | --- |
| | | 70nm | 5nm | 90nm | 30nm | | 40nm | |
| E2 | --- | SpA1 | HATCN | SpMA1 | IC1:TEG1 (90%:10%) | --- | 1 | LiQ |
| | | 70nm | 5nm | 90nm | 30nm | | 40nm | 3nm |
| E3 | HATCN | SpA1 | HATCN | SpMA1 | ST1:2:TEG1 | ST1 | ST2:LiQ (50%:50%) | --- |
| | 5nm | 140nm | 5nm | 20nm | (65%:25%:10%) 40nm | 5nm | 25nm | |
| E4 | --- | SpA1 | HATCN | SpMA1 | 3:TEG1 (90%:10%) | IC1 | ST2:LiQ (50%:50%) | --- |
| | | 70nm | 5nm | 90nm | 30nm | 10nm | 30nm | |
| E5 | --- | SpA1 | HATCN | SpMA1 | IC1:3:TEG1 | IC1 | ST2:LiQ (50%:50%) | --- |
| | | 70nm | 5nm | 90nm | (25%:65%:10%) 30nm | 10nm | 30nm | |
| E6 | --- | SpA1 | HATCN | SpMA1 | 4:TER1 | IC1 | ST2:LiQ (50%:50%) | --- |
| | | 90nm | 5nm | 130nm | (92%:8%) 40nm | 10nm | 30nm | |
| E7 | --- | SpA1 | HATCN | SpMA1 | 6:TER1 | IC1 | ST2:LiQ (50%:50%) | --- |
| | | 90nm | 5nm | 130nm | (92%:8%) 40nm | 10nm | 30nm | |
| E8 | --- | SpA1 | HATCN | SpMA1 | IC1:TEG1 (90%:10%) | --- | 7 | LiF |
| | | 70nm | 5nm | 90nm | 30nm | | 40nm | 1nm |
| E9 | --- | SpA1 | HATCN | SpMA1 | 7:TER1 | IC1 | ST2:LiQ (50%:50%) | --- |
| | | 90nm | 5nm | 130nm | (92%:8%) 40nm | 10nm | 30nm | |
| E10 | --- | SpA1 | HATCN | SpMA1 | 9:TEG1 (90%:10%) | - | ST2:LiQ (50%:50%) | --- |
| | | 70nm | 5nm | 90nm | 30nm | | 40nm | |
| E11 | --- | SpA1 | HATCN | SpMA1 | 9:TEG1 (90%:10%) | IC1 | ST2:LiQ (50%:50%) | --- |
| | | 70nm | 5nm | 90nm | 30nm | 10nm | 30nm | |
| E12 | --- | SpA1 | HATCN | SpMA1 | 10:TEG1 (90%:10%) | IC1 | ST2:LiQ (50%:50%) | --- |
| | | 70nm | 5nm | 90nm | 30nm | 10nm | 30nm | |
| E13 | --- | SpA1 | HATCN | SpMA1 | 11:IC3:TEG1 | IC1 | ST1:LiQ (50%:50%) | --- |
| | | 70nm | 5nm | 90nm | (65%:25%:10%) 30nm | 10nm | 30nm | |
| E14 | --- | SpA1 | HATCN | SpMA1 | 12:IC3:TEG1 | IC1 | ST1:LiQ (50%:50%) | --- |
| | | 70nm | 5nm | 90nm | (60%:30%:10%) 30nm | 10nm | 30nm | |
| E15 | --- | SpA1 | HATCN | SpMA1 | 13:TER1 | IC1 | ST2:LiQ (50%:50%) | --- |
| | | 90nm | 5nm | 130nm | (90%:10%) 40nm | 10nm | 30nm | |
| E16 | --- | SpA1 | HATCN | SpMA1 | 16:TEG1 (90%:10%) | IC1 | ST2:LiQ (50%:50%) | --- |
| | | 70nm | 5nm | 90nm | 30nm | 10nm | 30nm | |
| E17 | --- | SpA1 | HATCN | SpMA1 | 17:TEG1 (90%:10%) | IC1 | ST2:LiQ (50%:50%) | --- |
| | | 70nm | 5nm | 90nm | 30nm | 10nm | 30nm | |
| E18 | --- | SpA1 | HATCN | SpMA1 | 14:TEG1 (90%:10%) | IC1 | ST2:LiQ (50%:50%) | --- |
| | | 70nm | 5nm | 90nm | 30nm | 10nm | 30nm | |
| E19 | --- | SpA1 | HATCN | SpMA1 | IC2:14:TEG1 | IC1 | ST2:LiQ (50%:50%) | --- |
| | | 70nm | 5nm | 90nm | (40%:50%:10%) 30nm | 10nm | 30nm | |

**Tabelle 2: Daten der OLEDs**

| Bsp. | U1000 | SE1000 | LE1000 | EQE | CIE x/y bei | L0 | L1 | LD |
|---|---|---|---|---|---|---|---|---|
| | (V) | (cd/A) | (Im/W) | 1000 | 1000 cd/m2 | | % | (h) |
| V1 | 3.8 | 29 | 24 | 8.1% | 0.32/0.61 | 10000 | 70 | 70 |
| V2 | 4.8 | 6.2 | 4.1 | 6.7% | 0.66/0.33 | 4000 | 80 | 75 |
| V3 | 3.6 | 30 | 26 | 8.3% | 0.32/0.62 | 10000 | 70 | 95 |
| V4 | 3.7 | 27 | 23 | 7.4% | 0.32/0.61 | 10000 | 80 | 80 |
| V5 | 3.8 | 53 | 44 | 14.8% | 0.33/0.62 | 10000 | 70 | 220 |
| E1 | 3.4 | 59 | 55 | 16.5% | 0.33/0.62 | 10000 | 70 | 285 |
| E2 | 3.3 | 63 | 59 | 17.4% | 0.33/0.62 | 10000 | 70 | 255 |
| E3 | 3.1 | 64 | 65 | 17.7% | 0.33/0.63 | 8000 | 80 | 430 |
| E4 | 3.7 | 55 | 47 | 15.2% | 0.33/0.62 | 10000 | 80 | 210 |
| E5 | 3.6 | 56 | 49 | 15.5% | 0.33/0.63 | 10000 | 70 | 300 |
| E6 | 4.6 | 9.6 | 6.6 | 10.4% | 0.67/0.33 | 4000 | 80 | 330 |
| E7 | 4.8 | 12.1 | 8.0 | 13.1% | 0.67/0.33 | 4000 | 80 | 345 |
| E8 | 3.6 | 57 | 49 | 15.9% | 0.33/0.62 | 10000 | 70 | 230 |
| E9 | 4.7 | 11.0 | 7.4 | 11.9% | 0.67/0.33 | 4000 | 80 | 360 |
| E10 | 3.5 | 51 | 46 | 14.3% | 0.33/0.62 | 10000 | 80 | 195 |
| E11 | 3.5 | 58 | 51 | 16.0% | 0.33/0.62 | 10000 | 80 | 220 |
| E12 | 3.7 | 56 | 48 | 15.7% | 0.33/0.62 | 10000 | 80 | 235 |
| E13 | 3.6 | 55 | 48 | 15.3% | 0.34/0.62 | 10000 | 70 | 310 |
| E14 | 3.6 | 58 | 51 | 16.1% | 0.33/0.62 | 10000 | 70 | 335 |
| E15 | 4.6 | 11.1 | 7.6 | 12.1% | 0.67/0.33 | 4000 | 80 | 360 |
| E16 | 3.6 | 52 | 46 | 14.5% | 0.33/0.62 | 10000 | 70 | 230 |
| E17 | 3.8 | 55 | 45 | 15.2% | 0.33/0.62 | 10000 | 70 | 205 |
| E18 | 3.4 | 49 | 46 | 13.8% | 0.32/0.62 | 10000 | 70 | 240 |
| E19 | 3.3 | 59 | 55 | 16.2% | 0.33/0.62 | 10000 | 70 | 340 |

**Tabelle 3: Strukturformeln der Materialien für die OLEDs**

| | |
|---|---|
| 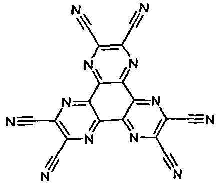 | 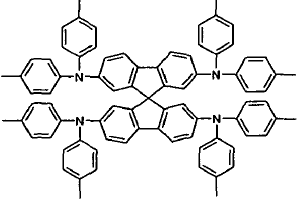 |
| HATCN | SpA1 (Stand der Technik) |
| 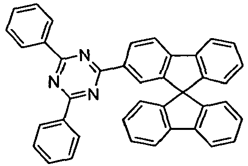 | 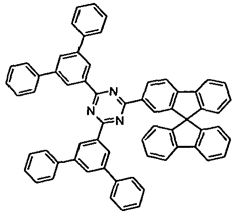 |
| ST1 | ST2 |
| 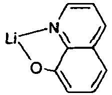 | 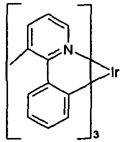 |
| LiQ | TEG1 |
| 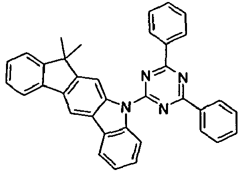 | 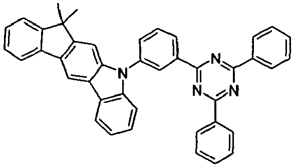 |
| IC1 | IC2 |
| 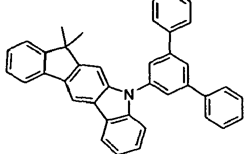 | 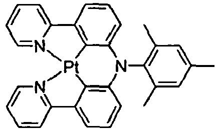 |
| IC3 | TER1 |
| 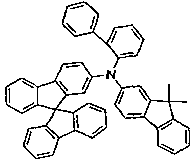 | 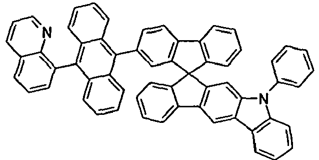 |
| SpMA1 | S1 (Stand der Technik) |
| 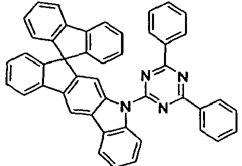 | 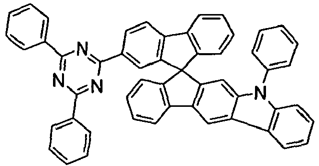 |
| IC4 (Stand der Technik) | 1 |
| 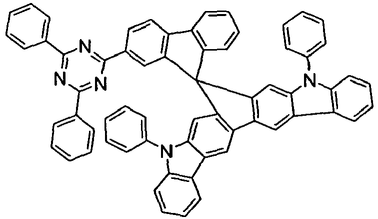 | 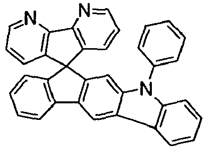 |
| 2 | 3 |
| 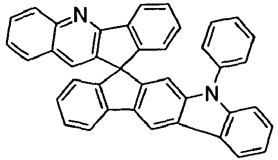 | 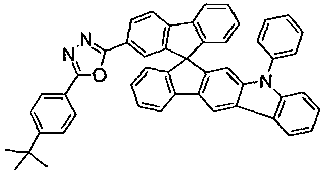 |
| 4 | 5 |
| 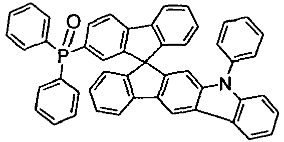 | 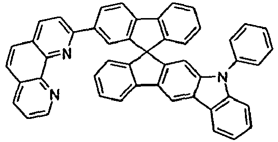 |
| 6 | 7 |
| 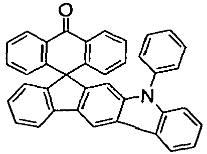 | 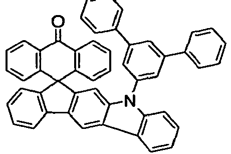 |
| 9 | 10 |
| 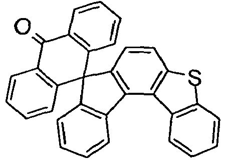 | 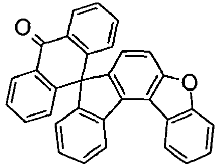 |
| 11 | 12 |
| 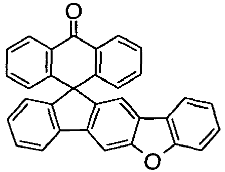 | 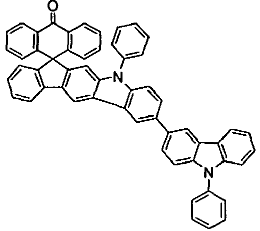 |
| 13 | 14 |
| 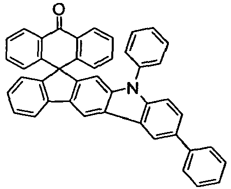 | 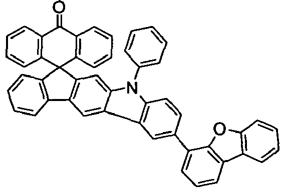 |
| 16 | 17 |

## Patentansprüche

1. Verbindung einer Formel (I) oder (II) 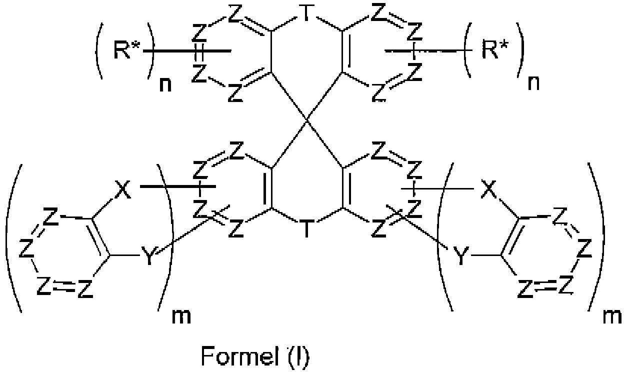 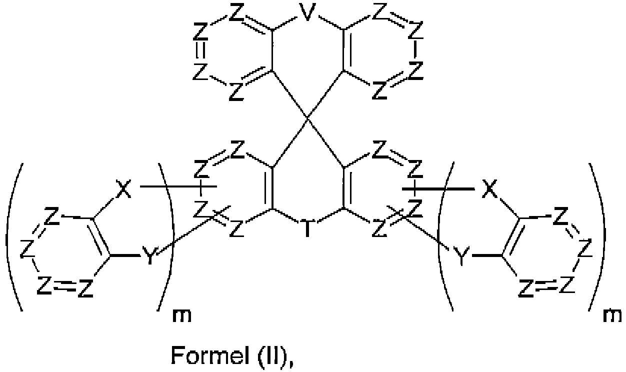 wobei für die auftretenden Symbole und Indices gilt:
R* ist bei jedem Auftreten gleich oder verschieden eine elektronenarme Heteroarylgruppe mit 5 bis 14 aromatischen Ringatomen oder eine Ketogruppe oder eine Phosphoroxidgruppe oder eine Schwefeloxidgruppe, welche jeweils direkt oder über eine Gruppe L gebunden sind und welche mit einem oder mehreren Resten R² substituiert sein können, oder-CN;
L ist bei jedem Auftreten gleich oder verschieden eine divalente Gruppe der Formel -(Ar¹)ₖ-, wobei
Ar¹ bei jedem Auftreten gleich oder verschieden eine Arylen- oder Heteroarylengruppe mit 5 bis 10 aromatischen Ringatomen darstellt, welche mit einem oder mehreren Resten R² substituiert sein kann; und
k bei jedem Auftreten gleich oder verschieden gewählt ist aus 1, 2, 3, 4 oder 5;
X, Y sind bei jedem Auftreten gleich oder verschieden ausgewählt aus einer Einfachbindung, C(R¹)₂, NR¹, PR¹, P(O)R¹, O, S, SO und SO₂, wobei Fälle ausgeschlossen sind, in denen keine der beiden Gruppen X und Y eines Rings ausgewählt ist aus NR¹, PR¹, P(O)R¹, O, S, SO und SO₂;
V ist ausgewählt aus einer Einfachbindung, CO, CS, P(O)R¹, SO und SO₂, wobei V nur dann eine Einfachbindung sein kann, wenn mindestens eine der Gruppen Z in den an V gebundenen Ringen gleich N ist;
T ist bei jedem Auftreten gleich oder verschieden ausgewählt aus einer Einfachbindung, C(R¹)₂, CO, CS, Si(R¹)₂, NR¹, PR¹, P(O)R¹, O, S, SO und SO₂;
Z ist bei jedem Auftreten gleich oder verschieden ausgewählt aus CR¹ und N, wenn keine Gruppe an Z gebunden ist, und ist gleich C, wenn eine Gruppe an Z gebunden ist;
R¹ ist bei jedem Auftreten gleich oder verschieden H, D, F, Cl, Br, I, B(OR²)₂, CHO, C(=O)R², CR²=C(R²)₂, CN, C(=O)OR², C(=O)N(R²)₂, Si(R²)₃, N(R²)₂, NO₂, P(=O)(R²)₂, OSO₂R², OR², S(=O)R², S(=O)₂R², eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R² substituiert sein können und wobei eine oder mehrere CH₂-Gruppen in den oben genannten Gruppen durch -R²C=CR²-, -CΞC-, Si(R²)₂, C=O, C=S, C=NR², -C(=O)O-, -C(=O)NR²-, NR², P(=O)(R²), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 30 aromatischen Ringatomen, das jeweils durch einen oder mehrere Reste R² substituiert sein kann, oder eine Aryloxy- oder Heteroaryloxygruppe mit 5 bis 30 aromatischen Ringatomen, die durch einen oder mehrere Reste R² substituiert sein kann, wobei zwei oder mehr Reste R¹ miteinander verknüpft sein können und einen Ring bilden können;
R² ist bei jedem Auftreten gleich oder verschieden H, D, F, Cl, Br, I, B(OR³)₂, CHO, C(=O)R³, CR³=C(R³)₂, CN, C(=O)OR³, C(=O)N(R³)₂, Si(R³)₃, N(R³)₂, NO₂, P(=O)(R³)₂, OSO₂R³, OR³, S(=O)R³, S(=O)₂R³, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch -R³C=CR³-, -CΞC-, Si(R³)₂, C=O, C=S, C=NR³, -C(=O)O-, -C(=O)NR³-, NR³, P(=O)(R³), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 30 aromatischen Ringatomen, das jeweils durch einen oder mehrere Reste R³ substituiert sein kann, oder eine Aryloxy- oder Heteroaryloxygruppe mit 5 bis 30 aromatischen Ringatomen, die durch einen oder mehrere Reste R³ substituiert sein kann;
R³ ist bei jedem Auftreten gleich oder verschieden H, D, F oder ein aliphatischer, aromatischer oder heteroaromatischer organischer Rest mit 1 bis 20 C-Atomen, in dem auch ein oder mehrere H-Atome durch D oder F ersetzt sein können; dabei können zwei oder mehr Substituenten R³ miteinander verknüpft sein und einen Ring bilden;
m ist bei jedem Auftreten gleich oder verschieden 0 oder 1, wobei nicht beide Indices m in einer Formel gleich 0 sein können;
n ist bei jedem Auftreten gleich oder verschieden 0 oder 1, wobei nicht beide Indices n in einer Formel gleich 0 sein können,
und wobei die Gruppen X und Y jeweils in benachbarten Positionen am Sechsring des Spirobifluorenderivats binden.

2. Verbindung nach Anspruch 1, **dadurch gekennzeichnet, dass** sie keine kondensierten Arylgruppen mit mehr als 16 aromatischen Ringatomen aufweist.

3. Verbindung nach Anspruch 1 oder 2, **dadurch gekennzeichnet, dass** an die Gruppen X und Y keine Heteroarylgruppe, keine Ketogruppe, keine Phosphoroxidgruppe und keine Schwefeloxidgruppe gebunden ist.

4. Verbindung nach einem oder mehreren der Ansprüche 1 bis 3, **dadurch gekennzeichnet, dass** der Rest R* eine Gruppe -CN oder eine Ketogruppe der Formel (K) 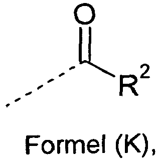 oder eine Phosphoroxidgruppe der Formel (P) 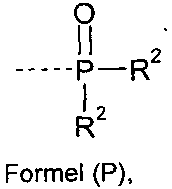 oder eine Schwefeloxidgruppe der Formel (S) 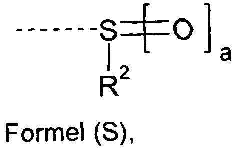 wobei a gleich 1 oder 2 sein kann, darstellt,
oder eine Gruppe der untenstehend aufgeführten Formeln (H-1) bis (H-10) darstellt 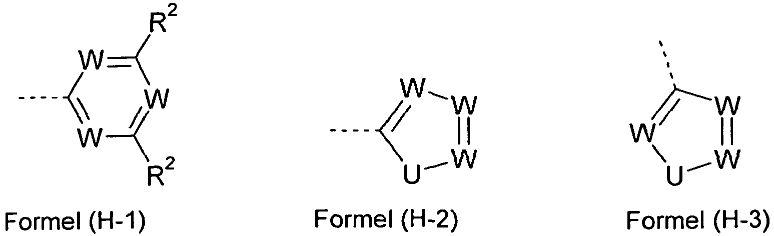 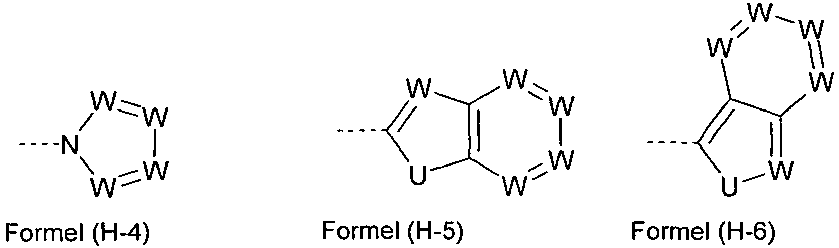 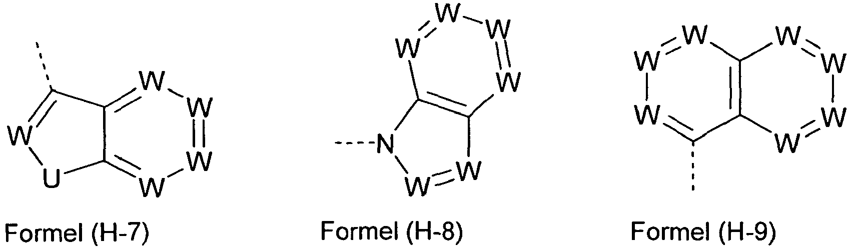 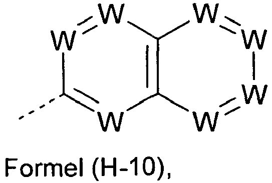 wobei die gestrichelte Bindung die Anbindungsposition markiert, R² wie in Anspruch 1 definiert ist und
W bei jedem Auftreten gleich oder verschieden CR² oder N ist, wobei mindestens eine Gruppe W pro Formel gleich N ist, und
U NR², O oder S darstellt.

5. Verbindung nach einem oder mehreren der Ansprüche 1 bis 4, **dadurch gekennzeichnet, dass** die Gruppe R* in der Position 2' oder in der Position 7' am modifizierten Spirobifluoren-Grundgerüst gebunden ist.

6. Verbindung nach einem oder mehreren der Ansprüche 1 bis 5, **dadurch gekennzeichnet, dass** die Gruppen X und Y gleich oder verschieden ausgewählt sind aus einer Einfachbindung, C(R¹)₂, NR¹, O und S, wobei Fälle ausgeschlossen sind, in denen keine der beiden Gruppen X und Y eines Rings ausgewählt ist aus NR¹, O und S.

7. Verbindung nach einem oder mehreren der Ansprüche 1 bis 6, **dadurch gekennzeichnet, dass** eine der beiden Gruppen X und Y in einem Ring eine Einfachbindung darstellt und die andere der beiden Gruppen X und Y eine Gruppe NR¹ darstellt.

8. Verbindung nach einem oder mehreren der Ansprüche 1 bis 7, **dadurch gekennzeichnet, dass** die Gruppe T bei jedem Auftreten gleich oder verschieden ausgewählt ist aus einer Einfachbindung, C(R¹)₂, O und S.

9. Verbindung nach einem oder mehreren der Ansprüche 1 bis 8, **dadurch gekennzeichnet, dass** einer der beiden Indices m pro Formel gleich 1 ist und der andere gleich Null ist.

10. Verbindung nach einem oder mehreren der Ansprüche 1 bis 9, **dadurch gekennzeichnet, dass** einer der beiden Indices n pro Formel gleich 1 ist und der andere gleich Null ist.

11. Oligomer, Polymer oder Dendrimer enthaltend eine oder mehrere Verbindungen nach einem oder mehreren der Ansprüche 1 bis 10, wobei die Bindung(en) zum Polymer, Oligomer oder Dendrimer an beliebigen, in Formel (I) oder (II) mit R¹, oder R² substituierten Positionen lokalisiert sein können.

12. Formulierung enthaltend mindestens eine Verbindung nach einem oder mehreren der Ansprüche 1 bis 10 oder mindestens ein Polymer, Oligomer oder Dendrimer nach Anspruch 11 sowie mindestens ein Lösungsmittel.

13. Verfahren zur Herstellung einer Verbindung nach einem oder mehreren der Ansprüche 1 bis 10, **dadurch gekennzeichnet, dass**
- an eine Spirobifluorengruppe, welche mit einer elektronenarmen Gruppe substituiert ist, eine Heteroarylgruppe ankondensiert wird, oder dass
- eine Zyklisierungsreaktion durchgeführt wird, über die eine modifizierte Spirobifluorengruppe erhalten wird.

14. Elektronische Vorrichtung, enthaltend mindestens eine Verbindung nach einem oder mehreren der Ansprüche 1 bis 10 oder mindestens ein Polymer, Dendrimer oder Oligomer nach Anspruch 11.

15. Elektronische Vorrichtung nach Anspruch 14, **dadurch gekennzeichnet, dass** sie ausgewählt ist aus der Gruppe bestehend aus organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und organischen Elektrolumineszenzvorrichtungen (OLEDs).

16. Elektronische Vorrichtung nach Anspruch 15, **dadurch gekennzeichnet, dass** sie ausgewählt ist aus organischen Elektrolumineszenzvorrichtungen und dass sie mindestens eine Verbindung nach einem oder mehreren der Ansprüche 1 bis 10 oder mindestens ein Polymer, Dendrimer oder Oligomer nach Anspruch 11 in einer oder mehreren der folgenden Funktionen enthält:
- als Matrixmaterial in einer emittierenden Schicht,
- als Elektronentransportmaterial in einer Elektronentransport- oder Elektroneninjektionsschicht,
- als Lochblockiermaterial in einer Lochblockierschicht.

## Claims

1. Compound of a formula (I) or (II) 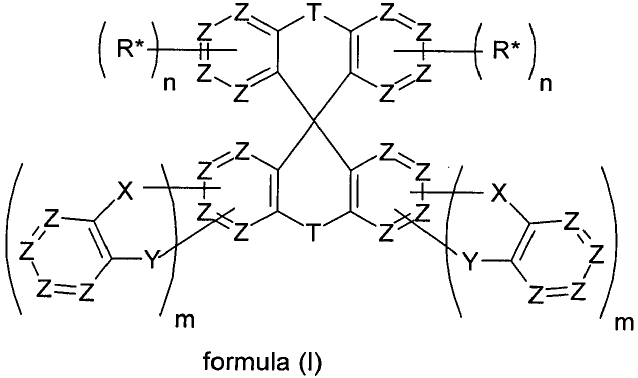 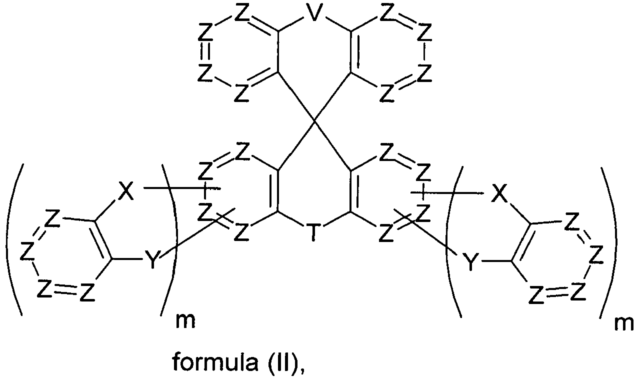 where the following applies to the symbols and indices occurring:
R* is on each occurrence, identically or differently, an electron-deficient heteroaryl group having 5 to 14 aromatic ring atoms or a keto group or a phosphorus oxide group or a sulfur oxide group, each of which is bonded directly or via a group L and which may be substituted by one or more radicals R², or -CN;
L is on each occurrence, identically or differently, a divalent group of the formula -(Ar¹)ₖ-, where
Ar¹ represents on each occurrence, identically or differently, an arylene or heteroarylene group having 5 to 10 aromatic ring atoms, which may be substituted by one or more radicals R²; and
k is selected on each occurrence, identically or differently, from 1, 2, 3, 4 or 5;
X, Y are selected on each occurrence, identically or differently, from a single bond, C(R¹)₂, NR¹, PR¹, P(O)R¹, O, S, SO and SO₂, where cases in which neither of the two groups X and Y of a ring is selected from NR¹, PR¹, P(O)R¹, O, S, SO and SO₂ are excluded;
V is selected from a single bond, CO, CS, P(O)R¹, SO and SO₂, where V may only be a single bond if at least one of the groups Z in the rings bonded to V is equal to N;
T is selected on each occurrence, identically or differently, from a single bond, C(R¹)₂, CO, CS, Si(R¹)₂, NR¹, PR¹, P(O)R¹, O, S, SO and SO₂;
Z is selected on each occurrence, identically or differently, from CR¹ and N if no group is bonded to Z and is equal to C if a group is bonded to Z;
R¹ is on each occurrence, identically or differently, H, D, F, Cl, Br, I, B(OR²)₂, CHO, C(=O)R², CR²=C(R²)₂, CN, C(=O)OR², C(=O)N(R²)₂, Si(R²)₃, N(R²)₂, NO₂, P(=O)(R²)₂, OSO₂R², OR², S(=O)R², S(=O)₂R², a straight-chain alkyl, alkoxy or thioalkyl group having 1 to 20 C atoms or a branched or cyclic alkyl, alkoxy or thioalkyl group having 3 to 20 C atoms or an alkenyl or alkynyl group having 2 to 20 C atoms, where the above-mentioned groups may each be substituted by one or more radicals R² and where one or more CH₂ groups in the above-mentioned groups may be replaced by -R²C=CR²-, -C≡C-, Si(R²)₂, C=O, C=S, C=NR², -C(=O)O-, -C(=O)NR²-, NR², P(=O)(R²), -O-, -S-, SO or SO₂ and where one or more H atoms in the above-mentioned groups may be replaced by D, F, Cl, Br, I, CN or NO₂, or an aromatic or heteroaromatic ring system having 5 to 30 aromatic ring atoms, which may in each case be substituted by one or more radicals R², or an aryloxy or heteroaryloxy group having 5 to 30 aromatic ring atoms, which may be substituted by one or more radicals R², where two or more radicals R¹ may be linked to one another and may form a ring;
R² is on each occurrence, identically or differently, H, D, F, Cl, Br, I, B(OR³)₂, CHO, C(=O)R³, CR³=C(R³)₂, CN, C(=O)OR³, C(=O)N(R³)₂, Si(R³)₃, N(R³)₂, NO₂, P(=O)(R³)₂, OSO₂R³, OR³, S(=O)R³, S(=O)₂R³, a straight-chain alkyl, alkoxy or thioalkyl group having 1 to 20 C atoms or a branched or cyclic alkyl, alkoxy or thioalkyl group having 3 to 20 C atoms or an alkenyl or alkynyl group having 2 to 20 C atoms, where the above-mentioned groups may each be substituted by one or more radicals R³ and where one or more CH₂ groups in the above-mentioned groups may be replaced by -R³C=CR³-, -C≡C-, Si(R³)₂, C=O, C=S, C=NR³, -C(=O)O-, -C(=O)NR³-, NR³, P(=O)(R³), -O-, -S-, SO or SO₂ and where one or more H atoms in the above-mentioned groups may be replaced by D, F, Cl, Br, I, CN or NO₂, or an aromatic or heteroaromatic ring system having 5 to 30 aromatic ring atoms, which may in each case be substituted by one or more radicals R³, or an aryloxy or heteroaryloxy group having 5 to 30 aromatic ring atoms, which may be substituted by one or more radicals R³;
R³ is on each occurrence, identically or differently, H, D, F or an aliphatic, aromatic or heteroaromatic organic radical having 1 to 20 C atoms, in which, in addition, one or more H atoms may be replaced by D or F; two or more substituents R³ here may be linked to one another and may form a ring;
m is on each occurrence, identically or differently, 0 or 1, where both indices m in a formula cannot be equal to 0;
n is on each occurrence, identically or differently, 0 or 1, where both indices n in a formula cannot be equal to 0,
and where the groups X and Y are each bonded in adjacent positions on the six-membered ring of the spirobifluorene derivative.

2. Compound according to Claim 1, **characterised in that** it contains no condensed aryl groups having more than 16 aromatic ring atoms.

3. Compound according to Claim 1 or 2, **characterised in that** no heteroaryl group, no keto group, no phosphorus oxide group and no sulfur oxide group is bonded to the groups X and Y.

4. Compound according to one or more of Claims 1 to 3, **characterised in that** the radical R* represents a group -CN or a keto group of the formula (K) 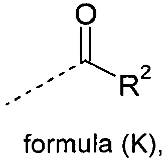 or a phosphorus oxide group of the formula (P) 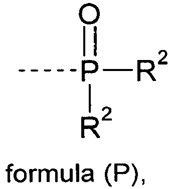 or a sulfur oxide group of the formula (S) 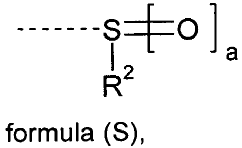 where a can be equal to 1 or 2,
or a group of the formulae (H-1) to (H-10) shown below 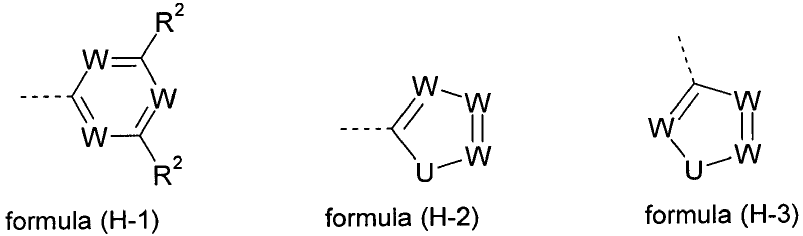 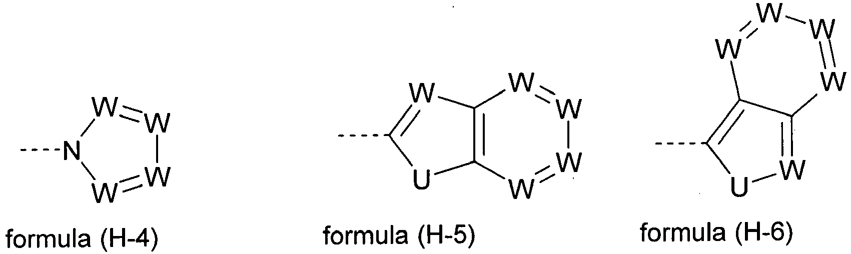 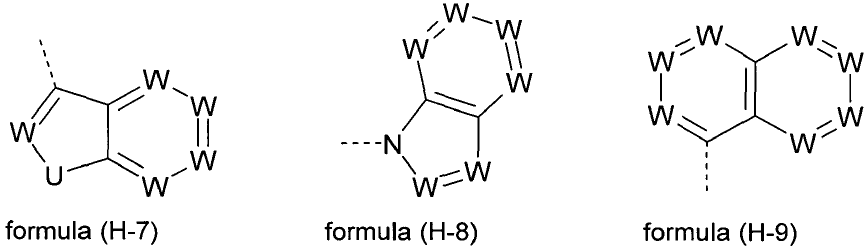 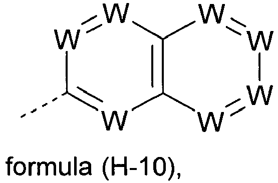 where the dashed bond marks the bonding position, R² is as defined in Claim 1 and
W is on each occurrence, identically or differently, CR² or N, where at least one group W per formula is equal to N, and
U represents NR², O or S.

5. Compound according to one or more of Claims 1 to 4, **characterised in that** the group R* is bonded to the modified spirobifluorene skeleton in position 2' or in position 7'.

6. Compound according to one or more of Claims 1 to 5, **characterised in that** the groups X and Y are selected, identically or differently, from a single bond, C(R¹)₂, NR¹, O and S, where cases in which neither of the two groups X and Y of a ring is selected from NR¹, O and S are excluded.

7. Compound according to one or more of Claims 1 to 6, **characterised in that** one of the two groups X and Y in a ring represents a single bond and the other of the two groups X and Y represents a group NR¹.

8. Compound according to one or more of Claims 1 to 7, **characterised in that** the group T is selected on each occurrence, identically or differently, from a single bond, C(R¹)₂, O and S.

9. Compound according to one or more of Claims 1 to 8, **characterised in that** one of the two indices m per formula is equal to 1 and the other is equal to zero.

10. Compound according to one or more of Claims 1 to 9, **characterised in that** one of the two indices n per formula is equal to 1 and the other is equal to zero.

11. Oligomer, polymer or dendrimer containing one or more compounds according to one or more of Claims 1 to 10, where the bond(s) to the polymer, oligomer or dendrimer may be localised at any desired positions in formula (I) or (II) that are substituted by R¹ or R².

12. Formulation comprising at least one compound according to one or more of Claims 1 to 10 or at least one polymer, oligomer or dendrimer according to Claim 11 and at least one solvent.

13. Process for the preparation of a compound according to one or more of Claims 1 to 10, **characterised in that**
- a heteroaryl group is condensed onto a spirobifluorene group which is substituted by an electron-deficient group, or **in that**
- a cyclisation reaction is carried out via which a modified spirobifluorene group is obtained.

14. Electronic device comprising at least one compound according to one or more of Claims 1 to 10 or at least one polymer, dendrimer or oligomer according to Claim 11.

15. Electronic device according to Claim 14, **characterised in that** it is selected from the group consisting of organic integrated circuits (O-ICs), organic field-effect transistors (O-FETs), organic thin-film transistors (O-TFTs), organic light-emitting transistors (O-LETs), organic solar cells (O-SCs), organic optical detectors, organic photoreceptors, organic field-quench devices (O-FQDs), light-emitting electrochemical cells (LECs), organic laser diodes (O-lasers) and organic electroluminescent devices (OLEDs).

16. Electronic device according to Claim 15, **characterised in that** it is selected from organic electroluminescent devices and **in that** it comprises at least one compound according to one or more of Claims 1 to 10 or at least one polymer, dendrimer or oligomer according to Claim 11 in one or more of the following functions:
- as matrix material in an emitting layer,
- as electron-transport material in an electron-transport or electron-injection layer,
- as hole-blocking material in a hole-blocking layer.

## Revendications

1. Composé de la formule (I) ou (II) : 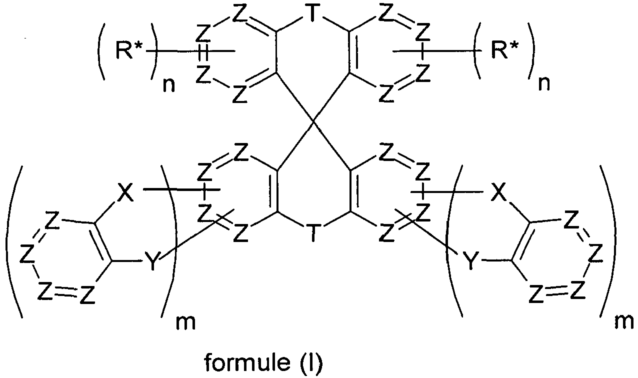 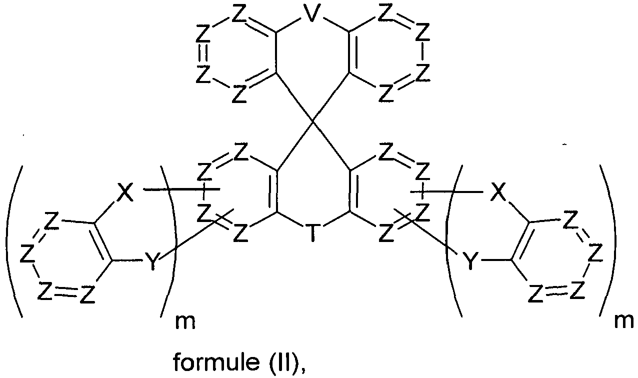 dans lesquelles ce qui suit s'applique aux symboles et indices rencontrés :
R* est pour chaque occurrence, de manière identique ou différente, un groupe hétéroaryle déficient en électrons comportant 5 à 14 atomes de cycle aromatique ou un groupe céto ou un groupe oxyde de phosphore ou un groupe oxyde de soufre, dont chacun est lié directement ou via un groupe L et peut être substitué par un radical ou plusieurs radicaux R², ou par -CN ;
L est pour chaque occurrence, de manière identique ou différente, un groupe divalent de la formule -(Ar¹)ₖ-, dans laquelle :
Ar¹ représente pour chaque occurrence, de manière identique ou différente, un groupe arylène ou hétéroarylène qui comporte 5 à 10 atomes de cycle aromatique, lequel peut être substitué par un radical ou plusieurs radicaux R² ; et
k est choisi pour chaque occurrence, de manière identique ou différente, parmi 1, 2, 3, 4 ou 5 ;
X, Y sont choisis pour chaque occurrence, de manière identique ou différente, parmi une liaison simple, C(R¹)₂, NR¹, PR¹, P(O)R¹, O, S, SO et SO₂, où les cas dans lesquels aucun des deux groupes X et Y d'un cycle n'est choisi parmi NR¹, PR¹, P(O)R¹, O, S, SO et SO₂ sont exclus ;
V est choisi parmi une liaison simple, CO, CS, P(O)R¹, SO et SO₂, où V peut seulement être une liaison simple si au moins l'un des groupes Z dans les cycles liés à V est égal à N ;
T est choisi pour chaque occurrence, de manière identique ou différente, parmi une liaison simple, C(R¹)₂, CO, CS, Si(R¹)₂, NR¹, PR¹, P(O)R¹, O, S, SO et SO₂ ;
Z est choisi pour chaque occurrence, de manière identique ou différente, parmi CR¹ et N si aucun groupe n'est lié à Z et est égal à C si un groupe est lié à Z ;
R¹ est pour chaque occurrence, de manière identique ou différente, H, D, F, Cl, Br, I, B(OR²)₂, CHO, C(=O)R²; CR²=C(R²)₂, CN, C(=O)OR², C(=O)N(R²)₂, Si(R²)₃, N(R²)₂, NO₂, P(=O)(R²)₂, OSO₂R², OR², S(=O)R², S(=O)₂R², un groupe alkyle, alcoxy ou thioalkyle en chaîne droite comportant 1 à 20 atome(s) de C ou un groupe alkyle, alcoxy ou thioalkyle ramifié ou cyclique comportant 3 à 20 atomes de C ou un groupe alkényle ou alkynyle comportant 2 à 20 atomes de C, où les groupes mentionnés ci avant peuvent chacun être substitués par un radical ou plusieurs radicaux R² et où un ou plusieurs groupe(s) CH₂ dans les groupes mentionnés ci avant peut/peuvent être remplacé(s) par -R²C=CR²-, -CΞC-, Si(R²)₂, C=O, C=S, C=NR², -C(=O)O-, -C(=O)NR²-, NR², P(=O)(R²), -O-, -S-, SO ou SO₂ et où un ou plusieurs atome(s) de H dans les groupes mentionnés ci avant peut/ peuvent être remplacé(s) par D, F, Cl, Br, I, CN ou NO₂, ou un système de cycle aromatique ou hétéroaromatique comportant 5 à 30 atomes de cycle aromatique, lequel peut dans chaque cas être substitué par un radical ou plusieurs radicaux R², ou un groupe aryloxy ou hétéroaryloxy comportant 5 à 30 atomes de cycle aromatique, lequel peut être substitué par un radical ou plusieurs radicaux R², où deux radicaux R¹ ou plus peuvent être liés l'un à l'autre ou les uns aux autres et peuvent former un cycle;
R² est pour chaque occurrence, de manière identique ou différente, H, D, F, Cl, Br, I, B(OR³)₂, CHO, C(=O)R³, CR³=C(R³)₂, CN, C(=O)OR³, C(=O)N(R³)₂, Si(R³)₃, N(R³)₂, NO₂, P(=O)(R³)₂, OSO₂R³, OR³, S(=O)R³, S(=O)₂R³, un groupe alkyle, alcoxy ou thioalkyle en chaîne droite comportant 1 à 20 atome(s) de C ou un groupe alkyle, alcoxy ou thioalkyle ramifié ou cyclique comportant 3 à 20 atomes de C ou un groupe alkényle ou alkynyle comportant 2 à 20 atomes de C, où les groupes mentionnés ci avant peuvent chacun être substitués par un radical ou plusieurs radicaux R³ et où un ou plusieurs groupe(s) CH₂ dans les groupes mentionnés ci avant peut/ peuvent être remplacé(s) par -R³C=CR³-, -CΞC-, Si(R³)₂, C=O, C=S, C=NR³, -C(=O)O-, -C(=O)NR³-, NR³, P(=O)(R³), -O-, -S-, SO ou SO₂ et où un ou plusieurs atome(s) de H dans les groupes mentionnés ci avant peut/peuvent être remplacé(s) par D, F, Cl, Br, I, CN ou NO₂, ou un système de cycle aromatique ou hétéroaromatique comportant 5 à 30 atomes de cycle aromatique, lequel peut dans chaque cas être substitué par un radical ou plusieurs radicaux R³, ou un groupe aryloxy ou hétéroaryloxy comportant 5 à 30 atomes de cycle aromatique, lequel peut être substitué par un radical ou plusieurs radicaux R³ ;
R³ est pour chaque occurrence, de manière identique ou différente, H, D, F ou un radical organique aliphatique, aromatique ou hétéroaromatique comportant 1 à 20 atome(s) de C, où, en outre, un ou plusieurs atome(s) de H peut/peuvent être remplacé(s) par D ou F ; deux substituants R³ ou plus peuvent ici être liés l'un à l'autre ou les uns aux autres et peuvent former un cycle ;
m est pour chaque occurrence, de manière identique ou différente, 0 ou 1, où les deux indices m dans une formule ne peuvent pas être égaux à 0 ;
n est pour chaque occurrence, de manière identique ou différente, 0 ou 1, où les deux indices n dans une formule ne peuvent pas être égaux à 0,
et où les groupes X et Y sont chacun liés au niveau de positions adjacentes sur le cycle à six éléments du dérivé de spirobifluorène.

2. Composé selon la revendication 1, **caractérisé en ce qu'**il ne contient pas de groupes aryle condensés comportant plus de 16 atomes de cycle aromatique.

3. Composé selon la revendication 1 ou 2, **caractérisé en ce qu'**aucun groupe hétéroaryle, aucun groupe céto, aucun groupe oxyde de phosphore et aucun groupe oxyde de soufre ne sont liés aux groupes X et Y.

4. Composé selon une ou plusieurs des revendications 1 à 3, **caractérisé en ce que** le radical R* représente un groupe -CN ou un groupe céto de la formule (K) : 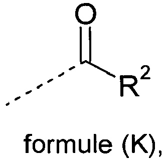 ou un groupe oxyde de phosphore de la formule (P) : 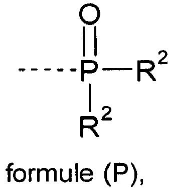 ou un groupe oxyde de soufre de la formule (S) : 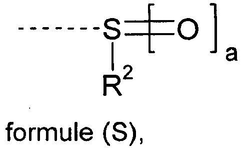 dans laquelle a peut être égal à 1 ou 2,
ou un groupe des formules (H-1) à (H-10) présentées ci après : 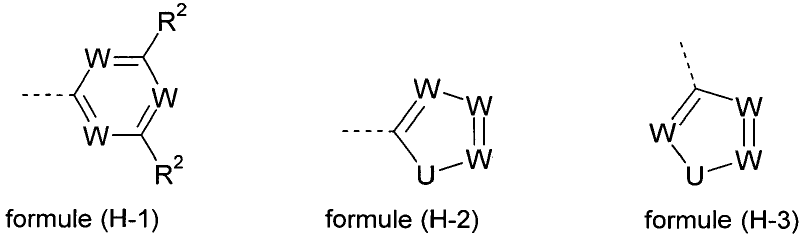 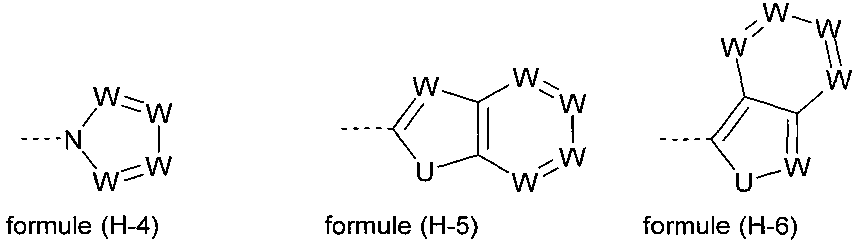 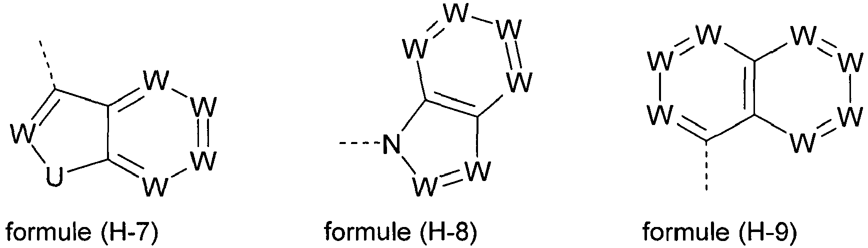 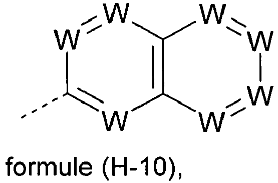 dans lesquelles la liaison en pointillés indique la position de liaison, R² est comme défini selon la revendication 1 et
W est pour chaque occurrence, de manière identique ou différente, CR² ou N, où au moins un groupe W par formule est égal à N, et
U représente NR², O ou S.

5. Composé selon une ou plusieurs des revendications 1 à 4, **caractérisé en ce que** le groupe R* est lié au squelette spirobifluorène modifié à la position 2' ou à la position 7'.

6. Composé selon une ou plusieurs des revendications 1 à 5, **caractérisé en ce que** les groupes X et Y sont choisis, de manière identique ou différente, parmi une liaison simple, C(R¹)₂, NR¹, O et S, où les cas dans lesquels aucun des deux groupes X et Y d'un cycle n'est choisi parmi NR¹, O et S sont exclus.

7. Composé selon une ou plusieurs des revendications 1 à 6, **caractérisé en ce que** l'un des deux groupes X et Y dans un cycle représente une liaison simple et l'autre des deux groupes X et Y représente un groupe NR¹.

8. Composé selon une ou plusieurs des revendications 1 à 7, **caractérisé en ce que** le groupe T est choisi pour chaque occurrence, de manière identique ou différente, parmi une liaison simple, C(R¹)₂, O et S.

9. Composé selon une ou plusieurs des revendications 1 à 8, **caractérisé en ce que** l'un des deux indices m par formule est égal à 1 et l'autre est égal à zéro.

10. Composé selon une ou plusieurs des revendications 1 à 9, **caractérisé en ce que** l'un des deux indices n par formule est égal à 1 et l'autre est égal à zéro.

11. Oligomère, polymère ou dendrimère contenant un ou plusieurs composé(s) selon une ou plusieurs des revendications 1 à 10, où la/les liaison(s) sur le polymère, l'oligomère ou le dendrimère peut/peuvent être située(s) en n'importe quelle(s) position(s) souhaitée(s) dans la formule (I) ou (II) qui est/sont substituée(s) par R¹ ou par R².

12. Formulation comprenant au moins un composé selon une ou plusieurs des revendications 1 à 10 ou au moins un polymère, un oligomère ou un dendrimère selon la revendication 11 et au moins un solvant.

13. Procédé pour la préparation d'un composé selon une ou plusieurs des revendications 1 à 10, **caractérisé en ce que** :
- un groupe hétéroaryle est condensé sur un groupe spirobifluorène qui est substitué par un groupe déficient en électrons, ou **en ce que** :
- une réaction de cyclisation est mise en oeuvre, réaction via laquelle un groupe spirobifluorène modifié est obtenu.

14. Dispositif électronique comprenant au moins un composé selon une ou plusieurs des revendications 1 à 10 ou au moins un polymère, un dendrimère ou un oligomère selon la revendication 11.

15. Dispositif électronique selon la revendication 14, **caractérisé en ce qu'**il est choisi parmi le groupe constitué par les circuits intégrés organiques (O-IC), les transistors à effet de champ organiques (O-FET), les transistors à film mince organiques (O-TFT), les transistors à émission de lumière organiques (O-LET), les cellules solaires organiques (O-SC), les détecteurs optiques organiques, les photorécepteurs organiques, les dispositifs à extinction de champ organiques (O-FQD), les cellules électrochimiques à émission de lumière (LEC), les diodes laser organiques (O-laser) et les dispositifs électroluminescents organiques (OLED).

16. Dispositif électronique selon la revendication 15, **caractérisé en ce qu'**il est choisi parmi les dispositifs électroluminescents organiques et **en ce qu'**il comprend au moins un composé selon une ou plusieurs des revendications 1 à 10 ou au moins un polymère, un dendrimère ou un oligomère selon la revendication 11 selon une ou plusieurs des fonctions qui suivent :
- en tant que matériau de matrice dans une couche d'émission,
- en tant que matériau de transport d'électrons dans une couche de transport d'électrons ou dans une couche d'injection d'électrons,
- en tant que matériau de blocage de trous dans une couche de blocage de trous.
